# EUROPEAN PATENT APPLICATION

(11) **EP 3 157 026 A1**
(43) Date of publication of application: **19.04.2017**
(21) Application number: 15805896.6
(22) Date of filing: 09.06.2015
(51) Int. Cl.: H01G 9/20, C07D 213/22, C07D 401/04, C07D 405/14, C07D 409/06, C07D 495/04, C09B 57/10, C09B 67/44

(54) **PHOTOELECTRIC CONVERSION ELEMENT, DYE-SENSITIZED SOLAR CELL, METAL-COMPLEX PIGMENT, PIGMENT SOLUTION, AND TERPYRIDINE COMPOUND OR ESTERIFIED TERPYRIDINE COMPOUND**

(30) Priority: 11.06.2014 JP 2014121016; 09.03.2015 JP 2015046443
(71) Applicant: Fujifilm Corporation, Minato-ku Tokyo 106-8620 (JP)
(72) Inventor: TSUNA, Kazuhiro, Ashigarakami-gun Kanagawa 258-8577 (JP); SUGIURA, Hiroki, Ashigarakami-gun Kanagawa 258-8577 (JP); YOSHIOKA, Tomoaki, Ashigarakami-gun Kanagawa 258-8577 (JP); WATANABE, Kousuke, Ashigarakami-gun Kanagawa 258-8577 (JP); KOBAYASHI, Katsumi, Ashigarakami-gun Kanagawa 258-8577 (JP)
(74) Representative: Hoffmann Eitle
(86) International application number: PCT/JP2015/066573
(87) International publication number: WO 2015/190467

(57) **Abstract**

A photoelectric conversion element, a dye-sensitized solar cell, and a metal-complex pigment, pigment solution, and terpyridine compound or esterified terpyridine compound used therein. The photoelectric conversion element has an electrically-conductive support, an electrolyte-containing photoreceptor layer, an electrolyte-containing charge-transfer layer, and a counter electrode. The photoreceptor layer contains semiconductor microparticles on which a metal-complex pigment that can be represented by a specific formula (1) is supported.

## Description

### BACKGROUND OF THE INVENTION

### 1. Field of the Invention

The present invention relates to a photoelectric conversion element, a dye-sensitized solar cell, a metal complex dye, a dye solution, and a terpyridine compound or an esterified product thereof.

### 2. Description of the Related Art

Photoelectric conversion elements are used in various photosensors, copying machines, photoelectrochemical cells such as solar cells, and the like. These photoelectric conversion elements have adopted various systems to be put into use, such as systems utilizing metals, systems utilizing semiconductors, systems utilizing organic pigments or dyes, or combinations of these elements. In particular, solar cells utilizing inexhaustible solar energy do not necessitate fuels, and full-fledged practicalization of solar cells as an inexhaustible clean energy is being highly expected. Above all, research and development of silicon-based solar cells has long been in progress, and many countries also support policy-wise considerations, and thus dissemination of silicon-based solar cells is still in progress. However, silicon is an inorganic material, and thus, naturally has limitations in terms of improvement of throughput, cost, and the like.

Thus, research is being vigorously carried out on photoelectrochemical cells (also referred to as dye-sensitized solar cells) using metal complex dyes. In particular, what have built momentum toward such research was the research results from Graetzel et al. of École Polytechnique Fédérale de Lausanne in Switzerland. They employed a structure in which a dye formed from a ruthenium complex was fixed on the surface of a porous titanium oxide film, and realized a photoelectric conversion efficiency which was comparable to that of amorphous silicon. Thus, dye-sensitized solar cells that can be produced even without use of expensive vacuum devices have instantly attracted the attention of researchers all over the world.

Hitherto, dyes called N3, N719, N749 (also referred to as Black Dye), Z907, and J2 have generally been developed as metal complex dyes for use in dye-sensitized solar cells. However, all the photoelectric conversion elements and dye-sensitized solar cells using these dyes are not sufficient in terms of photoelectric conversion efficiency and durability (heat stability).

Therefore, development of metal complex dyes capable of improving the photoelectric conversion efficiency or the durability of photoelectric conversion elements and dye-sensitized solar cells is in progress.

For example, JP2012-36237A describes a metal complex dye having a tridentate ligand and a bidentate ligand which coordinate to metal atoms with a lone electron pair of a ring-forming nitrogen atom, and also describes that a photoelectrochemical cell using the metal complex dye has high photoelectric conversion efficiency and excellent durability.

In addition, Advanced Functional Materials 2013, 23, pp. 1817-1823 describes a Ru complex having a 4-methylstyryl group-containing terpyridine ligand and three thiocyanate ligands, and also describes that the overall conversion efficiency (η) of a dye-sensitized solar cell using the Ru complex is higher than that of the Black Dye.

### SUMMARY OF THE INVENTION

However, photoelectric conversion elements and dye-sensitized solar cells increasingly require higher performance every year, and there is a demand for, in particular, further modifications and improvements in their photoelectric conversion efficiency and durability.

Furthermore, in photoelectric conversion elements and dye-sensitized solar cells, a layer (also referred to as a semiconductor layer) formed of semiconductor fine particles carrying a metal complex dye is usually formed as a layer having a thickness of 10 to several hundred µm. At this time, the photoelectric conversion efficiency varies depending on the film thickness of the semiconductor layer, and accordingly, there is a tendency that the photoelectric conversion efficiency is reduced as the film thickness decreases. It could be seen that for metal complex dyes capable of absorbing near infrared light in the related art, in any of a case where the film thickness of the semiconductor layer was 10 to several hundred µm or a case where the semiconductor layer was even thinner, the photoelectric conversion efficiency was not necessarily satisfactory.

The present invention has an object to provide a photoelectric conversion element and a dye-sensitized solar cell, each of which exhibits excellent photoelectric conversion efficiency and has high durability, irrespective of the film thickness of a semiconductor layer, in particular, even when the film thickness is small; and a metal complex dye, a dye solution, and a terpyridine compound or an esterified product thereof, each of which is used in the photoelectric conversion element and the dye-sensitized solar cell.

The present inventors have conducted extensive studies on metal complex dyes for use in photoelectric conversion elements and dye-sensitized solar cells, and as a result, they have found that it is important to use a combination of a tridentate ligand which coordinates to a metal ion of a metal complex dye with a lone electron pair of a ring-constituting atom, as a ligand adsorbed on semiconductor fine particles (also referred to as an acceptor ligand), and a bidentate or tridentate ligand which coordinates to a metal ion of a metal complex dye with an anion of a ring-constituting atom, as a ligand not adsorbed on semiconductor fine particles (also referred to as a donor ligand), and in addition, introduce a specific group including an aliphatic unsaturated group and an aromatic ring group into a specific ring constituting an acceptor ligand, and introduce a specific organic group such as an amino group into a donor ligand, in order to improve the photoelectric conversion efficiency and the durability, and furthermore, realize high photoelectric conversion efficiency even when the semiconductor layer is a thin film. Based on these findings, the present invention has been completed.

That is, the tasks of the present invention have been achieved by the following means.
<1> A photoelectric conversion element comprising:
   an electrically conductive support;
   a photoconductor layer including an electrolyte;
   a charge transfer layer including an electrolyte; and
   a counter electrode,
   in which the photoconductor layer has semiconductor fine particles carrying a metal complex dye represented by the following Formula (1).

      Formula (1) ML1L2(X)ₙ₁·CI_{mY}

      In Formula (1),
      M represents a metal ion.
      L1 represents a tridentate ligand represented by the following Formula (L1-1). 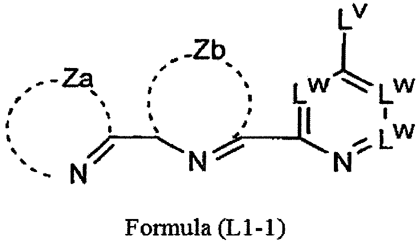
      In Formula (L1-1), Za and Zb each independently represent a non-metal atomic group required for completing a 5- or 6-membered ring. Here, at least one side of the rings formed by the respective Za and Zb has one or more acidic groups. L^{W}'s each independently represent a nitrogen atom or CR^{W}, and R^{W} represents a hydrogen atom or a substituent. L^{V} represents a group represented by the following Formula (LV-1) or (LV-2).

      **-R^{V1}=R^{V2}-R^{V3}** **Formula (LV-1)**

      **-C≡C-R^{V3}** **Formula (LV-2)**

      In Formula (LV-1), R^{V1} and R^{V2} each independently represent a nitrogen atom or CR^{V4}, and R^{V4} represents a hydrogen atom or a substituent. In Formula (LV-1) and Formula (LV-2), R^{V3} represents an aryl group or a heteroaryl group.
      L2 represents a bidentate or tridentate ligand represented by any one of the following Formulae (L2-1) to (L2-8).

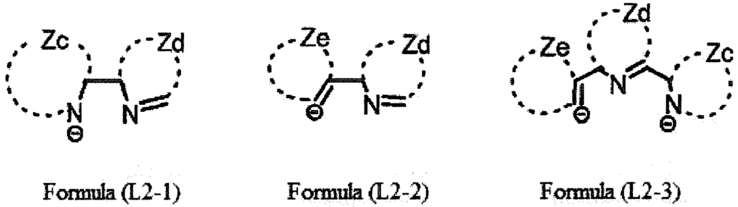

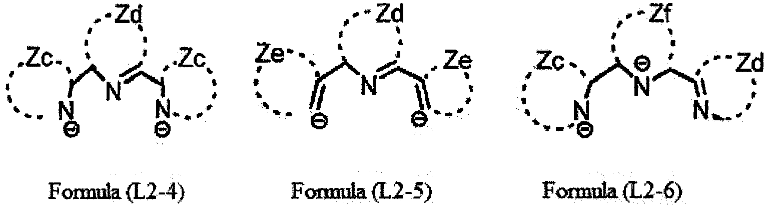

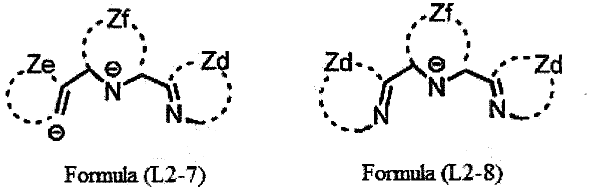

      In Formulae (L2-1) to (L2-8), Zc, Zd, Ze, and Zf each independently represent a non-metal atomic group required for completing a 5- or 6-membered aromatic ring.
      The ring formed by Zd has an organic group R^{VL} represented by the following Formula (R^{VL}).

      Formula (R^{VL}): -(R^{VL})n^{VL}

      In Formula (R^{VL}), R^{VL} represents an organic group selected from the group consisting of a monocyclic aromatic ring group bonded to the ring formed by Zd or a polycyclic aromatic ring group including the monocycle as a fused ring, in which the sp² carbon atoms at the α-position with respect to the ring-constituting atom bonded to the ring formed by Zd in a case where the monocycle is a 5-membered ring, and the sp² carbon atoms at the α- and β-positions with respect to the ring-constituting atom bonded to the ring formed by Zd in a case where the monocycle is a 6-membered ring are all bonded to hydrogen atoms or ring-constituting atoms of a fused ring different from the monocycle, an alkyl group, an alkenyl group, an alkynyl group, an alkylthio group, an alkoxy group, an amino group, and a silyl group. n^{VL} represents an integer of 0 or more, which is an integer that is not more than the number of hydrogen atoms when the ring formed by Zd is unsubstituted.
      X represents a monodentate ligand, and n1 represents 0 or 1.
      CI represents a counterion when the counterion is required to neutralize charges. mY represents an integer of 0 to 3.
<2> The photoelectric conversion element as described in <1>, in which the aromatic ring group of R^{VL} is a ring group represented by any one of the following Formulae (S-1) to (S-4). 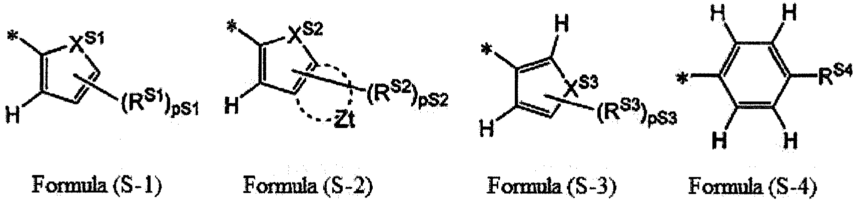
   In the formulae, X^{S1} to X^{S3} each independently represent -O-, -S-, -NR^{S}-, -C(R^{S})₂-, or -Si(R^{S})₂-, and R^{S}'s each represent a hydrogen atom or a substituent.
   Zt represents a non-metal atomic group required for forming a fused ring together with a ring including X^{S2}.
   R^{S1} to R^{S4} each independently represent a substituent. pS1 represents an integer of 0 to 2, pS2 is an integer of 0 or more, which is not more than the number of hydrogen atoms when the fused ring formed by Zt is unsubstituted, and pS3 represents 0 or 1.
   * represents a bonding moiety to the ring formed by Zd.
<3> The photoelectric conversion element as described in <2>, in which the organic group R^{VL} is an organic group selected from the group consisting of the ring group represented by Formula (S-1), the ring group represented by Formula (S-2), the ring group represented by Formula (S-3), the ring group represented by Formula (S-4), an alkyl group, an alkenyl group, an alkynyl group, an alkylthio group, an alkoxy group, and an amino group.
<4> The photoelectric conversion element as described in <2> or <3>, in which the organic group R^{VL} is a ring group represented by any one of Formula (S-1), Formula (S-2), Formula (S-3), and Formula (S-4), or an amino group.
<5> The photoelectric conversion element as described in any one of <2> to <4>, in which X^{S1} to X^{S3} each independently represent -O- or -S-.
<6> The photoelectric conversion element as described in any one of <1> to <5>, in which the organic group R^{VL} has at least one substituent selected from the group consisting of an aromatic ring group, an alkyl group, an alkenyl group, an alkynyl group, an alkylthio group, an alkoxy group, and an amino group.
<7> The photoelectric conversion element as described in <6>, in which the organic group R^{VL} is an amino group having the substituent.
<8> The photoelectric conversion element as described in any one of <1> to <7>, in which
   the ring formed by Za is at least one selected from the group consisting of a pyridine ring, a pyrimidine ring, a pyrazine ring, a pyridazine ring, a triazine ring, a tetrazine ring, a quinoline ring, an isoquinoline ring, a pyrazole ring, an imidazole ring, a triazole ring, a thiazole ring, an oxazole ring, a benzimidazole ring, a benzotriazole ring, a benzoxazole ring, and a benzothiazole ring,
   the ring formed by Zb is at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a triazole ring, a thiazole ring, an oxazole ring, a benzimidazole ring, a benzotriazole ring, a benzoxazole ring, and a benzothiazole ring, and
   the ring including L^{W} is at least one selected from the group consisting of a pyridine ring, a pyrimidine ring, a pyridazine ring, a triazine ring, a tetrazine ring, and a quinoline ring.
<9> The photoelectric conversion element as described in any one of <1> to <8>, in which
   the ring formed by Zc is at least one selected from the group consisting of a pyrazole ring, a pyrrole ring, an imidazole ring, a triazole ring, a benzimidazole ring, and an indole ring,
   the ring formed by Zd is at least one selected from the group consisting of a pyridine ring, a pyrimidine ring, a pyrazine ring, a pyridazine ring, a triazine ring, a tetrazine ring, a quinoline ring, a pyrazole ring, an imidazole ring, a triazole ring, a thiazole ring, an oxazole ring, a benzimidazole ring, a benzotriazole ring, a benzoxazole ring, and a benzothiazole ring,
   the ring formed by Ze is a benzene ring, and
   the ring formed by Zf is at least one selected from the group consisting of a pyrrole ring, an imidazole ring, a benzimidazole ring, and an indole ring.
<10> The photoelectric conversion element as described in any one of <1> to <9>, in which M is Ru²⁺ or Os²⁺.
<11> The photoelectric conversion element as described in any one of <1> to <10>, in which L1 is a tridentate ligand represented by the following Formula (L1-2). 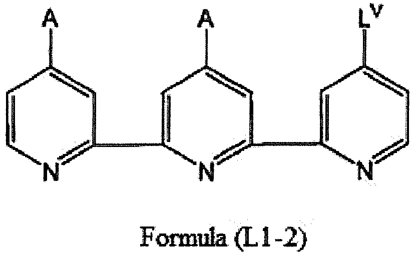
   In Formula (L1-2), A represents an acidic group. L^{V} has the same definition as L^{V} of Formula (L1-1).
<12> The photoelectric conversion element as described in any one of <1> to <11>, in which the acidic group is a carboxy group or a salt thereof.
<13> The photoelectric conversion element as described in any one of <1> to <12>, in which the metal complex dye represented by Formula (1) is a metal complex dye represented by any one of the following Formulae (2) to (6). 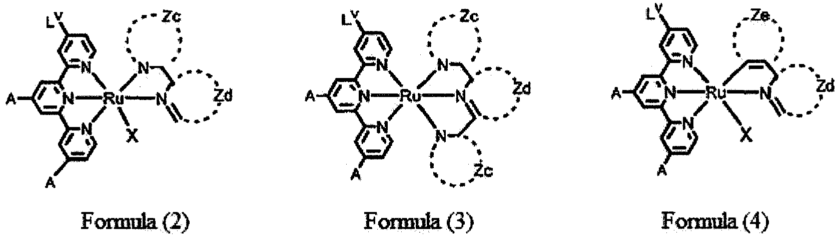 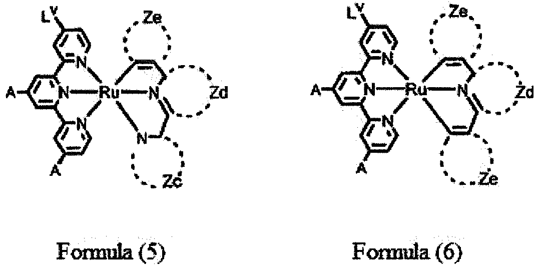
   In Formulae (2) to (6), X has the same definition as X of Formula (1). Zc, Zd, and Ze have the same definitions as Zc, Zd, and Ze, respectively, in Formulae (L2-1) to (L2-5). L^{V} has the same definition as L^{V} of Formula (L1-1). A represents an acidic group.
<14> The photoelectric conversion element as described in any one of <1> to <13>, in which L^{V} is the group represented by Formula (LV-2).
<15> The photoelectric conversion element as described in any one of <1> to <14>, in which R^{V3} is a heteroaryl group.
<16> The photoelectric conversion element as described in any one of <1> to <15>, in which R^{V3} has an alkyl group, an alkoxy group, or an alkylthio group as a substituent.
<17> The photoelectric conversion element as described in any one of <1> to <16>, in which the heteroaryl group of R^{V3} is a monocyclic group bonded to an ethynylene group in Formula (LV-2) or a polycyclic group including the monocycle as a fused ring, in which at least one of the sp² carbon atoms at the α-position with respect to the ring-constituting atom bonded to the ethynylene group in a case where the monocycle is a 5-membered ring has a substituent, and at least one of the sp² carbon atoms at the α- and β-positions with respect to the ring-constituting atom bonded to the ethynylene group in a case where the monocycle is a 6-membered ring has a substituent.
<18> The photoelectric conversion element as described in any one of <1> to <17>, in which the heteroaryl group of R^{V3} is represented by the following Formula (LV-3). 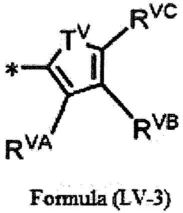
   In the formula, T^{V} represents -O-, -S-, -NR^{TV}-, -C(R^{TV})₂-, or -Si(R^{TV})₂-, and R^{TV}'s each represent a hydrogen atom or a substituent.
   R^{VA} represents a substituent, and R^{VB} and R^{VC} each independently represent a hydrogen atom or a substituent.
   * represents a binding position to an ethynylene group in Formula (LV-2).
<19> A dye-sensitized solar cell comprising the photoelectric conversion element as described in any one of <1> to <18>.
<20> A metal complex dye represented by the following Formula (1).

   Formula (1) ML1L2(X)ₙ₁·CI_{mY}

   In Formula (1),
   M represents a metal ion, and
   L1 represents a tridentate ligand represented by the following Formula (L1-1). 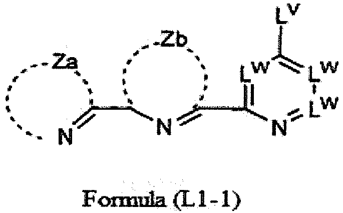
   In Formula (L1-1), Za and Zb each independently represent a non-metal atomic group required for completing a 5- or 6-membered ring. Here, at least one side of the rings formed by the respective Za and Zb has one or more acidic groups. L^{W}'s each independently represent a nitrogen atom or CR^{W}, and R^{W} represents a hydrogen atom or a substituent. L^{V} represents a group represented by the following Formula (LV-1) or (LV-2).

   **-R^{V1}=R^{V2}-R^{V3}** **Formula (LV-1)**

   **-C≡C-R^{V3}** **Formula (LV-2)**

   In Formula (LV-1), R^{V1} and R^{V2} each independently represent a nitrogen atom or CR^{V4}, and R^{V4} represents a hydrogen atom or a substituent. In Formula (LV-1) and Formula (LV-2), R^{V3} represents an aryl group or a heteroaryl group.
   L2 represents a bidentate or tridentate ligand represented by any one of the following Formulae (L2-1) to (L2-8).

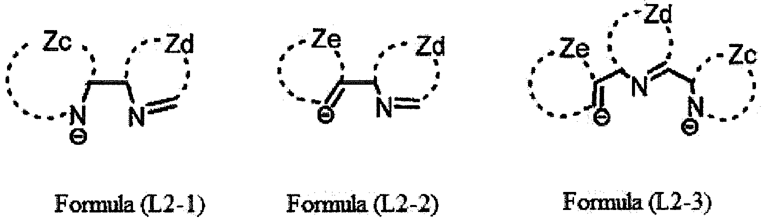

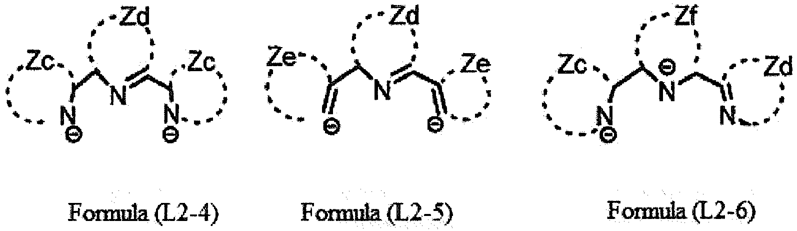

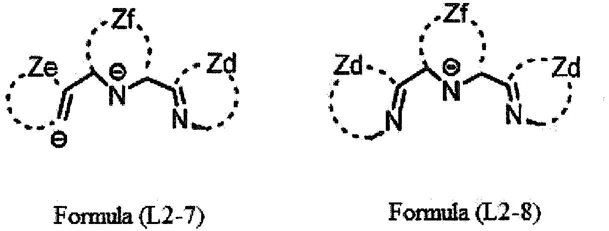

   In Formulae (L2-1) to (L2-8), Zc, Zd, Ze, and Zf each independently represent a non-metal atomic group required for completing a 5- or 6-membered aromatic ring.
   The ring formed by Zd has an organic group R^{VL} represented by the following Formula (R^{VL}).

   Formula (R^{VL}): -(R^{VL})ₙ^{VL}

   In Formula (R^{VL}), R^{VL} represents an organic group selected from the group consisting of a monocyclic aromatic ring group bonded to the ring formed by Zd or a polycyclic aromatic ring group including the monocycle as a fused ring, in which the sp² carbon atoms at the α-position with respect to the ring-constituting atom bonded to the ring formed by Zd in a case where the monocycle is a 5-membered ring, and the sp² carbon atoms at the α- and β-positions with respect to the ring-constituting atom bonded to the ring formed by Zd in a case where the monocycle is a 6-membered ring are all bonded to hydrogen atoms or ring-constituting atoms of a fused ring different from the monocycle, an alkyl group, an alkenyl group, an alkynyl group, an alkylthio group, an alkoxy group, an amino group, and a silyl group. n^{VL} represents an integer of 0 or more, which is an integer that is not more than the number of hydrogen atoms when the ring formed by Zd is unsubstituted.
   X represents a monodentate ligand and n1 represents 0 or 1.
   CI represents a counterion when the counterion is required to neutralize charges. mY represents an integer of 0 to 3.
<21> A dye solution comprising the metal complex dye as described in <20> and a solvent.
<22> A terpyridine compound represented by the following Formula (L1-2) or an esterified product thereof. 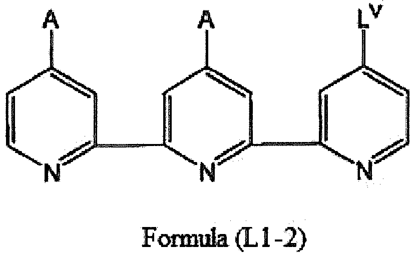
   In Formula (L1-2), A represents an acidic group, and L^{V} represents a group represented by the following Formula (LV-2).

   **-C≡-C-R^{V3}** **Formula (LV-2)**

   In Formula (LV-2), R^{V3} represents a heteroaryl group.

In the present specification, unless otherwise specified, in a case where the E configuration or the Z configuration exists in the molecule for a double bond, the double bond may be either one of the two configurations or a mixture thereof.

When there are a plurality of substituents, linking groups, ligands, or the like (hereinafter referred to as substituents or the like) represented by specific symbols, or when a plurality of substituents or the like are defined at the same time, each of the substituents or the like may be the same as or different from each another, unless otherwise specified. This also applies to the definition of the number of substituents or the like. Further, when a plurality of substituents or the like are close to one another (in particular, adjacent to each other), they may be linked to one another to form a ring, unless otherwise specified. In addition, rings, for example, aliphatic rings, aromatic rings, or heterocycles may further be fused to form a fused ring.

In the present specification, expressions of a compound (including a complex and a dye) are used to mean, in addition to the compound itself, salts and ions of the compound, and within a range exhibiting desired effects, include modifications of a part of the structure. Further, a compound in which substitution or non-substitution is not explicitly described is used to mean that the compound may have an arbitrary substituent within a range exhibiting desired effects. This shall apply to substituents, linking groups, and ligands.

Moreover, in the present specification, a numerical value range represented by "(a value) to (a value)" means a range including the numerical values represented before and after "to" as a lower limit value and an upper limit value, respectively.

The photoelectric conversion element and the dye-sensitized solar cell of the present invention exert excellent photoelectric conversion efficiency and high durability, irrespective of the film thickness of the semiconductor layer, by including a metal complex dye using a combination of a tridentate ligand L1 represented by Formula (L1-1) and a bidentate or tridentate ligand L2 represented by any one of Formulae (L2-1) to (L2-8). Therefore, according to the present invention, it is possible to provide a photoelectric conversion element and a dye-sensitized solar cell, each of which exhibits excellent photoelectric conversion efficiency and has high durability, irrespective of the film thickness of a semiconductor layer, in particular, even when the film thickness is small; and a metal complex dye, a dye solution, and a terpyridine compound or an esterified product thereof, each of which is used in the photoelectric conversion element and the dye-sensitized solar cell.

The above or other characteristics and advantages of the present invention will be further clarified from the following description with reference to drawings appropriately attached hereto.

### BRIEF DESCRIPTION OF THE DRAWINGS

Fig. 1 is a cross-sectional view schematically showing a photoelectric conversion element in the first aspect of the present invention, including an enlarged view of the circled portion in the layer thereof, in a system in which the photoelectric conversion element is applied in cell uses.
Fig. 2 is a cross-sectional view schematically showing a dye-sensitized solar cell including a photoelectric conversion element in the second aspect of the present invention.
Fig. 3 is a visible absorption spectrum diagram of metal complex dyes (1-1), (2-1), (21-1), and (26-1) to (28-1) of the present invention, synthesized in Example 1, in a TBAOH methanol solvent.
Fig. 4 is a ¹H-NMR spectrum of a compound (21-4) synthesized in Example 1.
Fig. 5 is a ¹H-NMR spectrum of a compound (36-2) synthesized in Example 1.

### DESCRIPTION OF THE PREFERRED EMBODIMENTS

### [Photoelectric Conversion Element and Dye-Sensitized Solar Cell]

The photoelectric conversion element of the present invention has an electrically conductive support, a photoconductor layer including an electrolyte, a charge transfer layer including an electrolyte, and a counter electrode (opposite electrode). The photoconductor layer, the charge transfer layer, and the counter electrode are provided in this order on an electrically conductive support.

In the photoelectric conversion element of the present invention, at least a portion of the semiconductor fine particles forming the photoconductor layer carries a metal complex dye represented by Formula (1) which will be described later, as a sensitizing dye. Here, examples of the aspect in which the metal complex dye is carried on the surface of the semiconductor fine particles include an aspect in which the metal complex dye is deposited on the surface of the semiconductor fine particles, an aspect in which the metal complex dye is adsorbed onto the surface of the semiconductor fine particles, and a mixture of the aspects. The adsorption includes chemical adsorption and physical adsorption, with the chemical adsorption being preferable.

The semiconductor fine particles carry other metal complex dyes, together with the metal complex dye of Formula (1) which will be described later.

The semiconductor fine particles preferably carry a co-adsorbent which will be described later, together with the metal complex dye.

Moreover, the photoconductor layer includes an electrolyte. The electrolyte included in the photoconductor layer may be different from or the same as the electrolyte included in the charge transfer layer, but they are preferably the same as each other. Here, the expression "electrolytes are the same as each other" is meant to encompass both an aspect in which the components included in the electrolyte of the photoconductor layer are the same as the components included in the electrolyte of the charge transfer layer and the contents of both the components are the same, and an aspect in which the components included in the electrolyte of the photoconductor layer are the same as the components included in the electrolyte of the charge transfer layer but the contents of both the components are different.

The photoelectric conversion element of the present invention is not particularly limited in terms of configurations other than the configuration defined in the present invention, and may adopt known configurations regarding photoelectric conversion elements. The respective layers constituting the photoelectric conversion element of the present invention are designed according to purposes, and may be formed in, for example, in a single layer or in multiple layers. Further, layers other than the layers may be included, as necessary.

The dye-sensitized solar cell of the present invention is formed by using the photoelectric conversion element of the present invention.

Hereinafter, preferred embodiments of the photoelectric conversion element and the dye-sensitized solar cell of the present invention will be described.

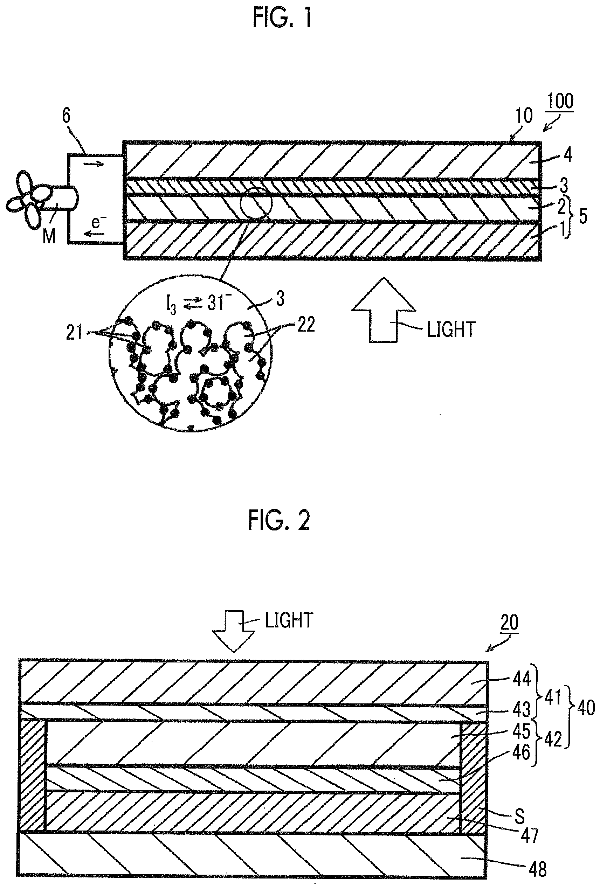

A system 100 shown in Fig. 1 is a system in which a photoelectric conversion element 10 in the first aspect of the present invention is applied in cell uses where an operating means M (for example, an electric motor) in an external circuit 6 is forced to work.

The photoelectric conversion element 10 includes semiconductor fine particles 22 sensitized by carrying an electrically conductive support 1 and a dye (metal complex dye) 21, a photoconductor layer 2 including an electrolyte between the semiconductor fine particles 22, a charge transfer layer 3 that is a hole transport layer, and a counter electrode 4.

In the photoelectric conversion element 10, the light-receiving electrode 5 has the electrically conductive support 1 and the photoconductor layer 2, and functions as a functional electrode.

In the system 100 in which the photoelectric conversion element 10 is applied, light incident to the photoconductor layer 2 excites the metal complex dye 21. The excited metal complex dye 21 has electrons having high energy, and these electrons are transferred from the metal complex dye 21 to a conduction band of the semiconductor fine particles 22, and further reach the electrically conductive support 1 by diffusion. At this time, the metal complex dye 21 is in an oxidized form (cation). While the electrons reaching the electrically conductive support 1 work in an external circuit 6, they reach the oxidized form of the metal complex dye 21 through the counter electrode 4 and the charge transfer layer 3, and reduce the oxidized form, whereby the system 100 functions as a solar cell.

A dye-sensitized solar cell 20 shown in Fig. 2 is constituted with a photoelectric conversion element in the second aspect of the present invention.

With respect to the photoelectric conversion element shown in Fig. 1, the photoelectric conversion element which becomes the dye-sensitized solar cell 20 is different in the configurations of the electrically conductive support 41 and the photoconductor layer 42, and also differs in that it has a spacer S, but except for these, has the same structure as the photoelectric conversion element 10 shown in Fig. 1. That is, the electrically conductive support 41 has a bilayered structure including a substrate 44 and a transparent electrically-conductive film 43 which is formed on the surface of the substrate 44. Further, the photoconductor layer 42 has a bilayered structure including a semiconductor layer 45 and a light-scattering layer 46 which is formed adjacent to the semiconductor layer 45. A spacer S is provided between the electrically conductive support 41 and the counter electrode 48. In the dye-sensitized solar cell 20, 40 is a light-receiving electrode, and 47 is a charge transfer layer.

The dye-sensitized solar cell 20, similar to the system 100 in which the photoelectric conversion element 10 is applied, functions as a solar cell by light incident on the photoconductor layer 42.

The photoelectric conversion element and the dye-sensitized solar cell of the present invention are not limited to the above preferred aspects, and the configuration of each of the aspects can be combined as appropriate within a range not departing from the scope of the present invention.

In the present invention, the materials and the respective members for use in the photoelectric conversion element and the dye-sensitized solar cell can be prepared by ordinary methods. Reference can be made to, for example, US4,927,721A, US4,684,537A, US5,084,365A, US5,350,644A, US5,463,057A, US5,525,440A, JP1995-249790A (JP-H07-249790A), JP2001-185244A, JP2001-210390A, JP2003-217688A, JP2004-220974A, and JP2008-135197.

### <Metal Complex Dye Represented by Formula (1)>

The metal complex dye for use in the present invention is represented by the following Formula (1). The metal complex dye of the present invention can impart high photoelectric conversion efficiency and excellent heat stability to the photoelectric conversion element and the dye-sensitized solar cell by including both of the following ligand L1 and the following ligand L2. By using the metal complex dye of the present invention, it is possible to attain wide absorption with respect to infrared light to visible light to near infrared light as well as a high molar light absorption coefficient even in the near infrared light region. Thus, it is possible to efficiently absorbing light even in a case where the film thickness of the semiconductor is small. Accordingly, the metal complex dye of the present invention is preferably used as a sensitizing dye in the dye-sensitized solar cell.

Formula (1) ML1L2(X)ₙ₁·CI_{mY}

In Formula (1), M represents a metal ion.
L1 represents a tridentate ligand represented by the following Formula (L1-1).

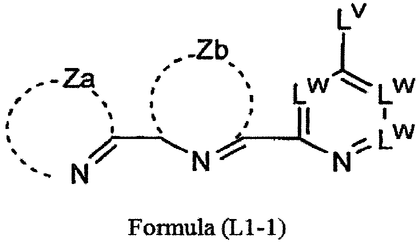

In Formula (L1-1), Za and Zb each independently represent a non-metal atomic group required for completing a 5- or 6-membered ring. Here, at least one side of the rings formed by the respective Za and Zb has one or more acidic groups. L^{W}'s each independently represent a nitrogen atom or CR^{W}, and R^{W} represents a hydrogen atom or a substituent. L^{V} represents a group represented by the following Formula (LV-1) or (LV-2).

**-R^{V1}=R^{V2}-R^{V3}** **Formula (LV-1)**

**-C≡C-R^{V3}** **Formula (LV-2)**

In Formula (LV-1), R^{V1} and R^{V2} each independently represent a nitrogen atom or CR^{V4}, and R^{V4} represents a hydrogen atom or a substituent. In Formula (LV-1) and Formula (LV-2), R^{V3} represents an aryl group or a heteroaryl group.

L2 represents a bidentate or tridentate ligand represented by any one of the following Formulae (L2-1) to (L2-8).

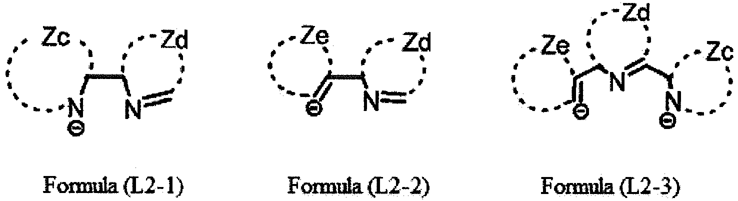

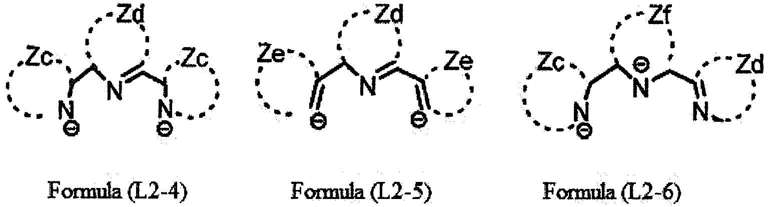

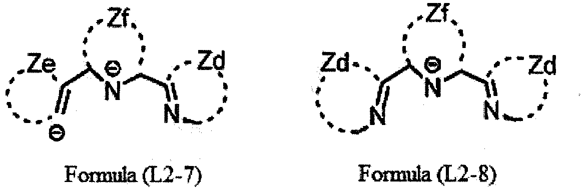

In Formulae (L2-1) to (L2-8), Zc, Zd, Ze, and Zf each independently represent a non-metal atomic group required for completing a 5- or 6-membered aromatic ring.

The ring formed by Zd has an organic group R^{VL} represented by the following Formula (R^{VL}).

Formula (R^{VL}): -(R^{VL})n^{VL}

In Formula (R^{VL}), R^{VL} represents an organic group selected from the group consisting of a monocyclic aromatic ring group bonded to the ring formed by Zd or a polycyclic aromatic ring group including the monocycle as a fused ring, in which the sp² carbon atoms at the α-position with respect to the ring-constituting atom bonded to the ring formed by Zd in a case where the monocycle is a 5-membered ring, and the sp² carbon atoms at the α- and β-positions with respect to the ring-constituting atom bonded to the ring formed by Zd in a case where the monocycle is a 6-membered ring are all bonded to hydrogen atoms or ring-constituting atoms of a fused ring different from the monocycle, an alkyl group, an alkenyl group, an alkynyl group, an alkylthio group, an alkoxy group, an amino group, and a silyl group. n^{VL} represents an integer of 0 or more, which is an integer that is not more than the number of hydrogen atoms when the ring formed by Zd is unsubstituted.

X represents a monodentate ligand and n1 represents 0 or 1. When the ligand L2 is a bidentate ligand, n1 represents 1, and when the ligand L2 is a tridentate ligand, n1 represents 0.

CI represents a counterion when the counterion is required to neutralize charges. mY represents an integer of 0 to 3, preferably 0 or 1, and more preferably 0.

### - Metal Ion M -

M is a central metal ion of the metal complex dye, and examples thereof include ions of elements belonging to Groups 6 to 12 on the long-form periodic table of the elements. Examples of such metal ions include respective ions of Ru, Fe, Os, Cu, W, Cr, Mo, Ni, Pd, Pt, Co, Ir, Rh, Re, Mn, and Zn. The metal ion M may be one kind of ion, or two or more kinds of ions.

In the present invention, the metal ion M is preferably Os²⁺, Ru²⁺, or Fe²⁺, more preferably Os²⁺ or Ru²⁺, and particularly preferably Ru²⁺ among them.

In addition, in a state of being incorporated in the photoelectric conversion element, the valence of M may be changed by the redox reaction with the surrounding material.

### - Ligand L1-

The ligand L1 is a tridentate ligand or compound represented by Formula (L1-1), in which three nitrogen atoms in Formula (L1-1) coordinate a metal ion M. Further, the ligand L1 has one or more acidic groups (also referred to as adsorptive groups) on at least one of the ring formed by Za or the ring formed by Zb which will be described later. The ligand L1 is a ligand making the metal complex dye of the present invention carried on semiconductor fine particles.

In Formula (L1-1), Za and Zb each independently represent a non-metal atomic group required for forming a 5-membered ring or 6-membered ring. Za and Zb are preferably a non-metal atomic group selected from a carbon atom, a nitrogen atom, an oxygen atom, a sulfur atom, and a phosphorus atom.

The rings formed by Za and Zb are preferably an aromatic ring of a 5-membered ring and an aromatic ring of a 6-membered ring. The aromatic ring of a 5-membered ring is preferably at least one of a pyrazole ring, an imidazole ring, a triazole ring, a thiazole ring, an oxazole ring, a benzimidazole ring, a benzotriazole ring, a benzoxazole ring, and a benzothiazole ring. The aromatic ring of a 6-membered ring is preferably at least one of a pyridine ring, a pyrimidine ring, a pyrazine ring, a pyridazine ring, a triazine ring, a tetrazine ring, a quinoline ring, and an isoquinoline ring.

As the rings formed by Za and Zb, an aromatic ring which is suitable for the structure of each ring represented by Formula (L1-1) of a group of the aromatic rings of 5-membered rings and a group of the aromatic rings of 6-membered rings. The ring formed by Za is preferably at least one of a pyridine ring, a pyrimidine ring, a pyrazine ring, a pyridazine ring, a triazine ring, a tetrazine ring, a quinoline ring, an isoquinoline ring, a pyrazole ring, an imidazole ring, a triazole ring, a thiazole ring, an oxazole ring, a benzimidazole ring, a benzotriazole ring, a benzoxazole ring, and a benzothiazole ring. The ring formed by Zb is preferably at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a triazole ring, a thiazole ring, an oxazole ring, a benzimidazole ring, a benzotriazole ring, a benzoxazole ring, and a benzothiazole ring.

Among those, the rings formed by Za and Zb are more preferably an imidazole ring, a pyridine ring, or a quinoline ring, and particularly preferably, they are all pyridine rings.

The rings formed by Za and Zb have one or more acidic groups on at least one side thereof, and preferably, each of the rings has one or more acidic groups. The number of acidic groups contained in each of the rings formed by Za and Zb is preferably 1 to 3, more preferably 1 or 2, and still more preferably 1.

The rings formed by Za and Zb may or may not have a substituent other than the respective acidic groups, and they may be a monocycle or a fused ring. Examples of the substituent that the rings may have include a group (excluding an acidic group) selected from the substituent group T which will be described later.

The acidic group in the present invention is a substituent which has a dissociative proton and has a pKa of 11 or less. The pKa of the acidic group can be determined in accordance with the "SMD/M05-2X/6-31G*" method described in J. Phys. Chem. A2011, 115, 6641-6645. Examples thereof include: an acid group showing acidity, such as a carboxyl group, a phosphonyl group, a phosphoryl group, a sulfo group, and a boric acid group; or a group having any of these acidic groups. Examples of the group having an acid group include groups having an acid group and a linking group. The linking group is not particularly limited, but examples thereof include a divalent group, and preferably an alkylene group, an alkenylene group, an alkynylene group, an arylene group, and a heteroarylene group. This linking group may have a group selected from the substituent group T which will be described later as a substituent. Preferred examples of the acidic group having an acid group and a linking group include carboxymethyl, carboxyvinylene, dicarboxyvinylene, cyanocarboxyvinylene, 2-carboxy-1-propenyl, 2-carboxy-1-butenyl, and carboxyphenyl.

The acidic group is preferably a group having a carboxy group or a carboxy group, and more preferably a carboxy group.

The acidic group may be in the form of a dissociated anion due to release of a proton or in the form of a salt, when the acidic group is included in the metal complex dye represented by Formula (1). When the acidic group is in the form of a salt, the counterion is not particularly limited, and examples thereof include those exemplified as positive ions in the following counterion CI. In addition, the acidic group may be esterified which will be described later.

In the rings formed by Za and Zb, the substitution position of the acidic group is not particularly limited. In the respective rings, the substitution position is preferably a ring-constituting atom which is the farthest from a nitrogen atom which coordinates to a metal ion M is preferable, and in a case where the ring is a 6-membered ring, the substitution position is preferably the 4-position with respect to the nitrogen atom.

In Formula (L1-1), a ring formed by a nitrogen atom, a carbon atom, and L^{W} (also referred to as a ring including L^{W} and the like) has the following group L^{V}, and preferably, does not has an acidic group. The ring including L^{W} and the like may be a monocycle or a fused ring. L^{W} represents a nitrogen atom or CR^{W}. R^{W} represents a hydrogen atom or a (monovalent) substituent, and is preferably a hydrogen atom. The substituent that can be adopted as R^{W} is not particularly limited, and examples thereof include a group (preferably excluding the acidic group and the following group L^{V}) selected from the substituent group T which will be described later. In a case where the ring including L^{W} and the like has a plurality of R^{W}'s, the plurality of R^{W}'s may be the same as or different from each other, and R^{W}'s may also be bonded to each other to form a ring.

As the ring including L^{W} and the like, a ring which is suitable for the ring structure in Formula (AL-1) is preferably selected from the aromatic rings of 6-membered rings described as the rings formed by Za and Zb. The ring including L^{W} and the like is more preferably at least one of a pyridine ring, a pyrimidine ring, a pyridazine ring, a triazine ring, a tetrazine ring, and a quinoline ring, and particularly preferably a pyridine ring.

L^{V} is a group represented by the following Formula (LV-1) or (LV-2). Incorporation of the group L^{V} into the ring-constituting nitrogen atom at the 4-position with respect to the ring-constituting nitrogen atom which coordinates to the metal ion M of the ring including L^{W} and the like in the ligand L1 to be used in combination with the following ligand L2 in the metal complex dye can contribute to improvement of photoelectric conversion efficiency.

In the present invention, L^{V} is preferably a group represented by (LV-2).

**-R^{V1}=R^{V2}-R^{V3}** **Formula (LV- 1)**

**-C≡C-R^{V3}** **Formula (LV-2)**

In the formula, R^{V1} and R^{V2} each independently represent a nitrogen atom or CR^{V4}. R^{V4} represents a hydrogen atom or a substituent (monovalent), and the substituent has the same definition as the substituent of R^{W}, and the preferred examples thereof are also the same.

Examples of the "-R^{V1}=R^{V2}-" group of Formula (LV-1) includes a -CR^{V4}=CR^{V4}-group, a -CR^{V4}=N- group, an -N=CR^{V4}- group, and an -N=N- group. The "-R^{V1}=R^{V2}-" group is preferably a -CR^{V4}=CR^{V4}- group, and more preferably a -CH=CH- group.

In both of the formulae, R^{V3} represents an aryl group or a heteroaryl group.

The aryl group may be a monocyclic hydrocarbon ring group exhibiting aromaticity or a ring group formed of a hydrocarbon ring exhibiting aromaticity, formed by fusion of two or more rings (a fused polycyclic aromatic ring group).

The monocyclic hydrocarbon ring group is preferably a 6-membered ring, and examples thereof include a benzene ring group.

Preferred examples of the fused polycyclic aromatic ring group include a hydrocarbon ring formed by fusion of two or more 5- or 6-membered rings, including, for example, a hydrocarbon ring group formed by fused by a plurality of monocyclic hydrocarbon rings exhibiting aromaticity (benzene rings). Examples of such the fused polycyclic aromatic ring group include a ring group formed by fusion of two or more benzene rings, including, for example, the respective groups of a naphthalene ring, an anthracene ring, a phenanthrene ring, a tetracene ring, a pentacene ring, a hexacene ring, a heptacene ring, a chrysene ring, a picene ring, a pyrene ring, a perylene ring, a coronene ring, an ovalene ring, a fluorene ring, or a triphenylene ring. Here, the number of fused hydrocarbon rings is not particularly limited, and for example, it is preferably 2 to 10, more preferably 2 to 5, and still more preferably 2 or 3.

As the aryl group, the respective groups of a benzene ring, a naphthalene ring, an anthracene ring, a phenanthrene ring, a triphenylene ring, a pyrene ring, a fluorene ring, and the like are preferable, the respective groups of a benzene ring, a naphthalene ring, a pyrene ring, a fluorene ring, and the like are more preferable, and a benzene ring, a naphthalene ring, or a fluorene ring is still more preferable.

Examples of the heteroaryl group include a monocyclic heterocyclic group exhibiting aromaticity, and the fused polycyclic heterocyclic group (fused polycyclic aromatic heterocyclic group) in which a plurality of ring groups including a hetero ring are fused.

As the monocyclic heterocyclic group, a 5-membered ring or 6-membered ring group including a hetero atom as a ring-constituting atom is preferable. The hetero atom is not particularly limited, but examples thereof include a nitrogen atom, an oxygen atom, a sulfur atom, a silicon atom, a selenium atom, and a phosphorus atom. Examples of the 5-membered ring group include the respective groups of a thiophene ring, a furan ring, a pyrrole ring, a selenophene ring, a thiazole ring, an oxazole ring, an isothiazole ring, an isoxazole ring, an imidazole ring, a pyrazole ring, a thiadiazole ring, an oxadiazole ring, a silole ring, a triazole ring, or the like. Examples of the group of a 6-membered ring include the respective groups of a pyridine ring, a pyrazine ring, a pyrimidine ring, a pyridazine ring, a triazine ring, a tetrazine ring, or the like.

Examples of the fused polycyclic heterocyclic group include a ring group formed by fusion of a plurality of monocyclic hetero rings, and a ring group formed by fusion of a plurality of monocyclic hetero rings and hydrocarbon rings. Preferred examples thereof include a ring group formed by fusion of a plurality of homogeneous or heterogeneous rings selected from the group consisting of the respective groups of a benzene ring, a cyclopentadiene ring, a thiophene ring, a furan ring, a pyrrole ring, a selenophene ring, a silole ring, a thiazole ring, an oxazole ring, an isothiazole ring, an isoxazole ring, an imidazole ring, a pyrazole ring, a thiadiazole ring, an oxadiazole ring, a pyridine ring, a pyrazine ring, a pyrimidine ring, a pyridazine ring, a triazine ring, and a tetrazine ring. Here, the number of rings to be fused is not particularly limited, and is, for example, preferably 2 to 10, more preferably 2 to 5, and still more preferably 2 or 3.

Examples of the fused polycyclic heterocyclic group include the respective groups of a benzofuran ring, a dibenzofuran ring, an isobenzofuran ring, a benzothiophene ring, a dibenzothiophene ring, a benzisothiophene ring, a benzimidazole ring, a dibenzopyrrole ring, a carbazole ring, a silafluorene ring (dibenzosilole ring), an indazole ring, an indole ring, an isoindole ring, an indolizine ring, a quinoline ring, an isoquinoline ring, a thienopyridine ring, a cyclopentadifuran ring, a cyclopentadithiophene ring, a thieno[3,2-b]thiophene ring, a thieno[3,4-b]thiophene ring, a trithiophene ring, a benzodifuran ring, a benzodithiophene ring, a dithienopyrrole ring, a dithienosilole ring, and the like.

As the heteroaryl group, a thiophene ring group, a furan ring group, a pyrrole ring group, or a selenophene ring group is more preferable, a thiophene ring group or a furan ring group is still more preferable, and a thiophene ring group is particularly preferable.

As R^{V3}, a ring group selected from the group consisting of the respective groups of a benzene ring, a thiophene ring, a furan ring, a pyrrole ring, or a selenophene ring, or a ring group formed by linkage or fusion of a plurality of homogeneous or heterogeneous rings selected from the above groups is preferable.

In view of high effects of improving photoelectric conversion efficiency, R^{V3} is preferably a group having high electron donating properties. As the group having electron donating properties, an aromatic ring group in which an electron donating group is substituted, a fused polycyclic aromatic ring group in which an electron donating group may be substituted, or a heteroaryl group in which an electron donating group may be substituted is preferable, an aromatic ring group in which an electron donating group is substituted, and a heteroaryl group are more preferable, a heteroaryl group is still more preferable, and a thiophene ring is particularly preferable. In the present invention, the "electron donating properties" mean that the σ Value in a Hammett equation is negative.

The aryl group and the heteroaryl group of R^{V3} may each independently have a substituent, and in this case, examples of the substituent include a group selected from the substituent group T which will be described later (excluding an acidic group), preferably including, for example, an alkyl group, an alkoxy group, an alkylthio group (all of the details are shown in substituent T^{VA} that can be adopted as R^{VA} to R^{VC} below), an amino group (including an alkylamino group and an arylamino group, and having the same definition as the amino group (R^{VL-7}) which will be described later), or an aryl group. The number of the substituents is preferably 1 to 3, and more preferably 1 or 2. In a case where a plurality of these groups are included, adjacent groups may be linked to each other to form a ring. As a group formed together with the ring-constituting atom of R^{V3}, a -O-R^{ve}-O- group having two alkoxy groups linked thereto is preferable. Here, R^{ve} represents an alkylene group, and examples thereof include ethylene and propylene.

In a case where R^{V3} has a substituent, the atom to which the substituent is bonded (substitution position) is not particularly limited. For example, the atom may be any of ring-constituting atoms constituting R^{V3}, or may be an atom constituting another substituent which R^{V3} has.

Among the groups of R^{V3}, the heteroaryl group is preferably a group in which a specific sp² carbon atom among the ring-constituting atoms constituting a monocycle bonded to R^{V2} in Formula (LV-1) or an ethynylene group in Formula (LV-2) has a substituent. That is, the heteroaryl group is preferably a heteroaryl group other than the heteroaryl groups in which all of the specific sp² carbon atoms are bonded to hydrogen atoms or to a ring-constituting atom of a fused ring different from the monocycle. In this case, the heteroaryl group makes it possible that the monocycle or polycycle is bonded to an ethynylene group through the specific ring-constituting atom such that the specific sp² carbon atom satisfies the above conditions.

The substituent in which the specific sp² carbon atom has is not particularly limited, and examples thereof include the substituents described as R^{VA} which will be described later. At least one of the substituents in which the specific sp² carbon atom has is the substituent itself or a substituent which is bonded to another adjacent substituent and does not form a fused ring with a monocycle. The other substituents may be substituents which form a fused ring together with a monocycle.

Such the heteroaryl group is a monocyclic group bonded to R^{V2} in Formula (LV-1) or an ethynylene group in Formula (LV-2), or a polycyclic group including the monocycle as a fused ring, in which at least one of the sp² carbon atoms at the α-position with respect to the ring-constituting atom bonded to the ethynylene group in a case where the monocycle is a 5-membered ring has a substituent, or at least one of the sp² carbon atoms at the α- and β-positions with respect to the ring-constituting atom bonded to the ethynylene group in a case where the monocycle is a 6-membered ring has a substituent.

The heteroaryl group may or may not have a substituent in a case where the ring-constituting atom at the α-position in the 5-membered ring, and the ring-constituting atom at the α- and β-positions in the 6-membered ring are not sp² carbon atoms.

The heteroaryl group having the substituent is preferably a group represented by the following Formula (LV-3).

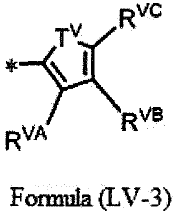

In the formula, T^{V} represents -O-, -S-, -NR^{TV}-, -C(R^{TV})₂-, or -Si(R^{TV})₂-, and R^{TV}'s each represent a hydrogen atom or a substituent.
R^{VA} represents a substituent, and R^{VB} and R^{VC} each independently represent a hydrogen atom or a substituent.
* represents a binding position to an ethynylene group in Formula (LV-2).

T^{V} is, among those, preferably -S-.

Here, R^{TV}'s in -NR^{TV}-, -C(R^{TV})₂-, or -Si(R^{TV})₂- each represent a hydrogen atom or a substituent, with a hydrogen atom being preferable. Examples of the substituent that can be adopted as R^{TV} include a group selected from the substituent group T which will be described later.

R^{VA} represents a substituent. R^{VB} represents a hydrogen atom or substituent, with a hydrogen atom being preferable. R^{VC} represents a hydrogen atom or a substituent, with a hydrogen atom being preferable.

The substituents that can be adopted as R^{VA} to R^{VC} are each not particularly limited, and examples thereof include the following substituents T^{VA}. In a case where R^{VB} or R^{VC} is a substituent, the respective substituents of R^{VA} to R^{VC} may be the same as or different from each other.

However, in the group represented by Formula (LV-3), R^{VA} is a substituent which does not form a fused ring together with a monocycle (a ring including T^{V}) bonded to R^{V2} in Formula (LV-1) or an ethynylene group in Formula (LV-2), and R^{VB} and R^{VC} may be a substituent which forms a fused ring together with this monocycle.

The substituent T^{VA} is not particularly limited, and examples thereof include a group selected from the substituent group T which will be described later. The substituent T^{VA} is preferably an alkyl group, a cycloalkyl group, an alkoxy group, a cycloalkoxy group, an aryl group, an aryloxy group, an alkylthio group, a cycloalkylthio group, an arylthio group, an amino group, an alkylamino group, a cycloalkylamino group, an arylamino group, a heterocyclic amino group, a silyl group, or a silyloxy group.

Among the respective groups, the substituent T^{VA} is more preferably an alkyl group, a cycloalkyl group, an alkoxy group, a cycloalkoxy group, an aryl group, an alkylthio group, a cycloalkylthio group, an amino group, an alkylamino group, a cycloalkylamino group, or an arylamino group, still more preferably an alkyl group, a cycloalkyl group, an aryl group, an alkylthio group, an alkylamino group, a cycloalkylamino group, or an arylamino group, particularly preferably an alkyl group, an aryl group, an alkylthio group, or an arylamino group, and most preferably an alkyl group or an aryl group. Among the substituents T^{VA}, R^{VA} is preferably an alkyl group.

The preferred substituents T^{VA} among the substituents that can be adopted as R^{VA} to R^{VC} are all preferably bonded to a thiophene ring (in a case where T^{V} is -S-) in view of photoelectric conversion efficiency.

The substituent T^{VA} that can be adopted as R^{VA} to R^{VC} may further be substituted with a group selected from the substituent group T which will be described later.

Examples of the alkyl group include a linear alkyl group and a branched alkyl group. The number of carbon atoms in the alkyl group is preferably 1 to 30, more preferably 4 to 30, still more preferably 5 to 26, and particularly preferably 6 to 20. Examples of the alkyl group include methyl, ethyl, n-butyl, t-butyl, n-pentyl, n-hexyl, n-octyl, 2-ethylhexyl, n-decyl, 3,7-dimethyloctyl, isodecyl, s-decyl, n-dodecyl, 2-butyloctyl, n-hexadecyl, isohexadecyl, n-eicosy, n-hexacosyl, isooctacosyl, trifluoromethyl, and pentafluoroethyl.

The number of carbon atoms in the cycloalkyl group is preferably 3 to 30, more preferably 5 to 30, still more preferably 6 to 26, and particularly preferably 6 to 20. Examples of the cycloalkyl group include cyclopropyl, cyclopentyl, cyclohexyl, cycloheptyl, and cyclooctyl. The cycloalkyl group may also be fused with an aliphatic ring, an aromatic ring, or a hetero ring.

Examples of the alkoxy group include a linear alkoxy group and a branched alkoxy group. The alkyl moiety of the alkoxy group has the same definition as the alkyl group, and the preferred examples thereof are also the same. Examples of the alkoxy group include methoxy, ethoxy, n-propoxy, i-propoxy, n-butoxy, t-butoxy, n-pentoxy, n-hexyloxy, n-octyloxy, 2-ethylhexyloxy, 3,7-dimethyloctyloxy, n-decyloxy, isodecyloxy, s-decyloxy, 2-butyloctyloxy, n-dodecyloxy, n-hexadecyloxy, isohexadecyloxy, n-eicosyoxy, n-hexacosyloxy, and isooctacosyloxy.

The cycloalkyl moiety of the cycloalkoxy group has the same definition as the cycloalkyl group, and the preferred examples thereof are also the same. Examples of the cycloalkoxy group include cyclopropyloxy, cyclopentyloxy, cyclohexyloxy, cycloheptyloxy, and cyclooctyloxy.

Examples of the aryl group include an aryl group, a hydrocarbon ring-based aryl group which is an aromatic hydrocarbon ring, and a heteroaryl group in which an aryl group is an aromatic heterocyclic group. The number of carbon atoms is preferably 3 to 30, more preferably 3 to 25, still more preferably 3 to 20, and particularly preferably 3 to 16. The aryl group is not particularly limited, but examples thereof include a phenyl group, a naphthyl group, an imidazolyl group, a benzimidazolyl group, a pyridyl group, a pyrimidinyl group, a furanyl group, and thienyl group. As the hetero ring of the heteroaryl group, a thiophene ring is preferable. The aryl group may further be substituted with a substituent T.

Examples of the aryloxy group include a hydrocarbon ring-based aryloxy group in which an aryl group is an aromatic hydrocarbon ring, and a heteroaryl group in which an aryl group is an aromatic heterocyclic group. The number of carbon atoms in the aryloxy group is preferably 3 to 30, more preferably 3 to 25, still more preferably 3 to 20, and particularly preferably 3 to 16. Examples of the aryloxy group include phenoxy, naphthoxy, imidazoyloxy, benzimidazoyloxy, pyridin-4-yloxy, pyrimidinyloxy, quinazolinyloxy, purinyloxy, and thiophen-3-yloxy. As the hetero ring of the heteroaryloxy group, a thiophene ring is preferable.

Examples of the alkylthio group include a linear alkylthio group and a branched alkylthio group. The alkyl moiety of the alkylthio group has the same definition as the alkyl group, and the preferred examples thereof are also the same. Examples of the alkylthio group include methylthio, ethylthio, n-propylthio, i-propylthio, n-butylthio, t-butylthio, n-pentylthio, n-hexylthio, n-octylthio, 2-ethylhexylthio, 3,7-dimethyloctylthio, n-decylthio, isodecylthio, s-decylthio, n-dodecylthio, 2-butyloctylthio, n-hexadecylthio, isohexadecylthio, n-eicosythio, n-hexacosylthio, and isooctacosylthio.

The cycloalkyl moiety of the cycloalkylthio group has the same definition as the cycloalkyl group, and the preferred examples thereof are also the same. Examples of the cycloalkylthio group include cyclopropylthio, cyclopentylthio, cyclohexylthio, cycloheptylthio, and cyclooctylthio.

Examples of the arylthio group include a hydrocarbon ring-based arylthio group in which an aryl group is an aromatic hydrocarbon ring, and a heteroarylthio group in which an aryl group is an aromatic heterocyclic group. The number of carbon atoms in the arylthio group is preferably 3 to 30, more preferably 3 to 25, still more preferably 3 to 20, and particularly preferably 3 to 16. Examples of the arylthio group include phenylthio, naphthylthio, imidazoylthio, benzimidazoylthio, pyridin-4-ylthio, pyrimidinylthio, quinazolinylthio, purinylthio, and thiophen-3-ylthio. As the hetero ring of the heteroarylthio group, a thiophene ring is preferable.

Examples of the alkylamino group is an N-alkylamino group and an N,N-dialkylamino group, and the number of carbon atoms in the alkyl group is preferably 1 to 30, and more preferably 2 to 30. Examples of the alkylamino group include ethylamino, diethylamino, 2-ethylhexylamino, bis(2-ethylhexyl)amino, or n-octadecylamino.

Examples of the cycloalkylamino group includes an N-cycloalkylamino group and an N,N-dicycloalkylamino group. The cycloalkyl moiety of the cycloalkylamino group has the same definition as the cycloalkyl group, and the preferred examples thereof are also the same. Examples of the cycloalkylamino group include cyclopropylamino, dicyclopropylamino, N-cyclopropyl-N-ethylamino, cyclopentylamino, dicyclopentylamino, N-cyclopentyl-N-methylamino, cyclohexylamino, dicyclohexylamino, cycloheptylamino, and cyclooctylamino.

Examples of the arylamino group include a hydrocarbon ring-based arylamino group in which an aryl group is an aromatic hydrocarbon ring, and a heteroarylamino group in which an aryl group is an aromatic heterocyclic group. Further, examples of the hydrocarbon ring-based arylamino group include an N-arylamino group, an N-alkyl-N-arylamino group, and an N,N-diarylamino group. Examples of the heteroarylamino group include an N-heteroarylamino group, an N-alkyl-N-heteroarylamino group, an N-aryl-N-heteroarylamino group, and an N,N-diheteroarylamino group.

The number of carbon atoms in the arylamino group is preferably 3 to 30, more preferably 3 to 25, still more preferably 3 to 20, and particularly preferably 3 to 16. Examples of the arylamino group include phenylamino, N-phenyl-N-ethylamino, naphthylamino, imidazoylamino, benzimidazoylamino, pyridin-4-ylamino, pyrimidinylamino, quinazolinylamino, purinylamino, and thiophen-3-ylamino.

The heterocyclic amino group is a heterocyclic amino group (aliphatic heterocyclic amino group) other than a heteroarylamino group. The number of carbon atoms is preferably 0 to 30, more preferably 1 to 25, still more preferably 2 to 20, and particularly preferably 2 to 16. Further, as the hetero ring, those in which the ring-constituting hetero atom is selected from an oxygen atom, a sulfur atom, and a nitrogen atom, and in terms of the number of ring members, 5- to 7-membered rings are preferable, and 5- or 6-membered rings are more preferable. Examples of the heterocyclic amino group include pyrrolidin-3-ylamino, imidazolidinylamino, benzimidazolidinylamino, piperidin-4-ylamino, and tetrahydrothiophen-3-ylamino.

Examples of the silyl group include an alkylsilyl group, a cycloalkylsilyl group, an arylsilyl group, an alkyloxysilyl group, a cycloalkyloxysilyl group, and an aryloxysilyl group. Preferred examples of the silyl group include an alkylsilyl group, a cycloalkylsilyl group, and an arylsilyl group. The number of carbon atoms in the silyl group is preferably 3 to 30, more preferably 3 to 24, still more preferably 3 to 20, and particularly preferably 3 to 18. Examples of the silyl group include trimethylsilyl, triethylsilyl, t-butyldimethylsilyl, cyclohexyldimethylsilyl, triisopropylsilyl, t-butyldiphenylsilyl, methyldimethoxysilyl, phenyldimethoxysilyl, and phenoxydimethylsilyl.

Examples of the silyloxy group include an alkylsilyloxy group, a cycloalkylsilyloxy group, and an arylsilyloxy group. The number of carbon atoms in the silyloxy group is preferably 3 to 30, more preferably 3 to 24, still more preferably 3 to 20, and particularly preferably 3 to 18. Examples of the silyloxy group include trimethylsilyloxy, triethylsilyloxy, t-butyldimethylsilyloxy, triisopropylsilyloxy, cyclohexyldimethylsilyloxy, and t-butyldiphenylsilyloxy.

The ligand L1 is preferably a tridentate ligand (terpyridine compound) represented by the following Formula (L1-2). This terpyridine compound can impart excellent photoelectric conversion efficiency to a photoelectric conversion element or a dye-sensitized solar cell when it is used in combination with the ligand L2 which will be described later as a ligand of a metal complex dye for use in a photoelectric conversion element or a dye-sensitized solar cell. Accordingly, this terpyridine compound is preferably used as a ligand of a dye-sensitized solar cell.

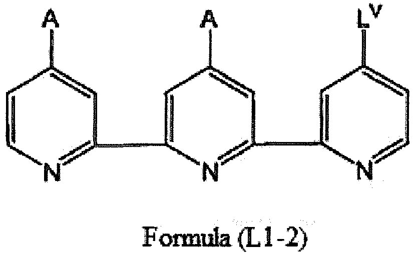

In Formula (L1-2), A represents an acidic group and has the same definition as the acidic group of Formula (L1-1), and the preferred examples thereof are also the same.

L^{V} has the same definition as L^{V} of Formula (L1-1).

For the terpyridine compound, L^{V} is preferably the group represented by Formula (LV-2), and R^{V3} is preferably a heteroaryl group.

The terpyridine compound is the ligand L1 itself, but in the present invention, the ligand L1 can also be used as a precursor compound of the ligand L1 which will be described later. Accordingly, in the present invention, the term, ligand L1, encompasses a precursor compound of the ligand L1, in addition to the ligand L1 itself (the terpyridine compound). Preferred examples of the precursor compound include an esterified product in which at least one of acidic groups A of the terpyridine compound is esterified (also referred to as an esterified product of a terpyridine compound).

This esterified product is a compound in which the acidic group is protected, which is an ester capable of being regenerated into an acidic group by hydrolysis or the like, and is not particularly limited. Examples thereof include an alkyl esterified product, an aryl esterified product, and a heteroaryl esterified product of the acidic group. Among these, the alkyl esterified product is preferable. The alkyl group which forms an alkyl esterified product is not particularly limited, but an alkyl group having 1 to 10 carbon atoms is preferable, and an alkyl group having 1 to 6 carbon atoms is more preferable, and an alkyl group having 1 to 4 carbon atoms is still more preferable. The aryl group which forms an aryl esterified product and the heteroaryl group which forms a heteroaryl esterified product are each not particularly limited, and examples thereof include the substituent group T which will be described later. These groups may have at least one substituent selected from the substituent group T which will be described later.

It is preferable that there are two esterified acidic groups. In this case, the two esters may be the same as or different from each other.

The ligand L1 can be synthesized with reference to various methods. For example, the ligand L1 represented by Formula (L1-6) can be synthesized by subjecting a compound represented by Formula (L1-3) and a compound represented by Formula (L1-4) to a coupling reaction, as shown in the following scheme, and hydrolyzing an ester group of a precursor represented by Formula (L1-5). In this synthesis method, an esterified product of a carboxy group is shown as the precursor compound, but the present invention is not limited thereto, and any of precursor compounds obtained by esterification of any one of the acidic groups may be used.

The coupling reaction herein can be carried out by, for example, "a Stille coupling reaction", a "Suzuki coupling method" described in "Experimental Chemistry Course, Fifth Edition", Maruzen Co., Ltd., edited by The Chemical Society of Japan, Vol. 13, pp. 92-117, or methods equivalent thereto. Further, the hydrolysis can be carried out in accordance with, for example, the method described in "Experimental Chemistry Course, Fifth Edition", Maruzen Co., Ltd., edited by The Chemical Society of Japan, Vol. 16, pp. 10-15.

In the present invention, the metal complex dye of the present invention can be synthesized using the ligand L1 synthesized by the hydrolysis of the precursor compound. Further, the metal complex dye of the present invention can also be synthesized by forming a metal complex dye using the precursor compound and then hydrolyzing ester groups in accordance with the above method, as in Example 1 which will be described later.

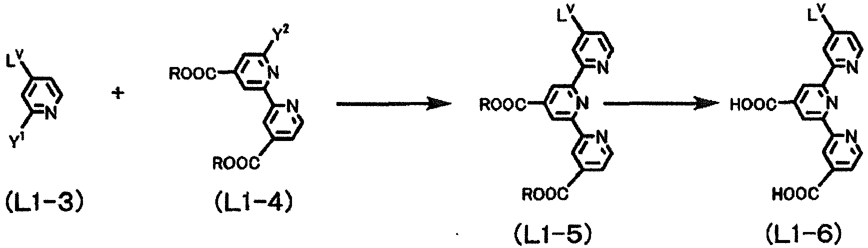

In Formula (L1-3), L^{V} has the same definition as L^{V} of Formula (L1-1). Y¹ represents a trialkyl tin group, a boronic acid group, a boronic acid ester group, a halogen atom, or a perfluoroalkylsulfonyloxy group.

In Formula (L1-4), in a case where Y¹ of Formula (L1-3) is a trialkyl tin group, a boronic acid group, or a boronic acid ester group, Y² represents a halogen atom or a perfluoroalkylsulfonyloxy group, and in a case where Y¹ of Formula (L1-3) is a halogen atom or a perfluoroalkylsulfonyloxy group, Y² represents a trialkyl tin group, a boronic acid group, or a boronic acid ester group.

In Formulae (L1-4) and (L1-5), R represents an alkyl group, an aryl group, or a heteroaryl group.

Specific examples of the ligand L1 are shown below. Examples of the ligand L1 also include the ligand L1 in the metal complex dye which will be described later. Other examples thereof include the ligands L1 in the following specific examples and specific examples of the metal complex dye, which are compounds in which at least one of -COOH's is formed into a salt of a carboxy group. In these compounds, examples of the counter cation that forms a salt of a carboxy group include the positive ions described in CI below. Further, examples of the esterified product of the terpyridine compound include the ligands L1 in the following specific examples and specific examples of the metal complex dye, which are compounds in which at least one of acidic groups is esterified. The present invention is not limited to these ligands L1, or salts or esterified products thereof. Me in the specific examples represents methyl.

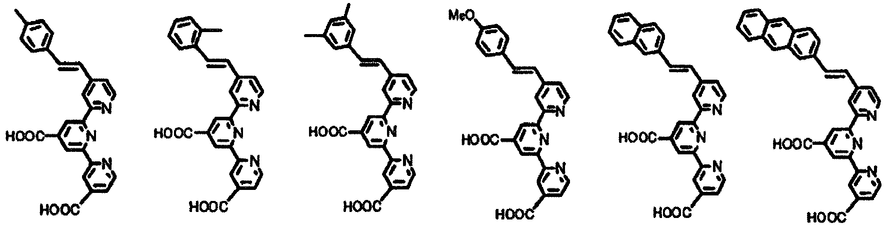

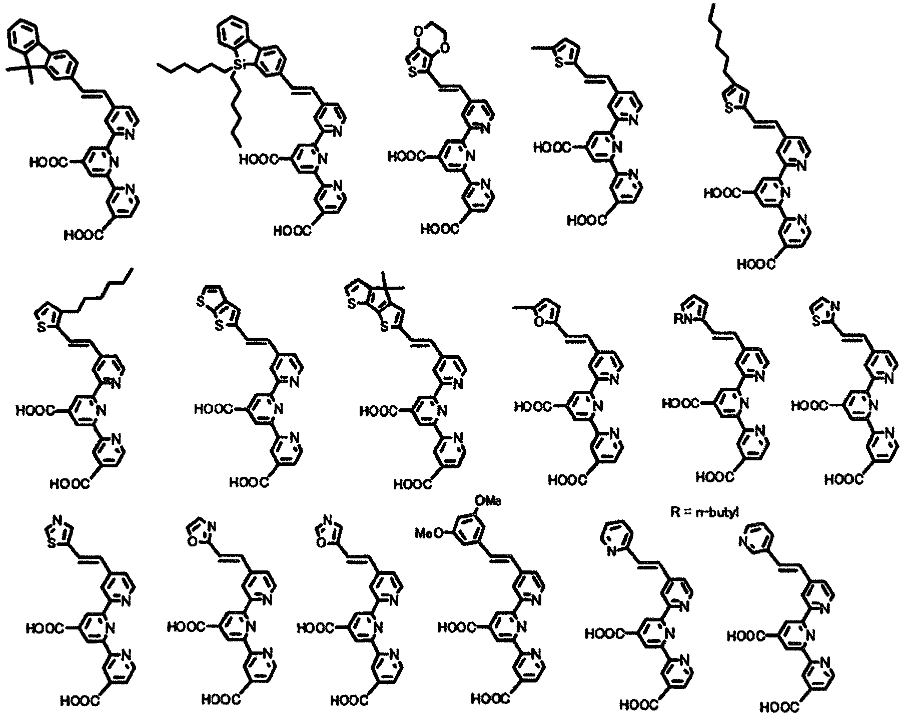

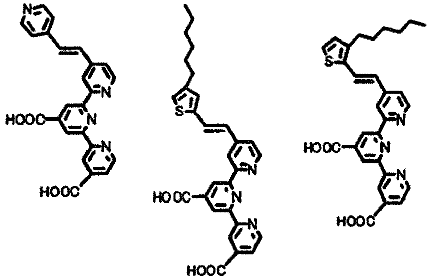

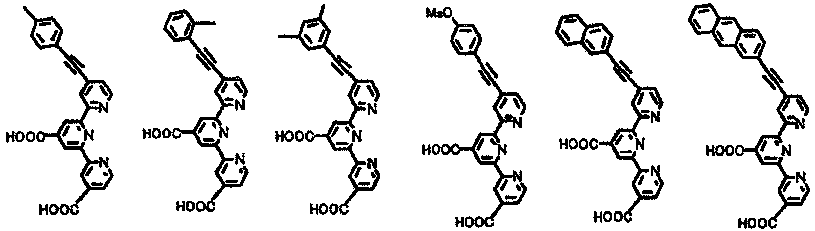

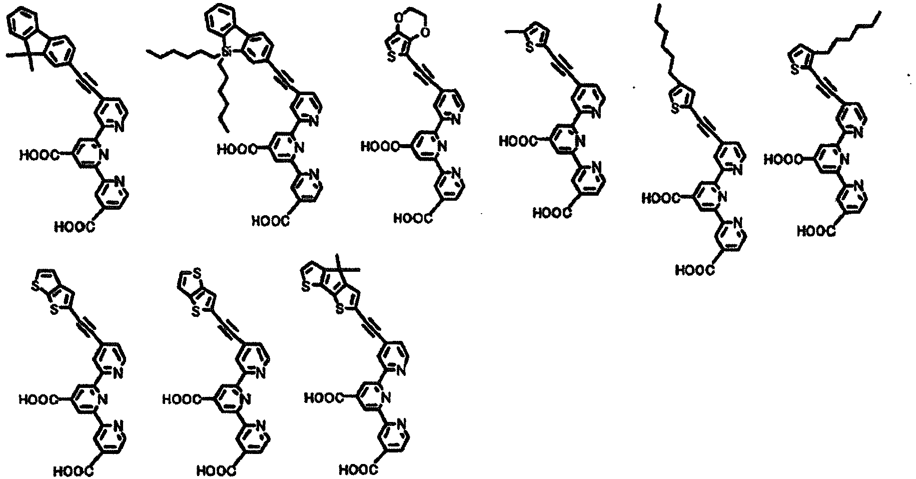

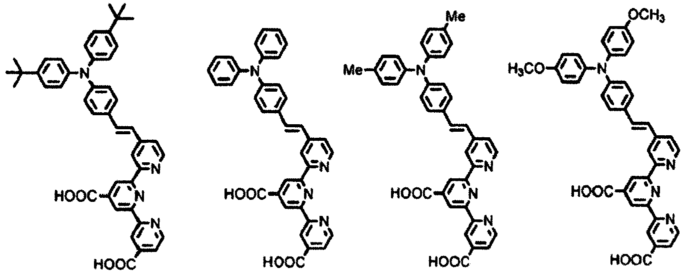

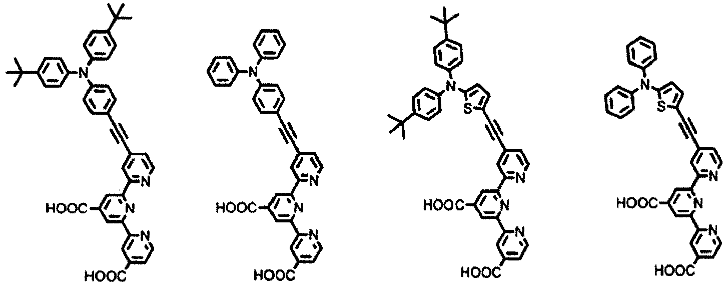

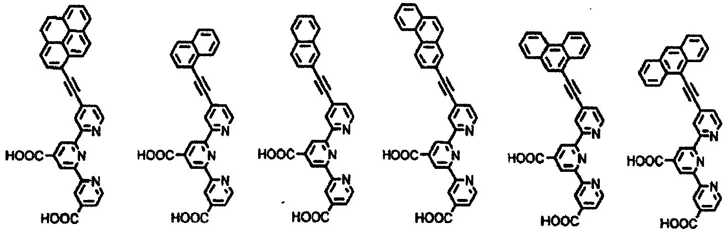

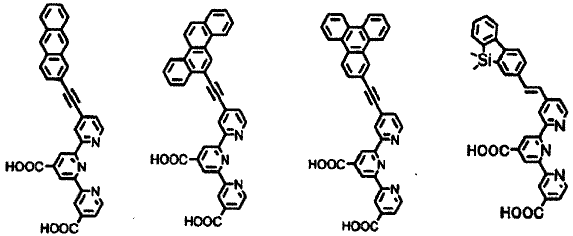

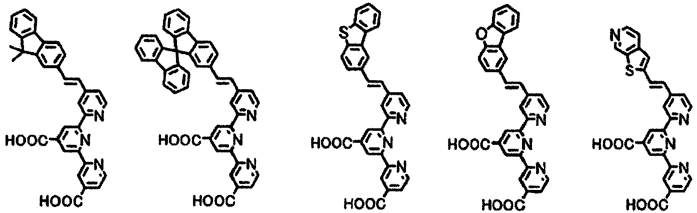

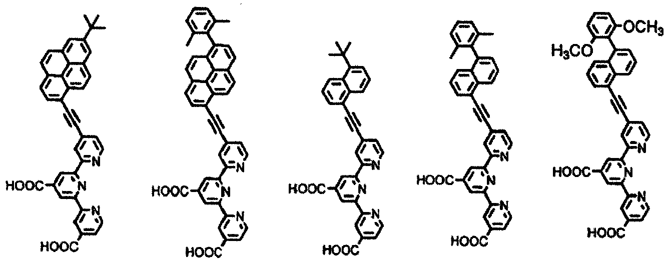

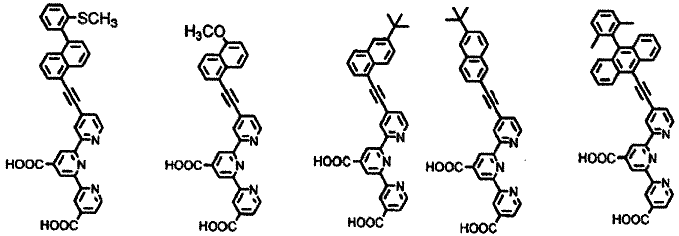

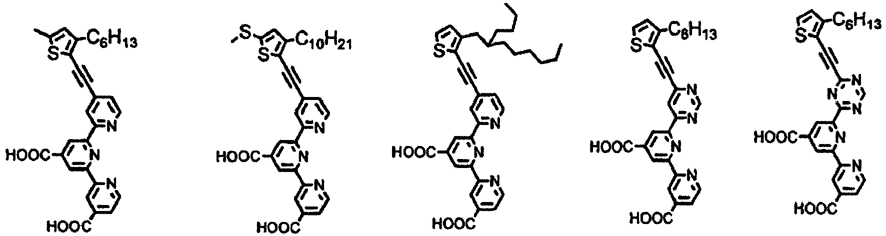

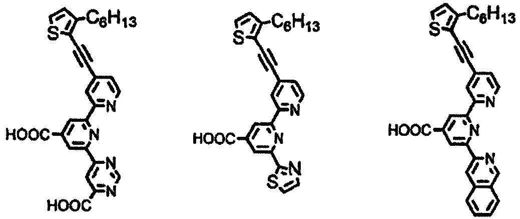

### - Ligand L2 -

The ligand L2 is a bidentate ligand represented by any one of the following Formula (L2-1) to Formula (L2-8), or a tridentate ligand different from the ligand L1. Here, in the ligand L2, at least one of the ring-constituting atoms bonded to the metal ion M is an anion. The expression, "being an anion", means that a hydrogen atom in the ring-constituting atom can be dissociated and bonded to a metal ion M. Examples of such an anion include carbon anions such as a =C⁻- ion, and nitrogen anions such as >N⁻ ion. If the metal complex dye has the ligand L2 which coordinates with the metal ion M with an anion of the ring-constituting atom, in combination with the ligand L1, the heat stability is improved and the durability becomes excellent.

Furthermore, the ligand L2 is a ligand having a ring having a ring-constituting nitrogen atom having lone electron pairs, in which at least one of the ring-constituting atoms bonded to the metal ion M is a ring-constituting nitrogen atom having lone electron pairs.

The ligand L2 is preferably a ligand which does not have an acidic group adsorbed on the surface of semiconductor fine particles. Even though a group corresponding to an acidic group is included in the ligand, the group is preferably not adsorbed on the surface of the semiconductor fine particles.

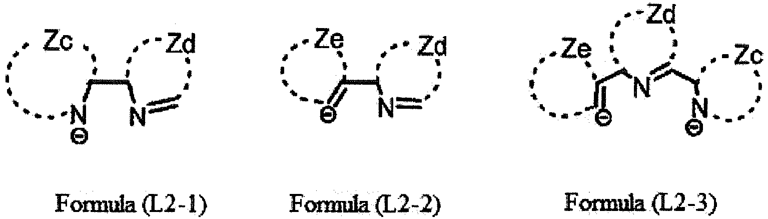

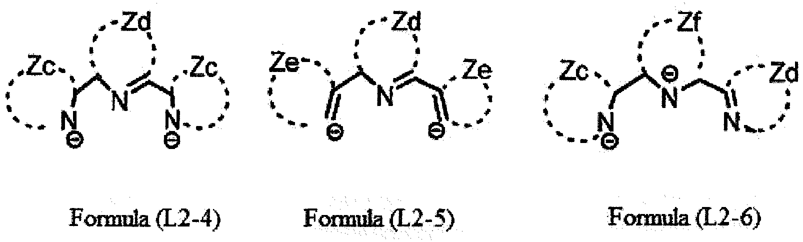

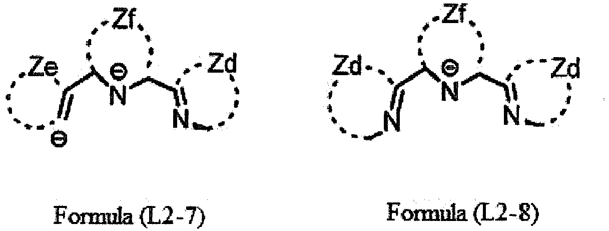

In Formulae (L2-1) to (L2-8), Zc, Zd, Ze, and Zf each independently represent a non-metal atomic group required for completing a 5- or 6-membered aromatic ring. Zc, Zd, Ze, and Zf are preferably a non-metal atomic group selected from a carbon atom, a nitrogen atom, an oxygen atom, a sulfur atom, and a phosphorus atom.

Examples of the aromatic ring formed by Zc, Zd, Ze, and Zf include, in addition to the respective groups exemplified as the rings formed by Za and Zb, a benzene ring, a pyrrole ring, and an indole ring. As the aromatic ring formed by Zc, Zd, Ze, and Zf, aromatic rings which are suitable for the structures of the respective rings represented by respective Formulae are preferably selected.

At least one of the aromatic rings formed by Zc, Ze, and Zf is an aromatic ring having a ring-constituting atom which becomes an anion. Examples of such an aromatic ring having a ring-constituting atom which becomes an anion include nitrogen-containing aromatic rings having a hydrogen atom bonded to ring-constituting nitrogen atom, such as a pyrrole ring, a pyrazole ring, an imidazole ring, a triazole ring, a benzimidazole ring, a benzotriazole ring, and an indole ring, and a benzene ring. Among those, a pyrrole ring, a pyrazole ring, a triazole ring, or a benzene ring is particularly preferable.

In the respective formulae, the ring formed by Zc is preferably the nitrogen-containing aromatic ring, more preferably a pyrrole ring or a pyrazole ring, and still more preferably a pyrazole ring.

The ring formed by Zd is a ring having a ring-constituting nitrogen atom having lone electron pairs, and preferably a ring in which a ring-constituting atom which becomes an anion does not coordinate to the metal ion M. The ring formed by Zd is not particularly limited as long as it is such a ring, and the ring is preferably the same as the ring formed by Za and Zb, and particularly preferably a pyridine ring.

The ring formed by Ze is preferably a benzene ring.

The ring formed by Zf is preferably a pyrrole ring, an imidazole ring, a benzimidazole ring, or an indole ring.

In the present invention, in view of excellent heat stability, the ligand L2 to be used in combination with the ligand L1 is preferably a ligand represented by any one of Formula (L2-1) to Formula (L2-5) among the respective formulae, more preferably a ligand represented by Formula (L2-1) or Formula (L2-4), and particularly preferably a ligand represented by Formula (L2-1).

In the ligand L2, the ring formed by Zd has an organic group R^{VL} represented by the following Formula (R^{VL}). If the ligand L2 to be used in combination with the ligand L1 has the organic group R^{VL} in the ring formed by Zd, the photoelectric conversion efficiency can be improved.

Formula (R^{VL}): -(R^{VL})n^{VL}

In the formula, R^{VL} is an organic group selected from the group G^{VL1} consisting of an aromatic ring group (R^{VL-1}), an alkyl group (R^{VL-2}), an alkenyl group (R^{VL-3}), an alkynyl group (R^{VL-4}), an alkylthio group (R^{VL-5}), an alkoxy group (R^{VL-6}), an amino group (R^{VL-7}), and a silyl group (R^{VL-8}).

Here, the aromatic ring group (k^{VL-1}) is a monocyclic ring group bonded to the ring formed by Zd or a polycyclic ring group including the monocycle as a fused ring, which is an aromatic ring group in which the sp² carbon atoms at the α-position with respect to the ring-constituting atom bonded to the ring formed by Zd in a case where the monocycle is a 5-membered ring, and the sp² carbon atoms at the α- and β-positions with respect to the ring-constituting atom bonded to the ring formed by Zd in a case where the monocycle is a 6-membered ring are all bonded to hydrogen atoms or ring-constituting atoms of a fused ring different from the monocycle.

The organic group R^{VL} selected from the group G^{VL1} may have a substituent. Such a substituent is not particularly limited, but examples thereof include a group selected from the substituent group T^{VL} which will be described later, or the respective groups included in the group G^{VL}. Preferred examples thereof include a substituent selected from the group consisting of an aromatic ring group, an alkyl group, an alkenyl group, an alkynyl group, an alkylthio group, an alkoxy group, and an amino group. These groups are the respective groups included in the group G^{VL}, and the aromatic ring group is preferably represented by any one of the following Formulae (S-1) to (S-4).

In a case where the organic group R^{VL} has a substituent, the number of the substituents is not particularly limited, but it is preferably 1 to 4, and more preferably 1 or 2. A plurality of substituents may be each bonded to the organic group R^{VL}, and may be bonded to the organic group R^{VL} as a binding substituent to which a plurality of substituents are bonded.

The organic group R^{VL} is preferably one which is directly bonded to the ring formed by Zd, but may be bonded through a linking group. The linking group may be a divalent group, and is preferably a group for bonding through π conjugation, and examples thereof ethenylene, ethynylene, an arylene group (preferably phenylene), and a heteroarylene group.

The number n^{VL} of the organic groups R^{VL} is an integer of 0 or more, and an integer that is not more than the number of hydrogen atoms when the ring formed by Zd is unsubstituted. n^{VL} is not particularly limited, but is preferably 1 or 2, and more preferably 1.

Examples of the aromatic ring group (R^{VL-1}) include an aromatic ring group formed of a monocycle bonded to the ring formed by Zd, and an aromatic ring group formed of a polycycle including the monocycle as a fused ring.

The monocycle is preferably a 5-membered ring or a 6-membered ring. Examples of the 5-membered ring include a thiophene ring, a furan ring, a pyrrole ring, a cyclopentadiene ring, a silole ring, a selenophene ring, a thiazole ring, an oxazole ring, an isothiazole ring, an isoxazole ring, an imidazole ring, a pyrazole ring, a thiadiazole ring, an oxadiazole ring, and a triazole ring. Examples of the 6-membered ring include a benzene ring, a pyridine ring, a pyrazine ring, a pyrimidine ring, a pyridazine ring, a triazine ring, and a tetrazine ring.

Examples of the polycycle include a monocycle bonded to the ring formed by Zd, and a ring formed by fusion of the monocycle with a different ring. The different ring is not particularly limited, and may be the same as or different from the monocycle bonded to the ring formed by Zd. Examples of the different ring include the 5-membered rings and the 6-membered rings exemplified as the monocycle.

The polycycle is not particularly limited as long as it is bonded to the ring formed by Zd as a 5-membered ring or a 6-membered ring, and examples thereof include the rings exemplified as the fused polycyclic aromatic ring group and the fused polycyclic heterocyclic group. Among those, suitable examples thereof include a naphthalene ring, a pyrene ring, a phenanthrene ring, an anthracene ring, a fluorene ring, a dibenzofuran ring, a dibenzothiophene ring, a dibenzopyrrole ring, a thienothiophene ring, a benzothiophene ring, a benzofuran ring, a benzopyrrole ring, a benzodithiophene ring, a benzodifuran ring, and a cyclopentadithiophene ring.

Among those, the monocycle or polycycle is more preferably a thiophene ring, a furan ring, a pyrrole ring, a cyclopentadiene ring, a silole ring, a benzene ring, a naphthalene ring, a dibenzothiophene ring, a pyrene ring, a fluorene ring, a benzothiophene ring, or a benzodithiophene ring, and still more preferably a thiophene ring, a furan ring, or a benzene ring.

The aromatic ring group (R^{VL-1}) is an aromatic ring group in which specific sp² carbon atoms in the ring-constituting atoms constituting the monocycle bonded to the ring formed by Zd in the monocycle or polycycle are bonded to hydrogen atoms or bonded to ring-constituting atoms of a fused ring different from the monocycle. That is, the aromatic ring group (R^{VL-1}) is an aromatic ring group other than an aromatic ring group in which the sp² carbon atoms all have a substituent which does not form a ring (including an aromatic ring and an aliphatic ring). In the present invention, the aromatic ring group (R^{VL-1}) makes it possible that the monocycle or polycycle is bonded to the ring formed by Zd through the specific ring-constituting atom such that the specific sp² carbon atoms satisfy the above conditions.

For such the aromatic ring group (R^{VL-1}), in a case where the monocycle is a 5-membered ring, all the sp² carbon atoms at the α-position with respect to the ring-constituting atom bonded to the ring formed by Zd among the ring-constituting atoms of the monocycle are all bonded to hydrogen atoms or to ring-constituting atoms of a fused ring different from the monocycle.

In a case where the monocycle is a 6-membered ring, all the sp² carbon atoms the α-and β-positions with respect to the ring-constituting atom bonded to the ring formed by Zd among the ring-constituting atoms of the monocycle are all bonded to hydrogen atoms or to ring-constituting atoms of a fused ring different from the monocycle.

In the monocycle or polycycle, the ring-constituting atom at the α-position in the 5-membered ring and the ring-constituting atoms at the α- and β-positions in the 6-membered ring are not sp² carbon atoms, a substituent may be bonded thereto.

The aromatic ring group (R^{VL-1}) is preferably a ring group represented by any one of the following Formulae (S-1) to (S-4).

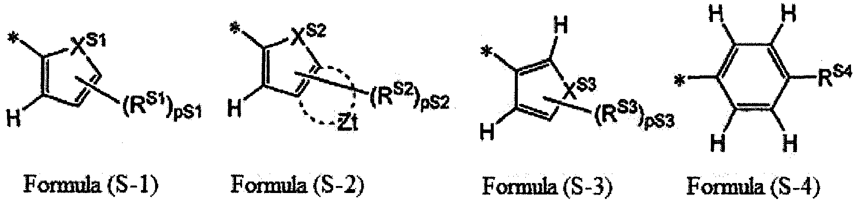

In the formula, * represents a bonding moiety to the ring formed by Zd.

X^{S1} to X^{S3} each independently represent a -O-, -S-, -NR^{S}-, -C(R^{S})₂-, or -Si(R^{S})₂-, and it is preferably -O- or -S-, and more preferably -S-.

R^{S}'s each represent a hydrogen atom or a substituent. This substituent is not particularly limited, and examples thereof include a group (excluding an acidic group) selected from the substituent group T which will be described later.

Zt represents a non-metal atomic group required for forming a fused ring with a ring including X^{S2}. Specifically, Zt is selected from an atomic group consisting of a carbon atom, a nitrogen atom, an oxygen atom, a sulfur atom, and a phosphorus atom. The number of the fused rings formed by Zt is not particularly limited as long as it is 1 or more, but is preferably a number with which the polycycle can be formed, and preferably 1 or 2.

R^{S1} to R^{S4} each independently represent a substituent. This substituent a group (excluding an acidic group) selected from the substituent group T which will be described later, or the respective groups included in the group G^{VL}. Among those, a substituent selected from the group consisting of an alkyl group, an alkenyl group, an alkynyl group, an alkylthio group, an alkoxy group, and an amino group, among the respective groups include in the group G^{VL} is preferable.

pS1 represents an integer of 0 to 2, preferably 0 or 1, and more preferably 1.

pS2 is an integer of 0 or more, which is not more than the number of hydrogen atoms when the fused ring formed by Zt is unsubstituted. It is preferably an integer of 0 to 4, more preferably 0 to 2, and still more preferably 1.

pS3 represents 0 or 1, and is preferably 1.

The ring group represented by Formula (S-4) is a phenyl group which may be substituted only at the 4-position.

Examples of the phenyl group which may be substituted only at the 4-position include a phenyl group and a phenyl group which is substituted only at the 4-position. The substituent R^{S4} for substitution at the 4-position of the phenyl group is as described above.

The alkyl group (R^{VL-2}) selected as the organic group R^{VL} may be any one of a linear alkyl group, a branched alkyl group, and a cycloalkyl group.

The number of carbon atoms in the linear alkyl group and the branched alkyl group is preferably 1 to 30, more preferably 4 to 30, still more preferably 5 to 26, and particularly preferably 6 to 20. Examples of the alkyl group include methyl, ethyl, n-butyl, t-butyl, n-pentyl, n-hexyl, n-octyl, 2-ethylhexyl, 3-ethylheptyl, n-decyl, 3,7-dimethyloctyl, isodecyl, s-decyl, n-dodecyl, 2-butyloctyl, n-hexadecyl, isohexadecyl, n-eicosy, n-hexacosyl, and isooctacosyl.

The number of carbon atoms in the cycloalkyl group is preferably 3 to 30, more preferably 5 to 30, still more preferably 6 to 26, and particularly preferably 6 to 20. Examples of the cycloalkyl group include cyclopropyl, cyclopentyl, cyclohexyl, cycloheptyl, and cyclooctyl. The cycloalkyl group may also be fused with an aliphatic ring, an aromatic ring, or a hetero ring.

In the present invention, the alkyl group may have a substituent, but it does not include a halogen-substituted alkyl group which is substituted with a halogen atom. Examples of the halogen-substituted alkyl group include the halogen-substituted alkyl groups which will be described later as a substituent which a ring formed by each of Zc, Ze, and Zf may have.

The number of carbon atoms in the alkenyl group (R^{VL-3}) is preferably 2 to 30, more preferably 4 to 30, still more preferably 5 to 26, and particularly preferably 6 to 20. Examples of the alkenyl group (R^{VL-3}) include vinyl, allyl, hexenyl, octenyl, and oleyl.

The number of carbon atoms in the alkynyl group (R^{VL-4}) is preferably 2 to 30, more preferably 4 to 30, still more preferably 5 to 26, and particularly preferably 6 to 20. Examples of the alkynyl group (R^{VL-4}) include ethynyl, butynyl, hexynyl, octynyl, and phenylethynyl.

The alkylthio group (R^{VL-5}) may be any one of a linear alkylthio group, a branched alkylthio group, and a cycloalkylthio group. The alkyl moiety of the alkylthio group has the same definition as the alkyl group, and the preferred examples thereof are also the same.

Examples of the linear alkylthio group and the branched alkylthio group include methylthio, ethylthio, n-propylthio, i-propylthio, n-butylthio, t-butylthio, n-pentylthio, n-hexylthio, n-octylthio, 2-ethylhexylthio, 3,7-dimethyloctylthio, n-decylthio, isodecylthio, s-decylthio, n-dodecylthio, 2-butyloctylthio, 3-butyloctylthio, n-hexadecylthio, isohexadecylthio, n-eicosythio, n-hexacosylthio, and isooctacosylthio. Examples of the cycloalkylthio group include cyclopropylthio, cyclopentylthio, cyclohexylthio, cycloheptylthio, and cyclooctylthio.

The alkoxy group (R^{VL-6}) may be any one of a linear alkoxy group, a branched alkoxy group, and a cycloalkoxy group. The alkyl moiety of the alkoxy group has the same definition as the alkyl group, and the preferred examples thereof are also the same.

Examples of the linear alkoxy group and the branched alkoxy group include methoxy, ethoxy, n-propoxy, i-propoxy, n-butoxy, t-butoxy, n-pentoxy, n-hexyloxy, n-octyloxy, 2-ethylhexyloxy, 3,7-dimethyloctyloxy, n-decyloxy, isodecyloxy, s-decyloxy, 2-butyloctyloxy, 3-butyloctyloxy, n-dodecyloxy, n-hexadecyloxy, isohexadecyloxy, n-eicosyoxy, n-hexacosyloxy, and isooctacosyloxy. Examples of the cycloalkoxy group include cyclopropyloxy, cyclopentyloxy, cyclohexyloxy, cycloheptyloxy, and cyclooctyloxy.

Examples of the amino group (R^{VL-7}) include an alkylamino group, an arylamino group, and a heteroarylamino group, in addition to an amino group(-NH₂).

Here, examples of the alkylamino group include an N-alkylamino group and an N,N-dialkylamino group. Examples of the arylamino group include an N-arylamino group, an N-alkyl-N-arylamino group, and an N,N-diarylamino group. Examples of the heteroarylamino group include an N-heteroarylamino group, an N-alkyl-N-heteroarylamino group, an N-aryl-N-heteroarylamino group, and an N,N-diheteroarylamino group.

The number of carbon atoms in the alkyl group to be substituted at a nitrogen atom in the respective amino groups is preferably 1 to 24, and more preferably 4 to 12. Further, the alkyl group may be either linear or branched. Examples of the alkyl group include methyl, ethyl, isopropyl, n-butyl, t-butyl, isobutyl, n-hexyl, n-octyl, 2-ethylhexyl, 3,7-dimethyloctyl, 2-butyloctyl, n-dodecyl, n-hexadecyl, and 2-hexyldecyl, and preferably t-butyl, n-hexyl, 2-ethylhexyl, and n-octyl.

The number of carbon atoms in the aryl group to be substituted at a nitrogen atom in the respective amino groups is preferably 6 to 24, and more preferably 6 to 18. Examples of the aryl group include phenyl and naphthyl, and preferably a phenyl group. Further, the aryl group may also be fused with an aliphatic ring, an aromatic ring, a hetero ring, or the like.

The number of carbon atoms in the heteroaryl group to be substituted at a nitrogen atom in the respective amino groups is preferably 0 to 24, and more preferably 1 to 18. The hetero ring in the heteroaryl group is preferably a 5-membered ring or a 6-membered ring, the hetero atom constituting the hetero ring is preferably a nitrogen atom, an oxygen atom, or a sulfur atom, and examples thereof include a thiophene ring, a furan ring, a pyrrole ring, a pyrazole ring, an imidazole ring, a triazole ring, an oxazole ring, a thiazole ring, a pyridine ring, a pyrazine ring, a pyrimidine ring, and a pyridazine ring. Further, the heteroaryl ring may also be fused with an aliphatic ring, an aromatic ring, a hetero ring, or the like.

In the case of an N,N-di-substituted amino group at a nitrogen atom of the respective amino groups, two substituents may not be bonded to each other or may be bonded to each other to form a ring.

Examples of the amino group, the alkylamino group, the arylamino group, or the heteroarylamino group include amino(-NH₂), methylamino, ethylamino, n-hexylamino, 2-ethylhexylamino, n-octadecylamino, N,N-dimethylamino, N,N-diethylamino, N,N-bis(n-hexyl)amino, N-methyl-N-n-hexylamino, N,N-bis(2-ethylhexyl)amino, phenylamino, N,N-diphenylamino, N-naphthylamino, N-methyl-N-phenylamino, N-imidazolylamino, pyrrolylamino, and thienylamino.

Among the amino group, the alkylamino group, the arylamino group, and the heteroarylamino group, the alkylamino group, the arylamino group, and the heteroarylamino group are preferable.

The amino group is preferably a group represented by the following Formula (AM).

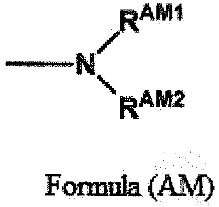

In the formula, R^{AM1} and R^{AM2} each independently represent an alkyl group or an aryl group. It is preferable that any one of R^{AM1} and R^{AM2} is aryl group. R^{AM1} and R^{AM2} may not be bonded to each other or may be bonded to each other to form a ring.

The amino group having a ring formed by the mutual bonding of R^{AM1} and R^{AM2} is preferably the following groups.

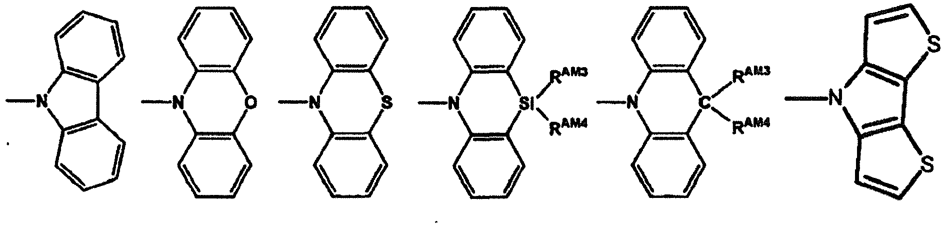

Here, R^{AM3} and R^{AM4} each independently represent an alkyl group or an aryl group.

The amino group (R^{VL-7}) may have a substituent (an amino group having a substituent may also be referred to as a group including an amino group). The substituent which the amino group may have is as described above. Such the amino group having a substituent is more preferably a group formed by combination of an amino group with an alkyl group or an alkoxy group. The group including an amino group is preferably one which is bonded to the ligand L2 at a nitrogen atom of the amino group. If R^{VL} of the ligand L2 used in combination with the ligand L1 is a group including an amino group, the absorption region of the metal complex dye extends up to a long-wavelength range, and the photoelectric conversion efficiency becomes excellent.

The group including an amino group is preferably a group formed by substituting an alkyl group, an aryl group, or a heteroaryl group bonded to a nitrogen atom of the amino group with a group selected from the group G^{VL}. The group formed by combination of an amino group with an alkyl group or an alkoxy group is a group formed by further substituting an alkyl group, an aryl group, or a heteroaryl group bonded to a nitrogen atom of the amino group with an alkyl group or an alkoxy group. The amino group in a group having an amino group has the same definition as the amino group, and the preferred examples thereof are also the same. Further, the alkyl group and the alkoxy group have the same definitions as the alkyl group and the alkoxy group of the organic group R^{VL}, respectively, and the preferred examples thereof are also the same.

The group formed by combination with an amino group with an alkyl group or an alkoxy group is preferably a group formed by substituting at least one of R^{AM1} or R^{AM2} in the group represented by Formula (AM) with an alkyl group or an alkoxy group. Examples thereof include N,N-di(4-alkylphenyl)amino, N,N-di(4-alkoxyphenyl)amino, and N,N-di(4-alkylthienyl)amino, and preferably N,N-di(4-methylphenyl)amino, N,N-di(4-(t-butyl)phenyl)amino, N,N-di(4-(n-hexyl)phenyl)amino, N,N-di(4-(n-octyloxy)phenyl)amino, and N,N-di(4-(n-hexyl)thienyl)amino.

The group formed by combination with an amino group with an alkyl group or an alkoxy group may be bonded to R^{AM1} and R^{AM2} to form a ring.

Preferred examples of the silyl group (R^{VL-8}) include the silyl groups described for the substituent group T which will be described later.

Among those described above, R^{VL} is preferably an organic group selected from the group G^{VL2} consisting of a ring group represented by Formula (S-1), a ring group represented by Formula (S-2), a ring group represented by Formula (S-3), a ring group represented by Formula (S-4), an alkyl group, an alkenyl group, an alkynyl group, an alkylthio group, an alkoxy group, and an amino group in views of photoelectric conversion efficiency and durability. R^{VL} is more preferably the ring group represented by Formula (S-1), the ring group represented by Formula (S-2), the ring group represented by Formula (S-3), the ring group represented by Formula (S-4), an alkyl group, an alkoxy group, or an amino group, more preferably the ring group represented by any one of Formula (S-1), Formula (S-2), Formula (S-3), and Formula (S-4), or an amino group, and particularly preferably an amino group (a group having an amino group) having the substituent.

For the ring formed by Zd, the position at which R^{VL} is bonded (substitution position) is not particularly limited as long as the ring has at least one R^{VL}. In a case where the ring formed by Zd is a 5-membered ring, the 3-position with respect to the ring-constituting nitrogen atom which coordinates to the metal atom M is preferable. In a case where the ring formed by Zd is a 6-membered ring, the 3- or 4-position with respect to the ring-constituting nitrogen atom which coordinates to the metal atom M is preferable, and the 4-poition is more preferable.

The ring formed by Zd may have a substituent other than the group R^{VL}. Examples of such a substituent include a group selected from the substituent group T^{VL} (excluding the group R^{VL}) which will be described later. In a case where a ring group directly bonded to the ring formed by Zd is included as a substituent, the ring-constituting atom adjacent to the ring-forming atom bonded to the ring formed by Zd in this ring group may not have a substituent.

The aromatic ring formed by each of Zc, Ze, and Zf may have a substituent. Such a substituent is not particularly limited, and is preferably an electron-withdrawing group. Here, the "electron-withdrawing group" refers to a group which has a Hammett's substituent constant is a positive integer. The electron-withdrawing group is more preferably a halogen atom, a halogen-substituted alkyl group, a halogen-substituted aryl group, a cyano group, an alkylsulfonyl group, an arylsulfonyl group, an aminosulfonyl group, an alkylsulfoxide group, an arylsulfoxide group, an aminosulfoxide group, an alkylcarbonyl group, or an aminocarbonyl group, and still more preferably a halogen atom, a halogen-substituted alkyl group, a halogen-substituted aryl group, a cyano group, an alkylsulfonyl group, an arylsulfonyl group, an aminosulfonyl group, an alkylcarbonyl group, or an aminocarbonyl group. Among those, the halogen-substituted alkyl group or the halogen-substituted aryl group is particularly preferable.

The alkyl moiety and the aryl moiety included in the electron-withdrawing group is not particularly limited, but preferably has the same definition as the alkyl group and the aryl group of the substituent group T which will be described later.

As the halogen-substituted alkyl group, a fluorine-substituted alkyl group having 1 to 30 carbon atoms is preferable, a fluorine-substituted alkyl group having 1 to 6 carbon atoms is more preferable, a fluorine-substituted alkyl group having 1 carbon atom is still more preferable, and a trifluoromethyl group is particularly preferable.

As the halogen-substituted aryl group, a phenyl group having 1 to 5 halogen atoms substituted therein is preferable, and a phenyl group having 1 to 4 halogen atoms substituted therein is more preferable.

The ligand L2 can be synthesized by, for example, the method described in JP2013-084594A, the method described in Angew. Chem. Int. Ed., 2011, 50, pp. 2054-2058, the method described in Energy Environ. Sci., 2012, 5, pp. 7549-7554, the method described in each specification of US2013/0018189A1, US2012/0073660A1 and US2012/0111410A1, the above patent documents regarding solar cells, or methods equivalent thereto.

Specific examples of the ligand L2 are shown below. The ligand L2 in metal complex dye which will be described later is also exemplified as the ligand L2. The present invention is not limited to these ligands L2.

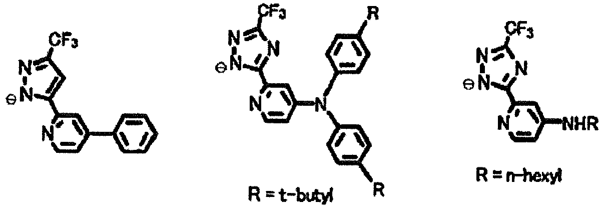

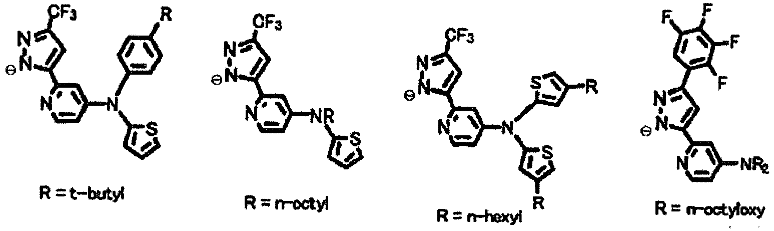

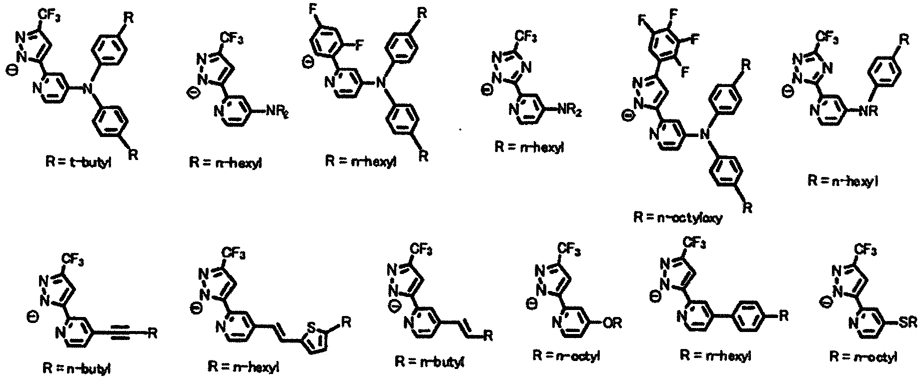

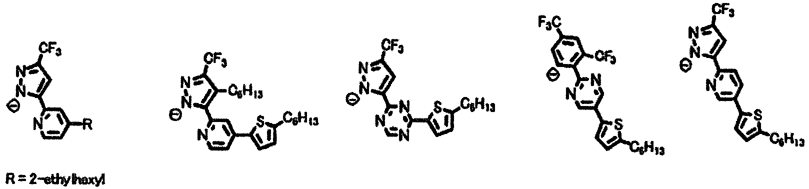

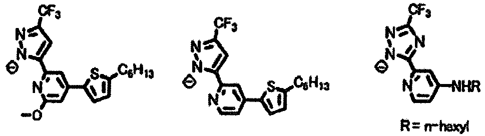

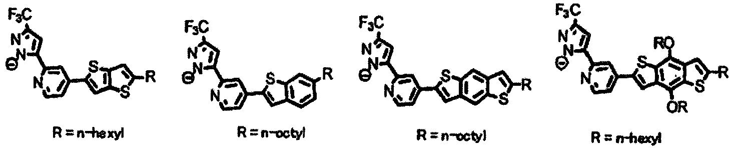

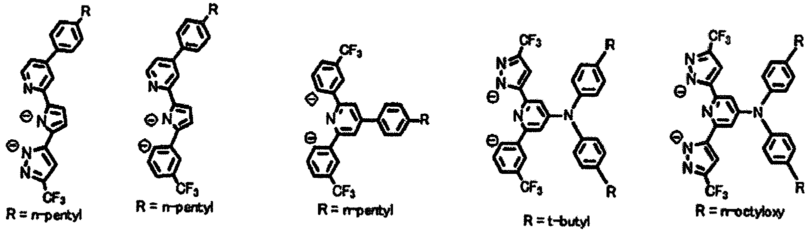

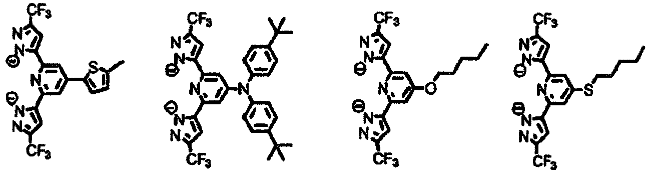

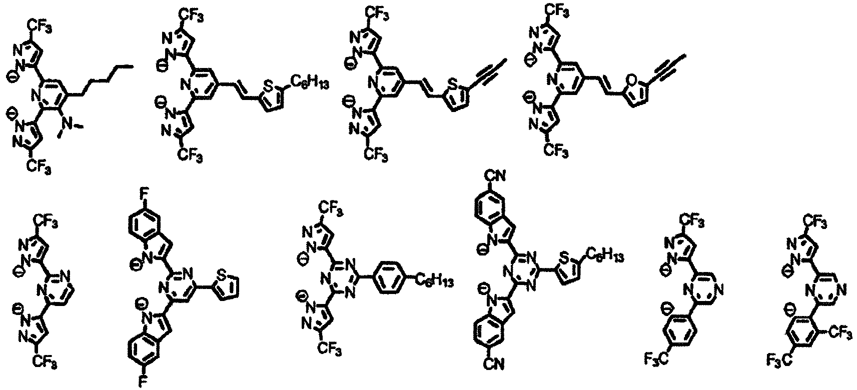

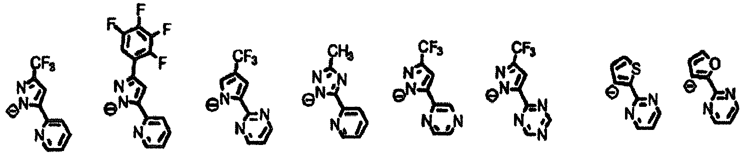

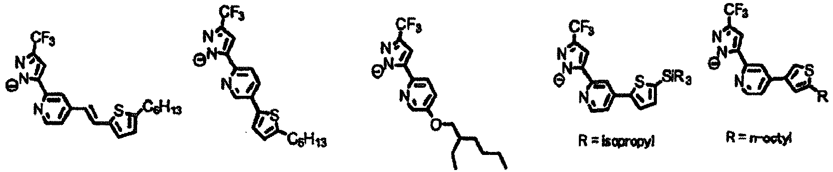

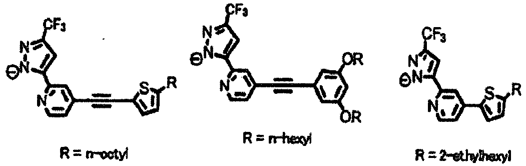

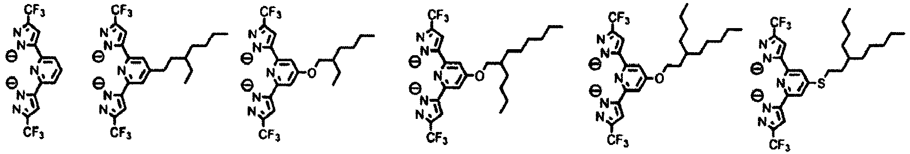

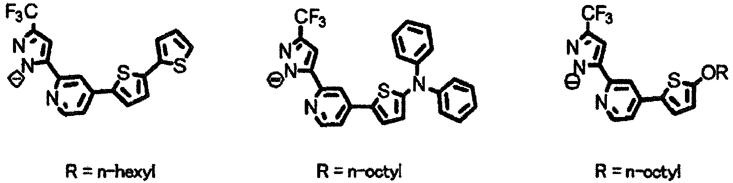

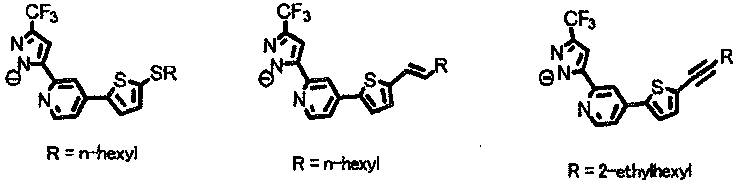

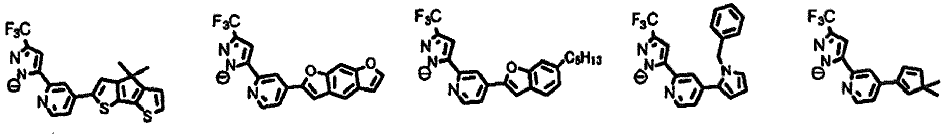

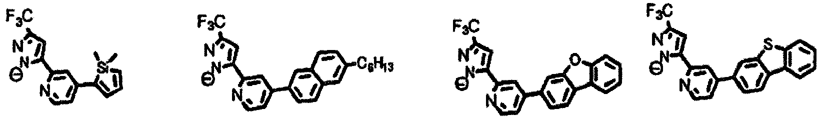

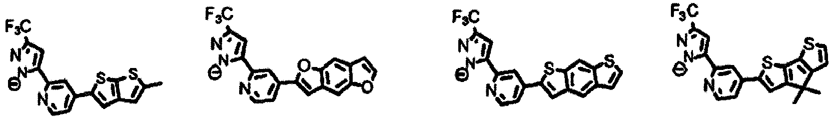

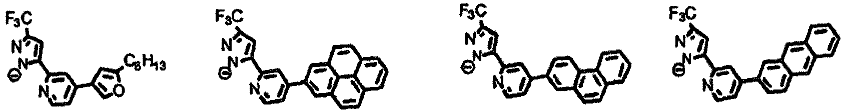

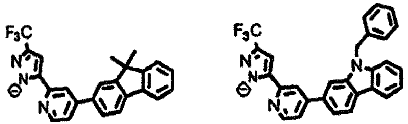

### - Ligand X -

The ligand X may be a monodentate ligand, and is preferably, for example, a group or atom selected from the group consisting of an acyloxy group, an acylthio group, a thioacyloxy group, a thioacylthio group, an acylaminooxy group, a thiocarbamate group, a dithiocarbamate group, a thiocarbonate group, a dithiocarbonate group, a trithiocarbonate group, an acyl group, a thiocyanate group, an isothiocyanate group, a cyanate group, an isocyanate group, a cyano group, an alkylthio group, an arylthio group, an alkoxy group, an aryloxy group, and a halogen atom, or anions thereof.

In a case where the ligand X includes an alkyl group, an alkenyl group, an alkynyl group, an alkylene group, or the like, these may be linear or branched, and may or may not have a substituent. Further, in a case where an aryl group, a heterocyclic group, a cycloalkyl group, or the like is included, these may or may not have a substituent, and may be a monocycle or be fused to form a ring.

Among those, the ligand X is preferably a cyanate group, an isocyanate group, a thiocyanate group, or an isothiocyanate group, or an anion thereof, more preferably an isocyanate group (an isocyanate anion) or an isothiocyanate group (an isothiocyanate anion), and particularly preferably an isothiocyanate group (NCS) (an isothiocyanate anion).

### - Counterion CI for Neutralizing Charge -

CI represents a counterion in a case where the counterion is required to neutralize charges. Generally, whether the metal complex dye is cationic or anionic or whether the metal complex dye has a net ionic charge depends on the metal, the ligand, and the substituent in the metal complex dye.

When the substituent has a dissociative group or the like, the metal complex dye may have a negative charge arising from dissociation. In this case, an electric charge of the metal complex dye as a whole is electrically neutralized by CI.

In a case where the counterion CI is a positive counterion, the counterion CI is, for example, an inorganic or organic ammonium ion (for example, a tetraalkyl ammonium ion and a pyridinium ion), a phosphonium ion (for example, a tetraalkylphosphonium ion and an alkyltriphenylphosphonium ion), an alkali metal ion (a Li ion, a Na ion, a K ion, and the like), an alkaline earth metal ion, a metal complex ion, or a proton. As the positive counterion, an inorganic or organic ammonium ion (triethylammonium ion, a tetrabutylammonium ion, a tetrahexylammonium ion, a tetraoctylammonium ion, a tetradecylammonium ion, and the like), an alkali metal ion, and a proton are preferable, and from the viewpoint of durability, an alkali metal ion and a proton are more preferable.

In a case where the counterion CI is a negative counterion, the counterion CI is, for example, an inorganic anion or an organic anion. Examples thereof include a hydroxide ion, a halogen anion (for example, a fluoride ion, a chloride ion, a bromide ion, and an iodide ion), a substituted or unsubstituted alkylcarboxylate ion (for example, an acetate ion and trifluoroacetate ion), a substituted or unsubstituted arylcarboxylate ion (for example, a benzoate ion), a substituted or unsubstituted alkylsulfonate ion (for example, methanesulfonate ion and a trifluoromethanesulfonate ion), a substituted or unsubstituted arylsulfonate ion (for example, a p-toluene sulfonate ion and a p-chlorobenzene sulfonate ion), an aryldisulfonate ion (for example, a 1,3-benzene disulfonate ion, a 1,5-naphthalene disulfonate ion, and a 2,6-naphthalene disulfonate ion), an alkylsulfate ion (for example, a methylsulfate ion), a sulfate ion, a thiocyanate ion, a perchlorate ion, a tetrafluoroborate ion, a hexafluorophosphate ion, and a picrate ion. Alternatively, as a charge balance counterion, an ionic polymer or another dye with an opposite charge from the dye in interest may be used, or a metal complex ion (for example, a bisbenzene-1,2-dithiolatonickel (III)) may also be used. As the negative counterion, a halogen anion, a substituted or unsubstituted alkylcarboxylate ion, a substituted or unsubstituted alkylsulfonate ion, a substituted or unsubstituted arylsulfonate ion, an aryldisulfonate ion, a perchlorate ion, and a hexafluorophosphate ion are preferable, and a halogen anion and a hexafluorophosphate ion are more preferable.

### - Metal Complex Dye -

In the metal complex dye represented by Formula (1), the ligand L1, the ligand L2, and the ligand X are as described above, and the combination of these ligands is not particularly limited. A preferred combination of the ligands is a combination of the preferred ligand L1, the preferred ligand L2, and the preferred ligand X.

The metal complex dye represented by Formula (1) is preferably a metal complex dye represented by any one of the following Formulae (2) to (6).

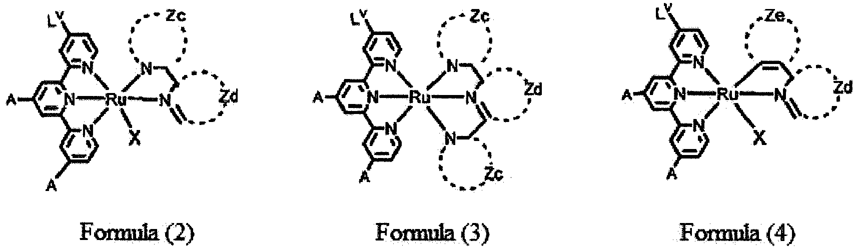

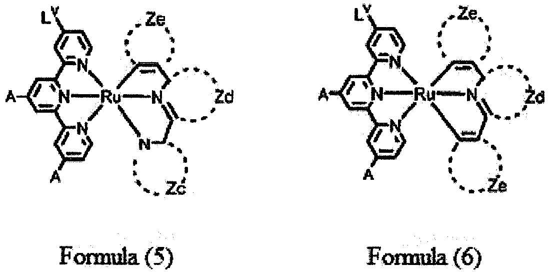

In Formulae (2) to (6), X has the same definition as X in Formula (1), a preferred range thereof is also the same. Zc, Zd, and Ze have the same definition of Zc, Zd, and Ze, respectively, in Formulae (L2-1) to (L2-5), and preferred ranges thereof are also the same. L^{V} has the same definition as L^{V} in Formula (L1-1), and a preferred range thereof is also the same. A represents an acidic group and has the same definition as the acidic group in Formula (L1-1), and the preferred examples thereof are also the same.

In the present invention, among the preferred metal complex dyes represented by the respective Formulae (2) to (6), the metal complex dye represented by Formula (2) and the metal complex dye represented by Formula (3) are more preferable.

The metal complex dye represented by Formula (1) can be synthesized by, for example, the method described in JP2013-084594A, the method described in JP4298799B, the method described in each specification of US2013/0018189A1, US2012/0073660A1, US2012/0111410A1, and US2010/0258175A1, the method described in Angew. Chem. Int. Ed., 2011, 50, pp. 2054-2058, the methods described in the reference documents listed in these documents, the patent documents regarding solar cells, known methods, or the methods equivalent thereto.

The metal complex dye represented by Formula (1) has a maximum absorption wavelength in a solution, preferably in a range from 300 to 1,000 nm, more preferably in a range from 350 to 950 nm, and particularly preferably in a range from 370 to 900 nm.

### <Substituent Group T>

In the present invention, preferred examples of the substituent include the groups selected from the following substituent group T.

Incidentally, in the present specification, a case where there is only a simple description of a substituent is intended to refer to this substituent group T, and further, in a case where each of the groups, for example, an alkyl group is merely described, a preferable range and specific examples for the corresponding group for the substituent group T are applied.

Moreover, in the present specification, in a case where an alkyl group is described as different from a cycloalkyl group (for example, the description of the substituents that can be adopted as the substituent T^{VA}), the alkyl group is used to mean inclusion of both of a linear alkyl group and a branched alkyl group. On the other hand, in a case where an alkyl group is not described as different from a cycloalkyl group (a case where an alkyl group is simply described, for example, an alkyl group (R^{VL-2}) that can be adopted as an organic group R^{VL}), and unless otherwise specified, the alkyl group is used to mean any of a linear alkyl group, a branched alkyl group, and a cycloalkyl group. This shall apply to a group (an alkoxy group, an alkylthio group, an alkenyloxy group, and the like) including a group (an alkyl group, an alkenyl group, an alkynyl group, and the like) which can adopt a cyclic structure, and a compound (the alkyl esterified product and the like) including a group which can adopt a cyclic structure. In the following description of the substituent group T, for example, a group with a linear or branched structure and a group with a cyclic structure may be sometimes separately described for clarification of both groups, as in the alkyl group and the cycloalkyl group.

Examples of the groups included in the substituent group T include the following groups or the groups formed by combination of a plurality of the following groups:
an alkyl group (preferably an alkyl group having 1 to 20 carbon atoms, for example, methyl, ethyl, isopropyl, t-butyl, pentyl, heptyl, 1-ethylpentyl, benzyl, 2-ethoxyethyl, 1-carboxymethyl, and trifluoromethyl), an alkenyl group (preferably an alkenyl group having 2 to 20 carbon atoms, for example, vinyl, allyl, and oleyl), an alkynyl group (preferably an alkynyl group having 2 to 20 carbon atoms, for example, ethynyl, butynyl, and phenylethynyl), a cycloalkyl group (preferably a cycloalkyl group having 3 to 20 carbon atoms, for example, cyclopropyl, cyclopentyl, cyclohexyl, and 4-methylcyclohexyl), an cycloalkenyl group (preferably a cycloalkenyl group having 5 to 20 carbon atoms, for example, cyclopentenyl and cyclohexenyl), an aryl group (preferably an aryl group having 6 to 26 carbon atoms, for example, phenyl, 1-naphthyl, 4-methoxyphenyl, 2-chlorophenyl, 3-methylphenyl, difluorophenyl, and tetrafluorophenyl), a heterocyclic group (preferably a heterocyclic group having 2 to 20 carbon atoms, more preferably a 5- or 6-membered heterocyclic group having at least one oxygen atom, sulfur atom, or nitrogen atom, for example, 2-pyridyl, 4-pyridyl, 2-imidazolyl, 2-benzimidazolyl, 2-thiazolyl, and 2-oxazolyl), an alkoxy group (preferably an alkoxy group having 1 to 20 carbon atoms, for example, methoxy, ethoxy, isopropyloxy, and benzyloxy), an alkenyloxy group (preferably an alkenyloxy group having 2 to 20 carbon atoms, for example, vinyloxy and allyloxy), an alkynyloxy group (preferably an alkynyloxy group having 2 to 20 carbon atoms, for example, 2-propynyloxy and 4-butynyloxy), a cycloalkyloxy group (preferably a cycloalkyloxy group having 3 to 20 carbon atoms, for example, cyclopropyloxy, cyclopentyloxy, cyclohexyloxy, and 4-methylcyclohexyloxy), an aryloxy group (preferably an aryloxy group having 6 to 26 carbon atoms, for example, phenoxy, 1-naphthyloxy, 3-methylphenoxy, and 4-methoxyphenoxy), a heterocyclic oxy group (for example, imidazolyloxy, benzimidazolyloxy, thiazolyloxy, benzothiazolyloxy, triazinyloxy, and purinyloxy),
an alkoxycarbonyl group (preferably an alkoxycarbonyl group having 2 to 20 carbon atoms, for example, ethoxycarbonyl and 2-ethylhexyloxycarbonyl), a cycloalkoxycarbonyl group (preferably a cycloalkoxycarbonyl group having 4 to 20 carbon atoms, for example, cyclopropyloxycarbonyl, cyclopentyloxycarbonyl, and cyclohexyloxycarbonyl), an aryloxycarbonyl group (preferably an aryloxycarbonyl group having 6 to 20 carbon atoms, for example, phenyloxycarbonyl, and naphthyloxycarbonyl), an amino group (preferably an amino group having 0 to 20 carbon atoms including an alkylamino group, an alkenylamino group, an alkynylamino group, a cycloalkylamino group, a cycloalkenylamino group, an arylamino group, and a heterocyclic amino group, for example, amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, and triazinylamino), a sulfamoyl group (preferably a sulfamoyl group having 0 to 20 carbon atoms, preferably an alkyl-, cycloalkyl-, or aryl-sulfamoyl group, for example, N,N-dimethylsulfamoyl, N-cyclohexylsulfamoyl, and N-phenylsulfamoyl), an acyl group (preferably an acyl group having 1 to 20 carbon atoms, for example, acetyl, cyclohexylcarbonyl, and benzoyl), an acyloxy group (preferably an acyloxy group having 1 to 20 carbon atoms, for example, acetyloxy, cyclohexylcarbonyloxy, and benzoyloxy), a carbamoyl group (preferably a carbamoyl group having 1 to 20 carbon atoms, preferably an alkyl-, cycloalkyl-, or aryl-carbamoyl group, for example, N,N-dimethylcarbamoyl, N-cyclohexylcarbamoyl, and N-phenylcarbamoyl),
an acylamino group (preferably an acylamino group having 1 to 20 carbon atoms, for example, acetylamino, cyclohexylcarbonylamino, and benzoylamino), a sulfonamido group (preferably a sulfonamido group having 0 to 20 carbon atoms, preferably an alkyl-, cycloalkyl-, or aryl-sulfonamido group, for example, methane sulfonamide, benzene sulfonamide, N-methyl methane sulfonamide, N-cyclohexyl sulfonamide, and N-ethyl benzene sulfonamide), an alkylthio group (preferably an alkylthio group having 1 to 20 carbon atoms, for example, methylthio, ethylthio, isopropylthio, and benzylthio), a cycloalkylthio group (preferably a cycloalkylthio group having 3 to 20 carbon atoms, for example, cyclopropylthio, cyclopentylthio, cyclohexylthio, and 4-methylcyclohexylthio), an arylthio group (preferably an arylthio group having 6 to 26 carbon atoms, for example, phenylthio, 1-naphthylthio, 3-methylphenylthio, and 4-methoxyphenylthio), an alkyl-, cycloalkyl-, or aryl-sulfonyl group (preferably a sulfonyl group having 1 to 20 carbon atoms, for example, methylsulfonyl, ethylsulfonyl, cyclohexylsulfonyl, and benzenesulfonyl),
a silyl group (preferably a silyl group having 1 to 20 carbon atoms, preferably an alkyl-, aryl-, alkoxy-, and aryloxy-substituted silyl group, for example, triethylsilyl, triisopropylsilyl, triphenylsilyl, diethylbenzylsilyl, and dimethylphenylsilyl), a silyloxy group (preferably a silyloxy group having 1 to 20 carbon atoms, preferably an alkyl-, aryl-, alkoxy-, and aryloxy-substituted silyloxy group, for example, triethylsilyloxy, triphenylsilyloxy, diethylbenzylsilyloxy, and dimethylphenylsilyloxy), a hydroxyl group, a cyano group, a nitro group, a halogen atom (for example, a fluorine atom, a chlorine atom, a bromine atom, and iodine atom), a carboxyl group, a sulfo group, a phosphonyl group, a phosphoryl group, and a boric acid group.

Examples of the group selected from the substituent group T more preferably include an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group (including an alkylamino group and an arylamino group), an acylamino group, a cyano group, and a halogen atom; and particularly preferably include an alkyl group, an alkenyl group, a heterocyclic group, an alkoxy group, an alkoxycarbonyl group, an amino group, an acylamino group, and a cyano group.

When the compound, the substituent, or the like includes an alkyl group, an alkenyl group, or the like, these may be substituted or unsubstituted. Further, when the compound, the substituent, or the like includes an aryl group, a heterocyclic group, or the like, these may be a monocycle or a fused ring, and may be substituted or unsubstituted.

### <Substituent Group T^{VL}>

The substituent group T^{VL} is a substituent group which does not include the respective groups included in the group G^{VL}. Examples of the groups included in the substituent group T^{VL} include substituents other than the aromatic ring group (R^{VL-1}), the alkyl group (R^{VL-2}), the alkenyl group (R^{VL-3}), the alkynyl group (R^{VL-4}), the alkylthio group (R^{VL-5}), the alkoxy group (R^{VL-6}), the amino group (R^{VL-7}), and the silyl group (R^{VL-8}) among the substituent groups exemplified as the substituent group T. Other examples of the substituent group T^{VL} include groups formed by combination of a plurality of the respective groups included in the substituent group T^{VL}.

Specific examples of the metal complex dye represented by Formula (1) are shown below and in Examples, and further include the specific examples of the following specific examples and the specific examples in Examples encompass metal complex dyes, in which at least one of -COOH's is formed into a salt of the carboxy group. In these metal complex dyes, examples of the counter cation that forms the salt of a carboxy group include the positive ions described for the CI. The present invention is not limited to these metal complex dyes. In a case where these metal complex dyes have optical isomers or geometric isomers, the metal complex dye may be any of these isomers or a mixture of these isomers.

The following specific examples each independently represent the specific examples of each of the ligand L1 and the ligand L2, irrespective of the specific combinations of the ligands L1 and L2 in each of the specific examples. In the specific examples, Me represents methyl and Et represents ethyl.

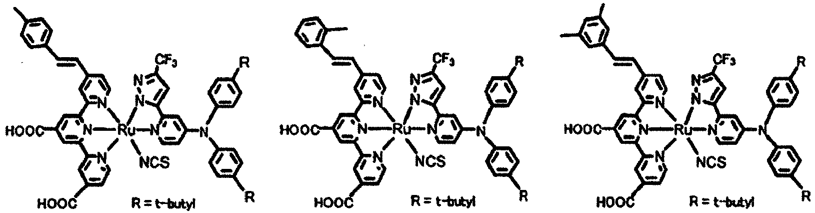

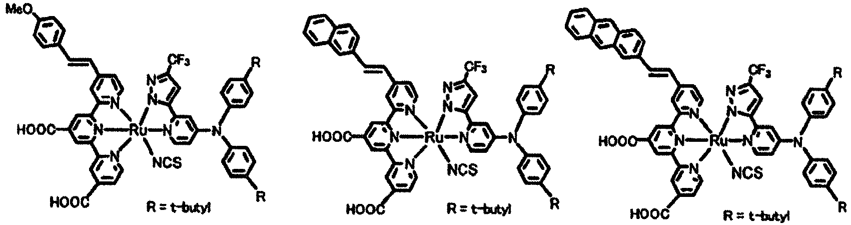

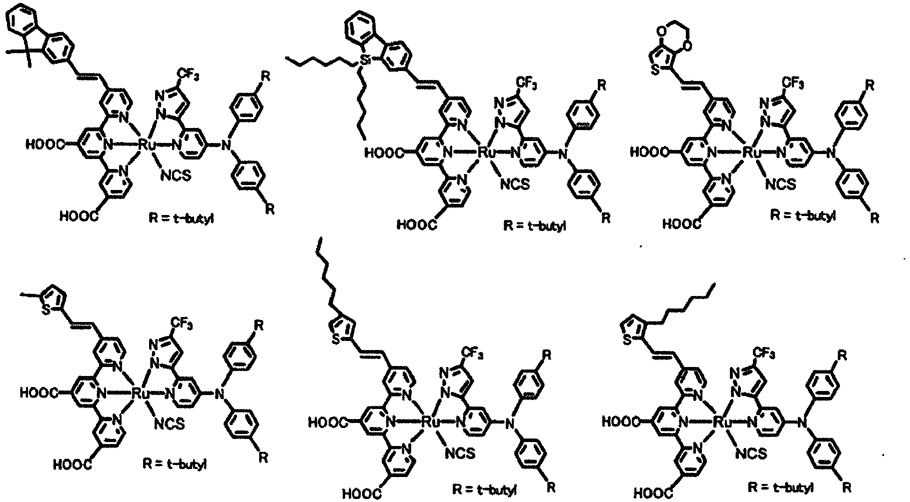

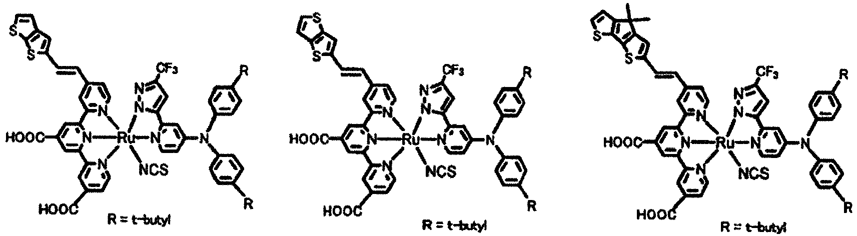

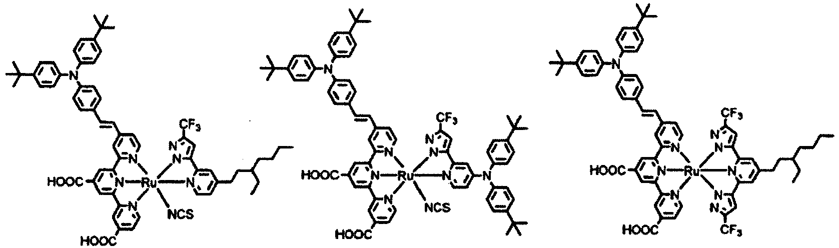

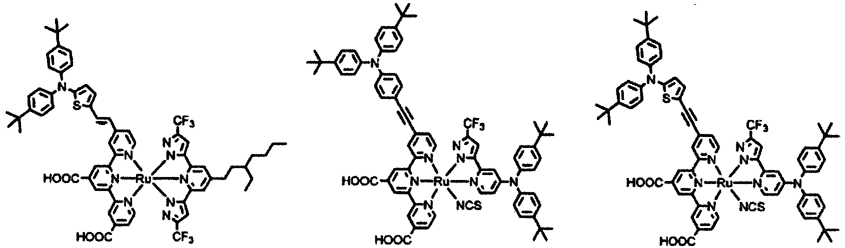

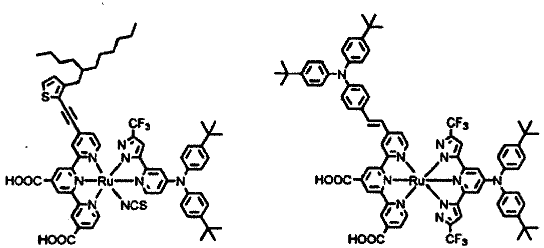

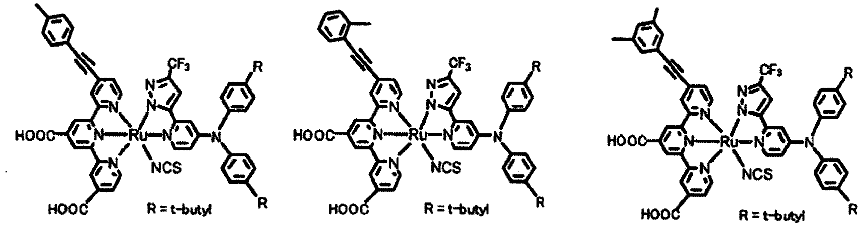

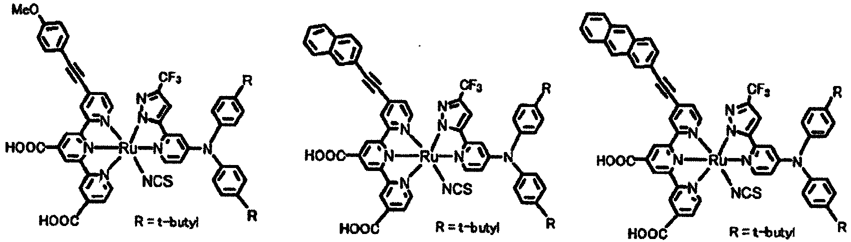

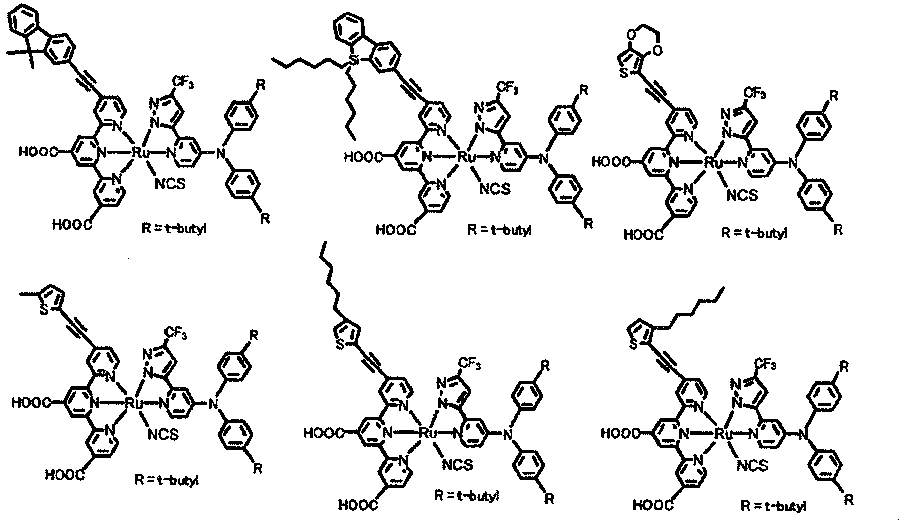

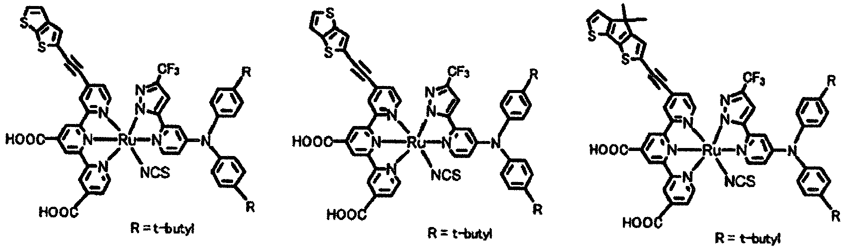

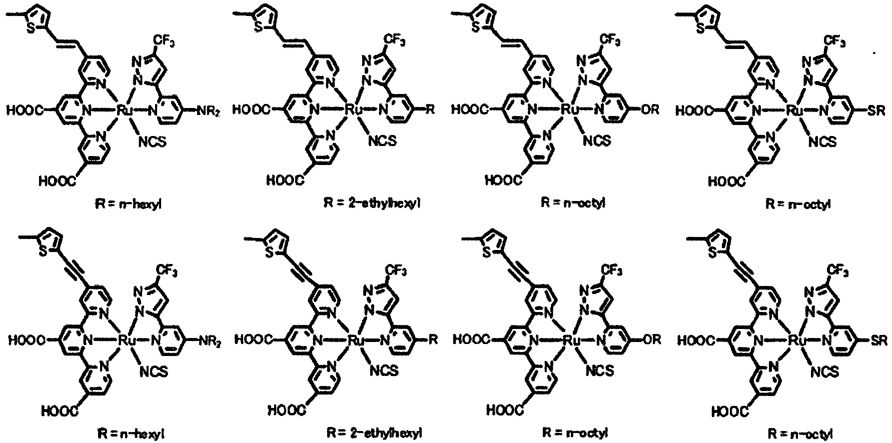

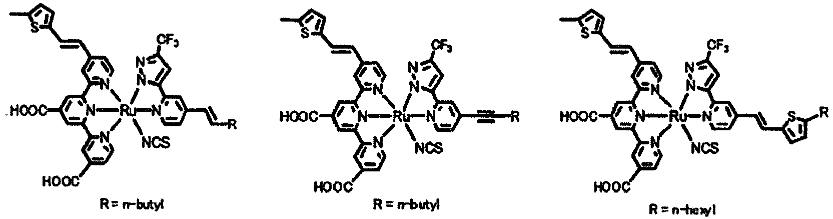

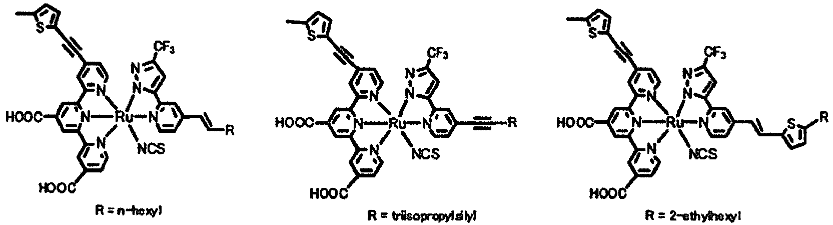

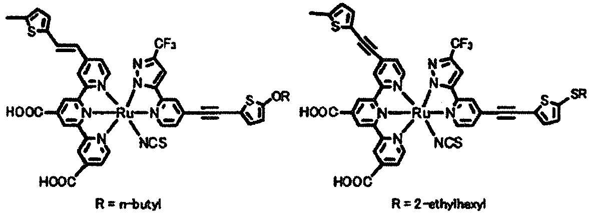

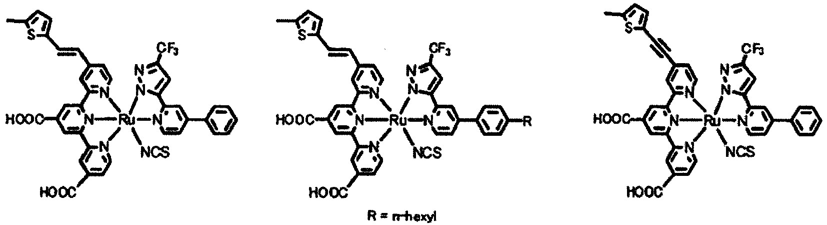

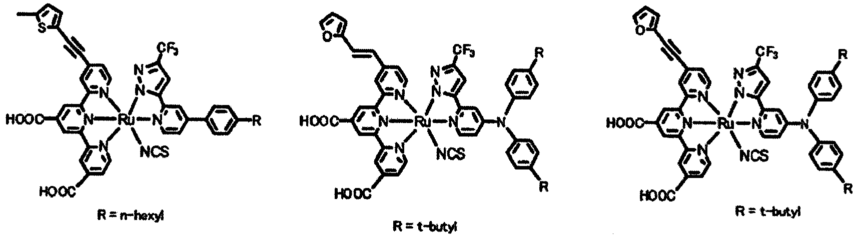

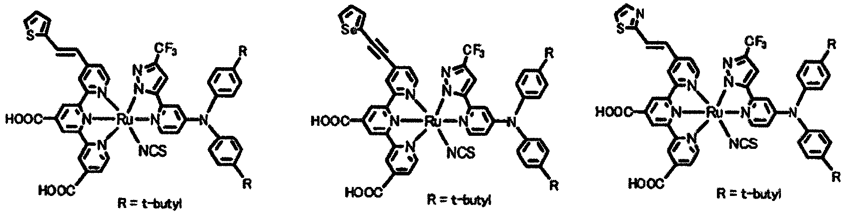

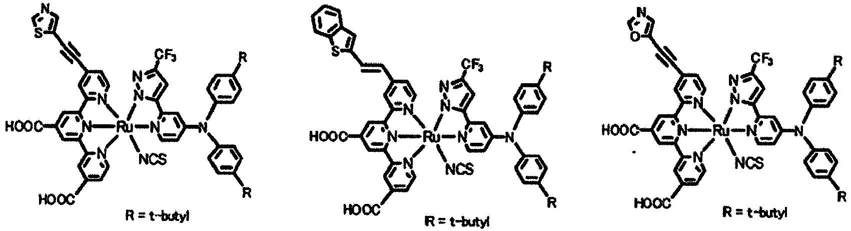

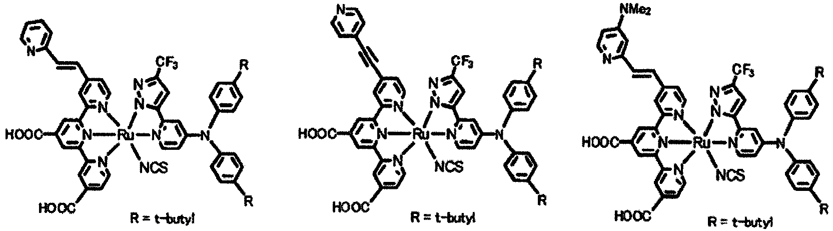

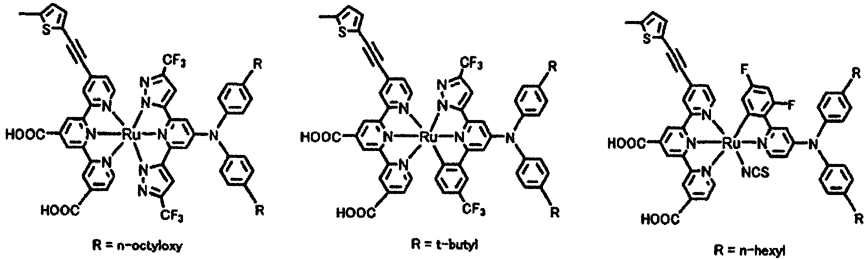

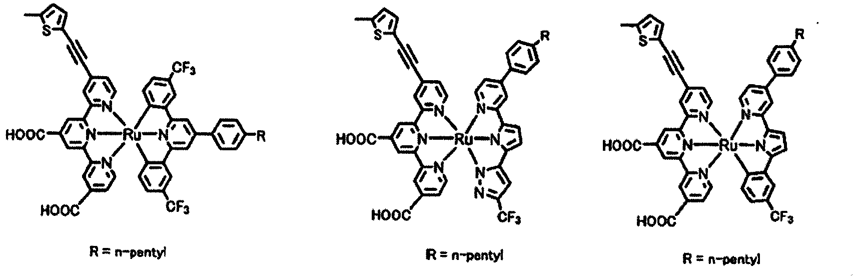

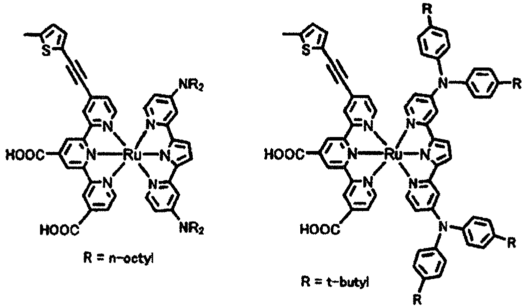

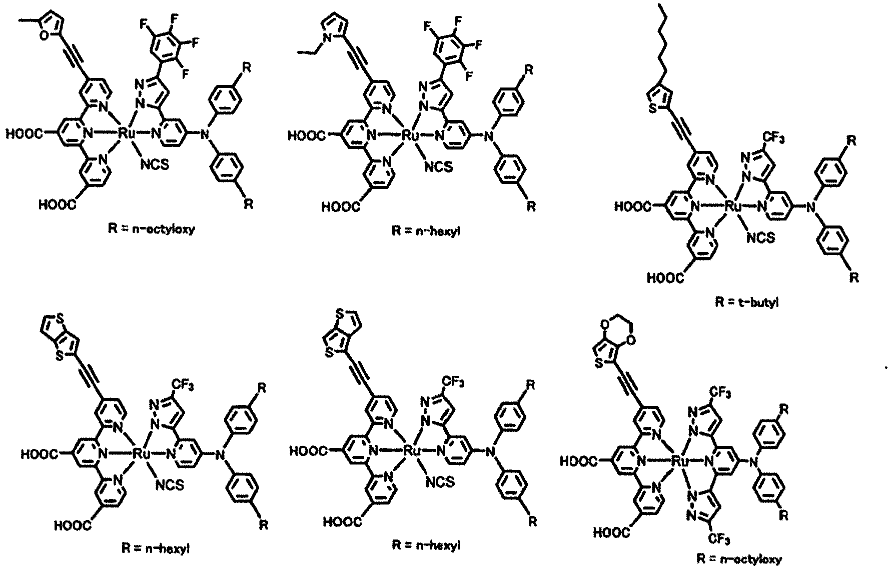

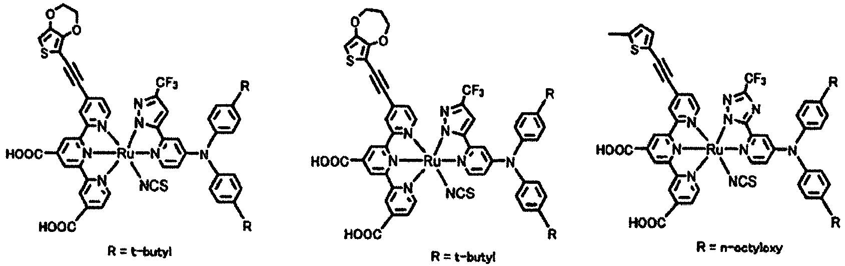

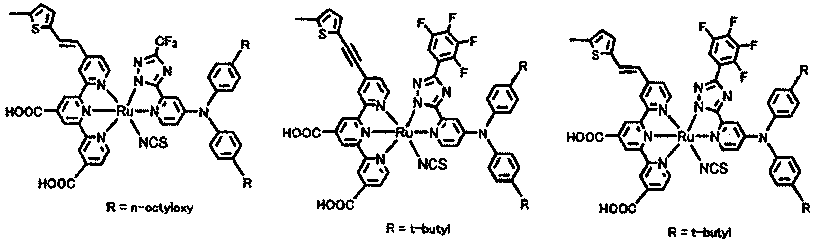

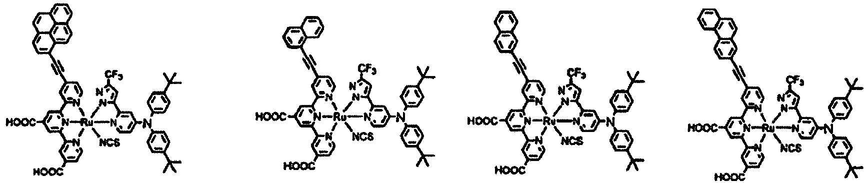

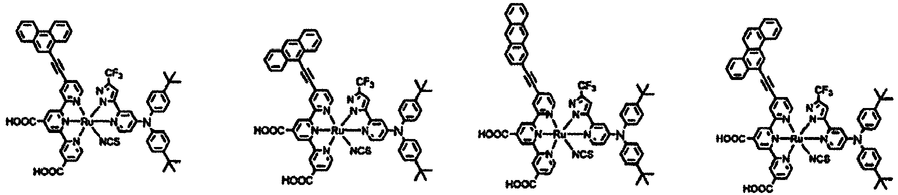

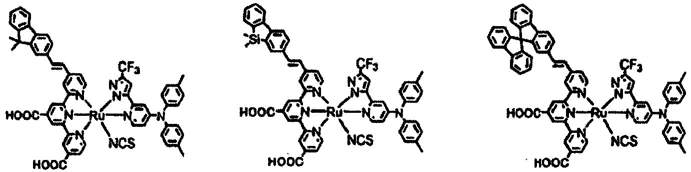

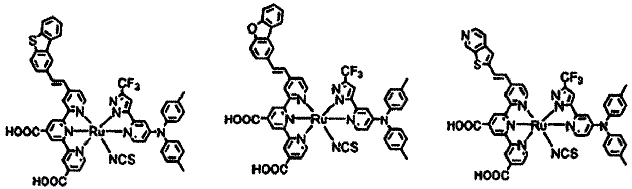

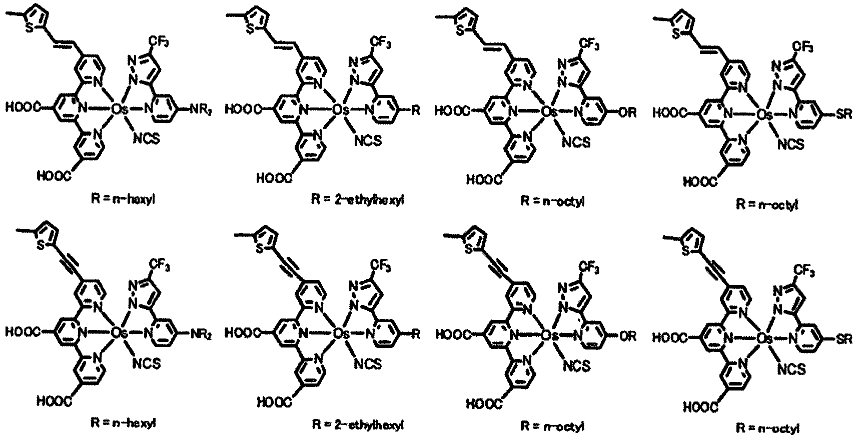

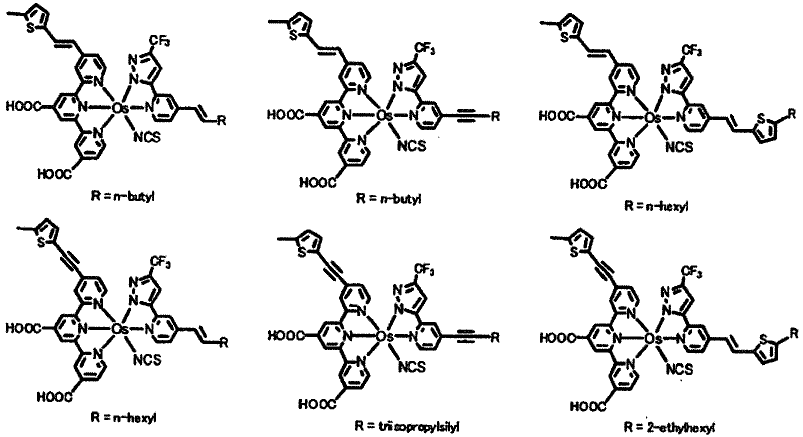

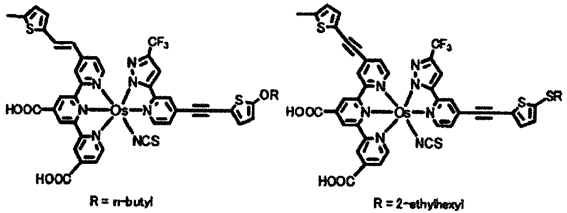

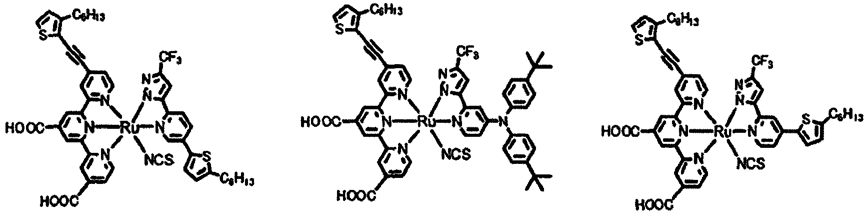

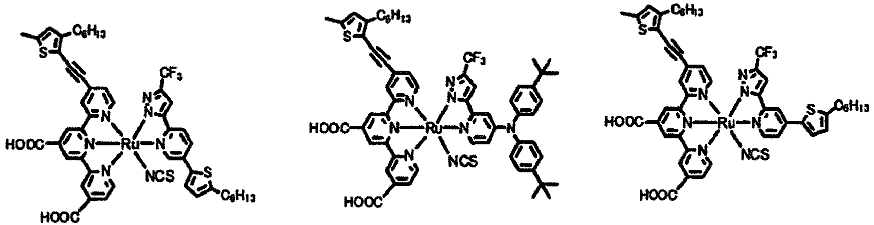

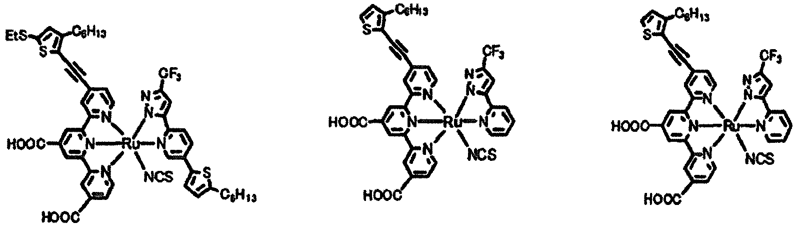

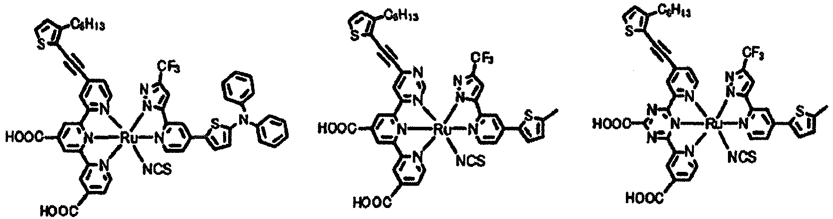

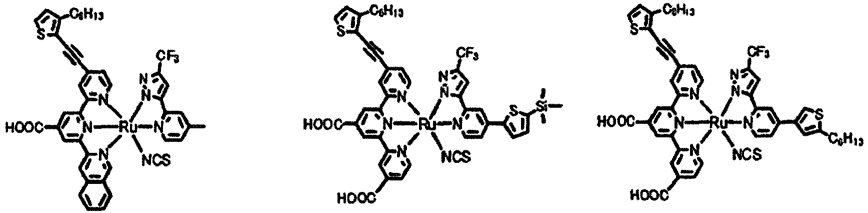

Next, preferred aspects of the main members of the photoelectric conversion element and the dye-sensitized solar cell will be described.

### <Electrically Conductive Support>

The electrically conductive support is not particularly limited as long as it has electrical conductivity and is capable of supporting a photoconductor layer 2 or the like. The electrically conductive support is a material having conductivity, such as an electrically conductive support 1 formed of a metal, or an electrically conductive support 41 having a glass or plastic substrate 44 and a transparent electrically-conductive film 43 formed on the surface of the substrate 44.

Between them, the electrically conductive support 41 in which the transparent electrically-conductive film 43 is formed by applying an electrically conductive metal oxide onto the surface of the substrate 44 is more preferable. Examples of the substrate 44 formed of plastics include the transparent polymer films described in paragraph No. 0153 of JP2001-291534A. Further, as a material which forms the substrate 44, ceramics (JP2005-135902A) or electrically conductive resins (JP2001-160425A) can be used, in addition to glass and plastics. As the metal oxide, tin oxide (TO) is preferable, and indium-tin oxide (tin-doped indium oxide; ITO), and fluorine-doped tin oxide such as tin oxide which has been doped with tin (FTO) are particularly preferable. In this case, the coating amount of the metal oxide is preferably 0.1 to 100 g, per square meter of the surface area of the substrate 44. In the case of using the electrically conductive support 41, it is preferable that light is incident from the substrate 44.

It is preferable that the electrically conductive supports 1 and 41 are substantially transparent. The expression, "substantially transparent", means that the transmittance of light (at a wavelength of 300 to 1,200 nm) is 10% or more, preferably 50% or more, and particularly preferably 80% or more.

The thickness of the electrically conductive supports 1 and 41 is not particularly limited, but is preferably 0.05 µm to 10 mm, more preferably 0.1 µm to 5 mm, and particularly preferably 0.3 µm to 4 mm.

In the case of providing a transparent electrically-conductive film 43, the thickness of the transparent electrically-conductive film 43 is preferably 0.01 to 30 µm, more preferably 0.03 to 25 µm, and particularly preferably 0.05 to 20 µm.

The electrically conductive supports 1 and 41 may be provided with a light management function at the surface, and may have, for example, the anti-reflection film having a high refractive index film and a low refractive index oxide film alternately laminated described in JP2003-123859A, and the light guide function described in JP2002-260746A on the surface.

### <Photoconductor Layer>

As long as the photoconductor layer has semiconductor fine particles 22 carrying the dye 21 and an electrolyte, it is not particularly limited in terms of other configurations. Preferred examples thereof include the photoconductor layer 2 and the photoconductor layer 42.

### - Semiconductor Fine Particles (Layer Formed by Semiconductor Fine Particles) -

The semiconductor fine particles 22 are preferably fine particles of chalcogenides of metals (for example, oxides, sulfides, and selenides) or of compounds having perovskite type crystal structures. Preferred examples of the chalcogenides of metals include oxides of titanium, tin, zinc, tungsten, zirconium, hafnium, strontium, indium, cerium, yttrium, lanthanum, vanadium, niobium, or tantalum, cadmium sulfide, and cadmium selenide. Preferred examples of the compounds having perovskite type crystal structures include strontium titanate and calcium titanate. Among these, titanium oxide (titania), zinc oxide, tin oxide, and tungsten oxide are particularly preferable.

Examples of the crystal structure of titania include structures of an anatase type, a brookite type, and a rutile type, with the structures of an anatase type and a brookite type being preferable. A titania nanotube, nanowire, or nanorod may be used singly or in mixture with titania fine particles.

The particle diameter of the semiconductor fine particles 22 is expressed in terms of an average particle size using a diameter when a projected area is converted into a circle, and is preferably 0.001 to 1 µm as primary particles, and 0.01 to 100 µm as an average particle size of dispersions. Examples of the method for coating the semiconductor fine particles 22 on the electrically conductive supports 1 or 41 include a wet method, a dry method, and other methods.

It is preferable that the semiconductor fine particles 22 have a large surface area so that they may adsorb a large amount of the dye 21. For example, in a state where the semiconductor fine particles 22 are coated on the electrically conductive support 1 or 41, the surface area is preferably 10 times or more, and more preferably 100 times or more, with respect to the projected surface area. The upper limit of this value is not particularly limited, and the upper limit is usually about 5,000 times. In general, as the thickness of the semiconductor layer 45 (having the same definition as the photoconductor layer 2 in the photoelectric conversion element 10) formed by the semiconductor fine particles 22 increases, the amount of dye 21 that can be carried per unit area increases, and therefore, the light absorption efficiency increases. However, since the diffusion distance of generated electrons increases correspondingly, the loss due to charge recombination also increases.

As described above, it can be expected that in the photoelectric conversion element and the dye-sensitized solar cell, as the diffusion distance of excited electrons is smaller, the electron transport efficiency more increases. However, when the thickness of the semiconductor layer is decreased, the photoelectric conversion efficiency may be reduced in some cases. The photoelectric conversion element and the dye-sensitized solar cell of the present invention have the metal complex dye of the present invention, which uses a combination of the ligand L1 and the ligand L2. Thus, even in a case where the semiconductor layer has the same thickness as that the related art or has a smaller thickness than that in the related art, excellent photoelectric conversion efficiency is exerted. Thus, according to the present invention, the effect of the film thickness of the semiconductor layer is little and excellent photoelectric conversion efficiency is exerted.

Although a preferred thickness of the semiconductor layer 45 (the photoconductor layer 2 in the photoelectric conversion element 10) may vary depending on the utility of the photoelectric conversion element, the thickness is typically 0.1 to 100 µm. In the case of using the photoelectric conversion element as a dye-sensitized solar cell, the thickness of the photoconductor layer is more preferably 1 to 50 µm, and still more preferably 3 to 30 µm.

In the present invention, by using the metal complex dye represented by Formula (1), the thickness of the semiconductor layer 45 can be reduced. For example, among the preferred ranges, the thickness can be adjusted to 8 µm or less.

It is preferable that the semiconductor fine particles 22 may be calcined at a temperature of 100°C to 800°C for 10 minutes to 10 hours after being applied on the electrically conductive support 1 or 41, so as to bring about cohesion of the particles. In the case of using glass as a material for the electrically conductive support 1 or the substrate 44 is used, the temperature is preferably 60°C to 600°C.

The coating amount of the semiconductor fine particles 22 per square meter of the surface area of the electrically conductive support 1 or 41 is preferably 0.5 to 500 g, and more preferably 5 to 100 g.

It is preferable that a short circuit-preventing layer is formed between the electrically conductive support 1 or 41 and the photoconductor layer 2 or 42 so as to prevent reverse current due to a direct contact between the electrolyte included in the photoconductor layer 2 or 42 and the electrically conductive support 1 or 41.

In addition, it is preferable to employ a spacer S (see Fig. 2) or a separator, so as to prevent contact between the light-receiving electrode 5 or 40 and the counter electrode 4 or 48.

### - Dye -

In the photoelectric conversion element 10 and the dye-sensitized solar cell 20, at least one kind of metal complex dye represented by Formula (1) is used as a sensitizing dye. The metal complex dye represented by Formula (1) is as described above.

In the present invention, examples of the dye that can be used in combination with the metal complex dye of Formula (1) include an Ru complex dye, a squarylium cyanine squarylium cyanine dye, an organic dye, a porphyrine dye, and a phthalocyanine dye.

Examples of the Ru complex dye include the Ru complex dyes described in JP1995-500630T (JP-H07-500630T) (in particular, the dyes synthesized in Examples 1 to 19 described in from line 5 on left lower column on page 5 to line 7 on right upper column on page 7), the Ru complex dyes described in JP2002-512729T (in particular, dyes synthesized in Examples 1 to 16 described in line 3 from the bottom of page 20 to line 23 on page 29), the Ru complex dyes described in JP2001-59062A (in particular, the dyes described in paragraph Nos. 0087 to 0104), the Ru complex dyes described in JP2001-6760A (in particular, the dyes described in paragraph Nos. 0093 to 0102), the Ru complex dyes described in JP2001-253894A (in particular, the dyes described in paragraph Nos. 0009 and 0010), the Ru complex dyes described in JP2003-212851A (in particular, the dyes described in paragraph No. 0005), the Ru complex dyes described in WO2007/91525A (in particular, the dyes described in [0067]), the Ru complex dyes described in JP2001-291534A (in particular, the dyes described in paragraph Nos. 0120 to 0144), the Ru complex dyes described in JP2012-012570A (in particular, the dyes described in paragraph Nos. 0095 to 0103), the Ru complex dyes described in JP2013-084594A (in particular, the dyes described in paragraph Nos. 0072 to 0081 and the like), the Ru complex dyes described in WO2013/088898A (in particular, the dyes described in [0286] to [0293]), and the Ru complex dyes described in WO2013/47615A (in particular, the dyes described in [0078] to [0082]).

Examples of the squarylium cyanine dye include the squarylium cyanine dyes described in JP1999-214730A (JP-H11-214730A) (in particular, the dyes described in paragraph Nos. 0036 to 0047), the squarylium cyanine dyes described in JP2012-144688A (in particular, the dyes described in paragraph Nos. 0039 to 0046 and 0054 to 0060), and the squarylium cyanine dyes described in JP2012-84503A (in particular, the dyes described in paragraph Nos. 0066 to 0076 and the like)

Examples of the organic dye include the organic dyes described in JP2004-063274A (in particular, the dyes described in paragraph Nos. 0017 to 0021), the organic dyes described in JP2005-123033A (in particular, the dyes described in paragraph Nos. 0021 to 0028), the organic dyes described in JP2007-287694A (in particular, the dyes described in paragraph Nos. 0091 to 0096), the organic dyes described in JP2008-71648A (in particular, the dyes described in paragraph Nos. 0030 to 0034), and the organic dyes described in WO2007/119525A (in particular, the dyes described in paragraph No. [0024]).

Examples of the porphyrine dye include the porphyrine dyes described in Angew. Chem. Int. Ed., 49, pp. 1 to 5 (2010), or the like, and the phthalocyanine dyes described in Angew. Chem. Int. Ed., 46, p. 8358 (2007), or the like.

As the dye to be used in combination, Ru complex dyes, squarylium cyanine dyes, or organic dyes are preferable.

The overall amount of the dye to be used is preferably 0.01 to 100 millimoles, more preferably 0.1 to 50 millimoles, and particularly preferably 0.1 to 10 millimoles, per square meter of the surface area of the electrically conductive support 1 or 41. Further, the amount of the dye 21 to be adsorbed onto the semiconductor fine particles 22 is preferably 0.001 to 1 millimole, and more preferably 0.1 to 0.5 millimoles, with respect to 1 g of the semiconductor fine particles 22. By setting the amount of the dye to such a range, the sensitization effect on the semiconductor fine particles 22 is sufficiently obtained.

In a case where the metal complex dye represented by Formula (1) is used in combination with another dye, the ratio of the mass of the metal complex dye represented by Formula (1)/the mass of another dye is preferably 95/5 to 10/90, more preferably 95/5 to 50/50, still more preferably 95/5 to 60/40, particularly preferably 95/5 to 65/35, and most preferably 95/5 to 70/30.

After the dye is carried on the semiconductor fine particles 22, the surface of the semiconductor fine particles 22 may be treated using an amine compound. Preferred examples of the amine compound include pyridine compounds (for example, 4-t-butylpyridine and polyvinylpyridine). These may be used as they are in a case where they are liquids, or may be used in a state where they are dissolved in an organic solvent.

### - Co-Adsorbent -

In the present invention, it is preferable to use a co-adsorbent together with the metal complex dye represented by Formula (1) or with another dye to be used in combination, if necessary. Such a co-adsorbent which includes a co-adsorbent having at least one acidic group (preferably a carboxyl group or a salt thereof) is preferable, and examples thereof include a fatty acid and a compound having a steroid skeleton.

The fatty acid may be a saturated fatty acid or an unsaturated fatty acid, and examples thereof include a butanoic acid, a hexanoic acid, an octanoic acid, a decanoic acid, a hexadecanoic acid, a dodecanoic acid, a palmitic acid, a stearic acid, an oleic acid, a linoleic acid, and a linolenic acid.

Examples of the compound having a steroid skeleton include cholic acid, glycocholic acid, chenodeoxycholic acid, hyocholic acid, deoxycholic acid, lithocholic acid, and ursodeoxycholic acid, among which cholic acid, deoxycholic acid, and chenodeoxycholic acid are preferable; and deoxycholic acid are more preferable.

A preferred co-adsorbent is a compound represented by the following Formula (CA).

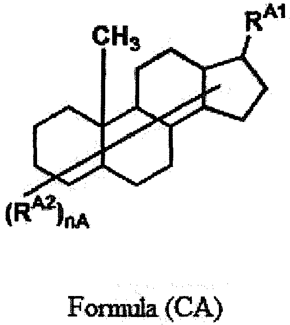

In the formula, R^{A1} represents a substituent having an acidic group. R^{A2} represents a substituent. nA represents an integer of 0 or more.

The acidic group has the same meaning as the acidic group in Formula (L1-1), and a preferable range thereof is also the same.

Among these, R^{A1} is preferably an alkyl group substituted with any one of a carboxyl group, a sulfo group, and a salt thereof, and more preferably -CH(CH₃)CH₂CH₂CO₂H or -CH(CH₃)CH₂CH₂CONHCH₂CH₂SO₃H.

Examples of R^{A2} include groups selected from the substituent group T. Among those, an alkyl group, a hydroxyl group, an acyloxy group, an alkylaminocarbonyloxy group, or an arylaminocarbonyloxy group is preferable; and an alkyl group, a hydroxyl group, or an acyloxy group is more preferable.

nA is preferably 2 to 4.

By making the co-adsorbent adsorbed onto the semiconductor fine particles 22, the co-adsorbent exhibits an effect of suppressing the inefficient association of the metal complex dye and an effect of preventing reverse electron transfer from the surface of the semiconductor fine particles to the redox system in the electrolyte. The amount of the co-adsorbent to be used is not particularly limited, and from the viewpoint of exhibiting the above effects effectively, the amount is preferably 1 to 200 moles, more preferably 10 to 150 moles, and particularly preferably 20 to 50 moles, with respect to 1 mole of the metal complex dye.

### - Light-Scattering Layer -

In the present invention, the light-scattering layer is different from the semiconductor layer in that the light-scattering layer has a function of scattering incident light.

In the dye-sensitized solar cell 20, the light-scattering layer 46 preferably contains rod-shaped or plate-shaped metal oxide particles. Examples of the metal oxide particles to be used in the light-scattering layer 46 include particles of the chalcogenides (oxides) of the metals. In the case of providing the light-scattering layer 46, it is preferable that the thickness of the light-scattering layer is set to 10% to 50% of the thickness of the photoconductor layer 42.

The light-scattering layer 46 is preferably the light-scattering layer described in JP2002-289274A, and the description of JP2002-289274A is preferably herein incorporated by reference.

### <Charge Transfer Layer>

The charge transfer layers 3 and 47 used in the photoelectric conversion element of the present invention are layers having a function of complementing electrons for the oxidized forms of the dye 21, and are provided between the light-receiving electrode 5 or 40 and the counter electrode 4 or 48.

The charge transfer layers 3 and 47 include electrolytes. Here, the expression, "the charge transfer layer includes an electrolyte", is meant to encompass both of an aspect in which the charge transfer layer consists of only electrolytes and an aspect in which the charge transfer layer consists of electrolytes and materials other than the electrolytes.

The charge transfer layers 3 and 47 may be any of a solid form, a liquid form, a gel form, or a mixture thereof.

### - Electrolyte -

Examples of the electrolyte include a liquid electrolyte having a redox pair dissolved in an organic solvent, and a so-called gel electrolyte in which a molten salt containing a redox pair and a liquid having a redox pair dissolved in an organic solvent are impregnated in a polymer matrix. Among those, from the viewpoint of photoelectric conversion efficiency, a liquid electrolyte is preferable.

Examples of the redox pair include a combination of iodine and an iodide (preferably an iodide salt or an iodide ionic liquid, and more preferably lithium iodide, tetrabutylammonium iodide, tetrapropylammonium iodide, and methylpropylimidazolium iodide), a combination of an alkylviologen (for example, methylviologen chloride, hexylviologen bromide, and benzylviologen tetrafluoroborate) and a reductant thereof, a combination of a polyhydroxybenzene (for example, hydroquinone and naphthohydroquinone) and an oxidized form thereof, a combination of a divalent iron complex and a trivalent iron complex (for example, a combination of potassium ferricyanide and potassium ferrocyanide), and a combination of a divalent cobalt complex and a trivalent cobalt complex. Among these, a combination of iodine and an iodide, or a combination of a divalent cobalt complex and a trivalent cobalt complex is preferable, and a combination of iodine and an iodide is particularly preferable.

As the cobalt complex, the complex represented by Formula (CC) described in paragraph Nos. 0144 to 0156 of JP2014-82189A is preferable, and the description of paragraph Nos. 0144 to 0156 of JP2014-82189A is preferably incorporated in the present specification.

In a case where a combination of iodine and iodide is used as an electrolyte, it is preferable that a nitrogen-containing aromatic cation iodide salt of a 5- or 6-membered ring is additionally used.

The organic solvent which is used in a liquid electrolyte and a gel electrolyte is not particularly limited, but is preferably an aprotic polar solvent (for example, acetonitrile, propylene carbonate, ethylene carbonate, dimethylformamide, dimethylsulfoxide, sulfolane, 1,3-dimethylimidazolinone, and 3-methyloxazolidinone).

In particular, as the organic solvent which is used for a liquid electrolyte, a nitrile compound, an ether compound, an ester compound, or the like is preferable, a nitrile compound is more preferable, and acetonitrile or methoxypropionitrile is particularly preferable.

As a molten salt, an ionic liquid including an imidazolium or triazolium type cation, an ionic liquid including an oxazolium type cation, an ionic liquid including a pyridinium type cation, an ionic liquid including a guanidium type cation, and combinations of these are preferable. Further, these cations may be used in combination with specific anions. Additives may be added to these molten salts. The molten salt may have a substituent having liquid crystalline properties. In addition, a molten salt of the quaternary ammonium salt may also be used as the molten salt.

Other examples of the molten salts include a molten salt to which fluidity at room temperature has been imparted by mixing lithium iodide and at least one kind of other lithium salt (for example, lithium acetate and lithium perchlorate) with polyethylene oxide. In this case, the amount of the polymer to be added is 1% to 50% by mass. Further, an electrolytic solution may contain γ-butyrolactone, and this γ-butyrolactone increases the diffusion efficiency of iodide ions, whereby the photoelectric conversion efficiency is enhanced.

Examples of the polymer (polymer matrix) to be used in a matrix of the gel electrolyte include polyacrylonitrile and polyvinylidene fluoride.

The electrolyte may be quasi-solidified by adding a gelling agent to an electrolytic solution formed of an electrolyte and a solvent, followed by gelling (the quasi-solidified electrolyte may also be hereinafter referred to as a "quasi-solidified electrolyte"). Examples of the gelling agent include an organic compound having a molecular weight of 1,000 or less, an Si-containing compound having a molecular weight in the range of 500 to 5,000, an organic salt generated from a specific acidic compound and a specific basic compound, a sorbitol derivative, and polyvinylpyridine.

Furthermore, a method of confining a polymer matrix, a crosslinkable polymer compound or monomer, a crosslinking agent, an electrolyte, and a solvent in a polymer may also be used.

Preferred examples of the polymer matrix include a polymer having a nitrogen-containing heterocycle in a repeating unit in the main chain or in the side chain, and a crosslinked structure formed by reacting the polymer with an electrophilic compound, a polymer having a triazine structure, a polymer having a ureide structure, a polymer containing a liquid crystalline compound, a polymer having an ether bond, polyvinylidene fluoride, a methacrylate, an acrylate, a thermosetting resin, crosslinked polysiloxane, polyvinyl alcohol (PVA), a clathrate compound of polyalkylene glycol with dextrin or the like, a system incorporated with an oxygen-containing or sulfur-containing polymer, and a naturally occurring polymer. An alkali-swellable polymer, a polymer having a cation moiety and a compound capable of forming a charge transfer complex with iodine within one polymer molecule, or the like may be added to those polymer matrixes.

A system containing, as a polymer matrix, a crosslinked polymer formed by reacting a bifunctional or higher-functional isocyanate as one component with a functional group such as a hydroxyl group, an amino group or a carboxyl group, may also be used. Furthermore, a crosslinked polymer based on a hydrosilyl group and a double-bonded compound, a crosslinking method involving reacting polysulfonic acid, polycarboxylic acid, or the like with a divalent or higher-valent metal ion compound, and the like may also be used.

Examples of the solvent that can be preferably used in combination with the quasi-solid electrolyte described above include a specific phosphoric ester, a mixed solvent including ethylene carbonate, a solvent having a specific relative permittivity, and the like. A liquid electrolyte solution may be retained in a solid electrolyte membrane or in pores, and preferred examples of the method for retaining the liquid electrolyte solution include a method using an electrically conductive polymer membrane, a fibrous solid, and a fabric-like solid such as a filter.

The electrolyte may contain aminopyridine compounds, benzimidazole compounds, aminotriazole compounds, aminothiazole compounds, imidazole compounds, aminotriazine compounds, urea compounds, amide compounds, pyrimidine compounds, and heterocycles not including nitrogen, in addition to pyridine compounds such as 4-t-butylpyridine, as an additive.

Moreover, a method of controlling the moisture content of the electrolytic solution may be employed in order to enhance the photoelectric conversion efficiency. Preferred examples of the method of controlling the moisture content include a method of controlling the concentration, and a method of adding a dehydrating agent. The moisture content of the electrolytic solution is preferably adjusted to 0% to 0.1% by mass.

Iodine can also be used as a clathrate compound of iodine with cyclodextrin. Furthermore, a cyclic amidine may be used, or an antioxidant, a hydrolysis inhibitor, a decomposition inhibitor, or zinc iodide may be added.

A solid-state charge transport layer such as a p-type semiconductor or a hole transport material, for example, CuI or CuNCS, may be used in place of the liquid electrolyte and the quasi-solid-state electrolyte as described above. Moreover, the electrolytes described in Nature, vol. 486, p. 487 (2012) and the like may also be used. For a solid-state charge transport layer, an organic hole transport material may be used. Preferred examples of the hole transport layer include electrically conductive polymers such as polythiophene, polyaniline, polypyrrole, and polysilane; a spiro compound in which two rings share a central element adopting a tetrahedral structure, such as C and Si; aromatic amine derivatives such as triarylamine; triphenylene derivatives; nitrogen-containing heterocyclic derivatives; and liquid crystalline cyano derivatives.

The redox pair serves as an electron carrier, and accordingly, it is preferably contained at a certain concentration. The concentration of the redox pair in total is preferably 0.01 mol/L or more, more preferably 0.1 mol/L or more, and particularly preferably 0.3 mol/L or more. In this case, the upper limit is not particularly limited, but is usually approximately 5 mol/L.

### <Counter Electrode>

The counter electrodes 4 and 48 preferably work as a positive electrode in a dye-sensitized solar cell. The counter electrodes 4 and 48 usually have the same configurations as the electrically conductive support 1 or 41, but in a configuration in which strength is sufficiently maintained, a substrate 44 is not necessarily required. A preferred structure of the counter electrodes 4 and 48 is a structure having a high charge collecting effect. At least one of the electrically conductive support 1 or 41 and the counter electrode 4 or 48 should be substantially transparent so that light may reach the photoconductor layers 2 and 42. In the dye-sensitized solar cell of the present invention, the electrically conductive support 1 or 41 is preferably transparent to allow sunlight to be incident from the side of the electrically conductive support 1 or 41. In this case, the counter electrodes 4 and 48 more preferably have light reflecting properties. As the counter electrodes 4 and 48 of the dye-sensitized solar cell, glass or plastic on which a metal or an electrically conductive oxide is deposited is preferable, and glass on which platinum is deposited is particularly preferable. In the dye-sensitized solar cell, a lateral side of the cell is preferably sealed with a polymer, an adhesive, or the like in order to prevent evaporation of components.

The present invention can be applied to the photoelectric conversion elements and the dye-sensitized solar cells described in, for example, JP4260494B, JP2004-146425A, JP2000-340269A, JP2002-289274A, JP2004-152613A, or JP1997-27352A (JP-H09-27352A). In addition, the present invention can be applied to the photoelectric conversion elements and the dye-sensitized solar cells described in, for example, JP2004-152613A, JP2000-90989A, JP2003-217688A, JP2002-367686A, JP2003-323818A, JP2001-43907A, JP2000-340269A, JP2005-85500A, JP2004-273272A, JP2000-323190A, JP2000-228234A, JP2001-266963A, JP2001-185244A, JP2001-525108T, JP2001-203377A, JP2000-100483A, JP2001-210390A, JP2002-280587A, JP2001-273937A, JP2000-285977A, or JP2001-320068A.

### [Method for Producing Photoelectric Conversion Element and Dye-Sensitized Solar Cell]

The photoelectric conversion element and the dye-sensitized solar cell of the present invention are preferably produced using a dye solution (the dye solution of the present invention) which contains the metal complex dye of the present invention and a solvent.

Such a dye solution is formed of the metal complex dye of the present invention dissolved in a solvent, and may also include a co-adsorbent and other components, if necessary.

Examples of the solvent to be used include the solvents described in JP2001-291534A, but are not particularly limited thereto. In the present invention, an organic solvent is preferable, and an alcohol solvent, an amide solvent, a nitrile solvent, a hydrocarbon solvent, and a mixed solvent of two or more kinds of these solvents are more preferable. As the mixed solvent, a mixed solvent of an alcohol solvent and a solvent selected from an amide solvent, a nitrile solvent, and a hydrocarbon solvent is preferable; a mixed solvent of an alcohol solvent and an amide solvent, a mixed solvent of an alcohol solvent and a hydrocarbon solvent, and a mixed solvent of an alcohol solvent and a nitrile solvent are more preferable; and a mixed solvent of an alcohol solvent and an amide solvent, and a mixed solvent of an alcohol solvent and a nitrile solvent are particularly preferable. Specifically, a mixed solvent of at least one kind of methanol, ethanol, propanol, butanol, and t-butanol, and at least one kind of dimethylformamide and dimethylacetamide, and a mixed solvent of at least one kind of methanol, ethanol, propanol, and t-butanol, and acetonitrile are preferable.

The dye solution preferably contains a co-adsorbent, and as the co-adsorbent, the aforementioned co-adsorbent is preferable. Among those, the compound represented by Formula (CA) is preferable.

Here, the dye solution of the present invention is preferably one in which the concentrations of the metal complex dye and the co-adsorbent have been adjusted so that the dye solution can be used as it is during production of the photoelectric conversion element or the dye-sensitized solar cell. In the present invention, the dye solution of the present invention preferably contains 0.001% to 0.1% by mass of the metal complex dye of the present invention. The amount of the co-adsorbent to be used is as described above.

For the dye solution, it is preferable to adjust the moisture content, and thus in the present invention, the water content is preferably adjusted 0% to 0.1% by mass.

In the present invention, it is preferable to manufacture a photoconductor layer by making the metal complex dye represented by Formula (1) or a dye including the same on the surface of the semiconductor fine particles, using the dye solution. That is, the photoconductor layer is preferably formed by applying (including a dip method) the dye solution onto the semiconductor fine particles provided on the electrically conductive support, followed by drying and curing.

By further providing a charge transfer layer, a counter electrode, or the like for a light-receiving electrode including the photoconductor layer as manufactured above, the photoelectric conversion element or the dye-sensitized solar cell of the present invention can be obtained.

The dye-sensitized solar cell is produced by connecting an external circuit 6 with the electrically conductive support 1 and the counter electrode 4 of the photoelectric conversion element thus manufactured.

### EXAMPLES

Hereinafter, the present invention will be described in more detail, based on Examples, but the invention is not limited thereto.

### Example 1 (Synthesis of Metal Complex Dye)

Hereinafter, a method for synthesizing the metal complex dye of the present invention will be described in detail, but starting materials, dye intermediates, and synthesis routes are not limited thereto.

In the present invention, the room temperature means 25°C. Further, in the following synthesis method, Me represents methyl, Et represents ethyl, and TBA represents tetrabutylammonium.

The metal complex dye and the synthesis intermediate synthesized in Example 1 were identified by mass spectrum (MS) measurement and ¹H-NMR measurement, if necessary.

The TBA salt, the K salt, and the Na salt of the synthesized metal complex dye become the same mass as the metal complex dye which is electrically neutral by protonization in the MS measurement, and therefore the results of the MS measurement are omitted for the TBA salt, the K salt, and the Na salt.

### (Synthesis of Metal Complex Dye (1-1) and Metal Complex Dye (1-1TBA))

According to the following scheme, a metal complex dye (1-1) and a metal complex dye (1-1TBA) were synthesized.

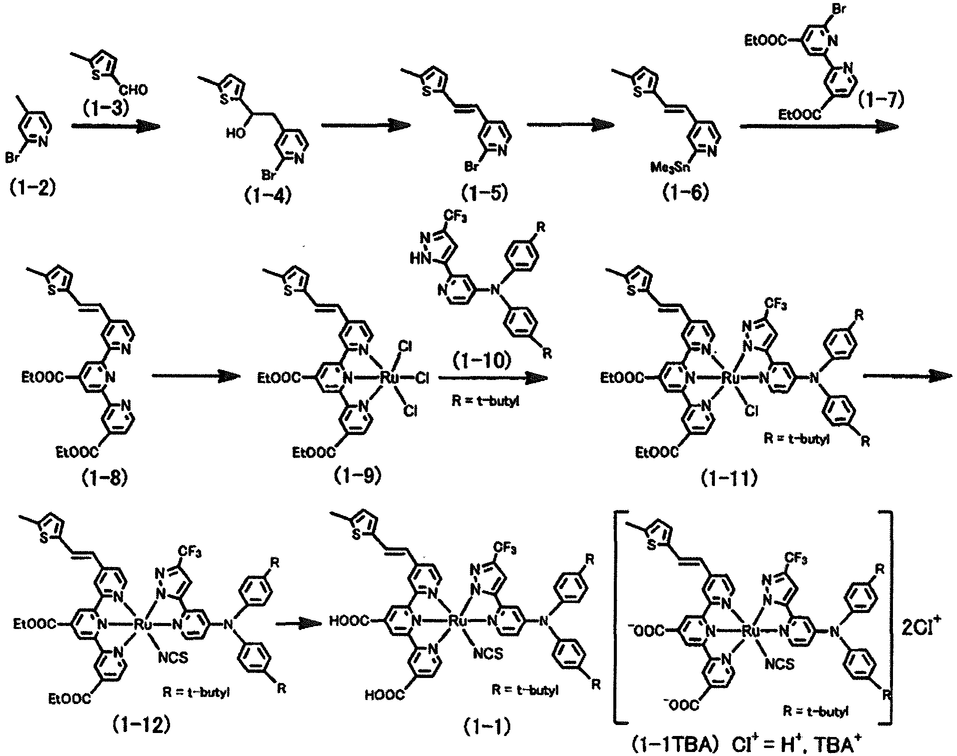

### (i) Synthesis of Compound (1-4)

Into a three-necked flask were introduced 200 mL of tetrahydrofuran (THF) and 5.8 mL of diisopropylamine (DIPA), and the mixture was cooled to 0°C in a nitrogen atmosphere. To this mixed liquid was added 25 mL of n-butyllithium (n-BuLi, 1.6 M solution), and the mixture was stirred at 0°C for 30 minutes. The obtained solution was cooled to -78°C, to this solution was added 5 g of a compound (1-2), and the mixture was stirred for I hour. Furthermore, 4.03 g of a compound (1-3) was added thereto, and the obtained mixture was stirred at room temperature for 2 hours. The obtained reaction liquid was neutralized with ammonium chloride, and the reaction product was extracted with ethyl acetate. The organic phase was concentrated and the concentrated residue was purified by silica gel column chromatography to obtain 8 g of a compound (1-4).

### (ii) Synthesis of Compound (1-5)

Into a three-necked flask were introduced 5 g of the compound (1-4), 100 mL of toluene, 2.7 mL of pyridine, 5.78 g of p-toluenesulfonic acid monohydrate (TsOH·H₂O), and the mixture was heated and refluxed for 3 hours in a nitrogen atmosphere. The obtained reaction liquid was returned to room temperature and neutralized with a saturated aqueous sodium hydrogen carbonate solution, and the reaction product was extracted with ethyl acetate. The organic phase was concentrated, and the concentrated residue was purified by silica gel column chromatography (eluent: n-hexane/ethyl acetate = 9/1 to 4/1) to obtain 4 g of a compound (1-5).

### (iii) Synthesis of Compound (1-8)

Into a three-necked flask were introduced 1.3 g of the compound (1-5), 40 mL of toluene, 0.22 g of Pd(PPh₃)₄, and 1.1 mL of (CH₃)₃SnSn(CH₃)₃, and the mixture was stirred for 2 hours in a nitrogen atmosphere. The obtained reaction liquid was returned to room temperature, 20 mL of water was added thereto, and the mixture was filtered through Celite. To the filtrate was added ethyl acetate to extract the reaction product. The organic phase was concentrated and the concentrated residue was dried at 50°C to obtain a compound (1-6).

Into a 100 mL three-necked flask were introduced the obtained compound (1-6) and additionally, 40 mL of toluene, 0.22 g of Pd(PPh₃)₄, and 1.5 g of a compound (1-7), and the mixture was stirred for 2 hours in a nitrogen atmosphere. The obtained reaction liquid was returned to room temperature and then concentrated, and the concentrated residue was purified by silica gel column chromatography to obtain 1 g of a compound (1-8) which is a diethyl esterified product of a terpyridine compound.

### (iv) Identification of Compound (1-8)

MS (ESI⁺) m/z: 500.4 ([M+H]⁺)
¹H-NMR (400 MHz, solvent: CDCl₃, internal standard substance: chemical shift σ (ppm) by tetramethylsilane (TMS)): 9.16 (1H, m), 9.00 (2H, m), 8.88 (1H, m), 8.67 (1H, m), 8.62 (1H, m), 7.94 (1H, m), 7.50 (1H, m), 7.40 (1H, m), 6.98 (1H, m), 6.86 (1H, m), 6.70 (1H, m), 4.50 (4H, m), 2.51 (3H, s), 1.47 (6H, m)

### (v) Synthesis of Compound (1-9)

To an eggplant flask were introduced 1.0 g of the compound (1-8), 0.63 g of ruthenium chloride, and 20 mL of ethanol, and the mixture was heated and refluxed for 3 hours in a nitrogen atmosphere. The produced precipitate was collected by filtration and washed with ethanol to obtain 1.2 g of a compound (1-9).

### (vi) Synthesis of Compound (1-11)

To an eggplant flask were introduced 0.6 g of the compound (1-9), 0.42 g of the compound (1-10), 10 mL of N,N-dimethylformamide (DMF), and 1 mL of tributylamine, and the mixture was returned to 140°C in a nitrogen atmosphere and heated for 3 hours. The reaction liquid was returned to room temperature and then concentrated, and the concentrated residue was purified by silica gel column chromatography to obtain 0.6 g of a compound (1-11).

### (vii) Synthesis of Compound (1-12)

To an eggplant flask were introduced 0.6 g of the compound (1-11), 0.42 g of ammonium thiocyanate, 40 mL of N,N-dimethylformaldehyde (DMF), and 4 mL of water, and the mixture was heated at 100°C for 3 hours. The reaction liquid was returned to room temperature and then concentrated, and the concentrated residue was purified by silica gel column chromatography to obtain 0.3 g of a compound (1-12).

### (viii) Synthesis of Metal Complex Dye (1-1)

To an eggplant flask were introduced 250 mg of the compound (1-12), 50 mL of N,N-dimethylformaldehyde (DMF), and 1.7 mL of a 1 N aqueous NaOH solution, and the mixture was reacted at room temperature. The obtained reaction liquid was adjusted to pH 2.9 with p-toluenesulfonic acid (TfOH), and the precipitated crystal was collected by filtration and washed with ultrapure water to obtain 200 mg of a metal complex dye (1-1).

### (iX) Synthesis of Metal Complex Dye (1-1TBA)

To an eggplant flask were introduced 100 mg of the metal complex dye (1-1), and 0.24 g of a 10% methanol solution of tetrabutylammonium hydroxide (TBAOH), and the mixture was reacted at room temperature. The obtained reaction liquid was concentrated to obtain 100 mg of a metal complex dye (1-1TBA).

### Identification of Metal Complex Dye (1-1)

MS (ESI⁺) m/z: 1095.5 ([M+H]⁺)

### (Synthesis of Metal Complex Dye (2-1) and Metal Complex Dye (2-1TBA))

According to the following scheme, a metal complex dye (2-1) and a metal complex dye (2-1TBA) were synthesized.

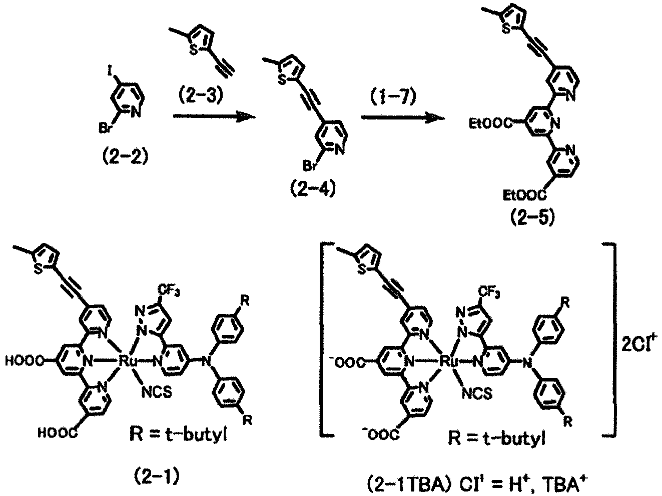

### (i) Synthesis of Compound (2-4)

To a mixture of 3.67 g (12.9 mmol) of the compound (2-2) and 1.63 g (13.3 mmol) of the compound (2-3) was added 52 mL of N,N-dimethylformamide, repeatedly subjected three times to pressure reduction and nitrogen gas substitution, and degassed. 905 mg (1.29 mmol) of bis(triphenylphosphine)dichloropalladium (II), 491 mg (2.58 mmol) of copper (I) iodide, and 13 mL of triethylamine were added thereto, and the mixture was stirred (reacted) at room temperature. After reacting the mixture for 2 hours, to the reaction liquid were added a saturated aqueous ammonium chloride solution and ethyl acetate to extract the reaction product. The organic phase was dried over magnesium sulfate, filtered over magnesium sulfate, and concentrated. The concentrated residue was purified by silica gel column chromatography (eluent: chloroform/hexane = 1/1). The obtained solid was recrystallized with isopropanol to obtain 2.16 g (yield of 60%) of a compound (2-4).

### Identification of Compound (2-4)

MS (ESI⁺) m/z: 280.1 ([M+H]⁺)
¹H-NMR (400 MHz, solvent: CDCl₃, internal standard substance: Chemical shift σ (ppm) by tetramethylsilane (TMS)): 2.52 (3H, s), 6.71 (1H, d), 7.18 (1H, d), 7.28 (1H, d), 7.55 (1H, s), 8.33 (1H, s)

### (ii) Synthesis of Compound (2-5)

1.41 g (5.06 mmol) of the compound (2-4) was dissolved in 126 mL of toluene, and the obtained solution was repeatedly subjected three times to pressure reduction and nitrogen gas substitution, and degassed. 244 mg (0.211 mmol) of tetrakis(triphenylphosphine)palladium (0) and 1.93 g (5.90 mmol) of hexamethylditin were added thereto, and the mixture was heated and refluxed. After reacting the mixture for 4 hours, the reaction liquid was left to be cooled to room temperature, filtered through Celite to remove the insolubles, and further concentrated. To the concentrated residue were added 126 mL of toluene and 1.60 g (4.22 mmol) of the compound (1-7), and the obtained mixture was repeatedly subjected three times to pressure reduction and nitrogen gas substitution, and degassed. 244 mg (0.211 mmol) of tetrakis(triphenylphosphine)palladium (0) was added thereto and the mixture was heated and refluxed. After reacting the mixture for 3 hours, the reaction liquid was left to be cooled to room temperature, filtered through Celite to remove the insolubles, and further concentrated to obtain a crude product. The obtained crude product was purified by silica gel column chromatography (eluent: methanol/chloroform = 5/95), recrystallized with isopropanol to obtain 1.48 g (yield of 70%) of a compound (2-5) which is a diethyl esterified product of a terpyridine compound.

### Identification of Compound (2-5)

MS (ESI⁺) m/z: 498.3 ([M+H]⁺)
¹H-NMR (400 MHz, solvent: CDCl₃, internal standard substance: Chemical shift σ (ppm) by tetramethylsilane (TMS)) = 1.48 (6H, m), 2.52 (3H, s), 4.50 (4H, m), 6.72 (1H, d), 7.21 (1H, d), 7.43 (1H, d), 7.94 (1H, d), 8.67 (1H, s), 8.72 (1H, d), 8.90 (1H, d), 9.01 (2H, s), 9.13 (1H, s)

### (iii) Synthesis of Metal Complex Dye (2-1)

In the same manner as in Synthesis of Metal Complex Dye (1-1) except that the compound (2-5) was used instead of the compound (1-8) in Synthesis of Metal Complex Dye (1-1), a metal complex dye (2-1) was synthesized.

### Identification of Metal Complex Dye (2-1)

MS (ESI⁺) m/z: 1093.5 ([M+H]⁺)

### (iv) Synthesis of Metal Complex Dye (2-1TBA)

In the same manner as in Synthesis of Metal Complex Dye (1-1TBA) except that the metal complex dye (2-1) was used instead of the metal complex dye (1-1) in Synthesis of Metal Complex Dye (1-1TBA), a metal complex dye (2-1TBA) was synthesized.

### (Synthesis of Metal Complex Dye (3-1) and Metal Complex Dye (3-1TBA))

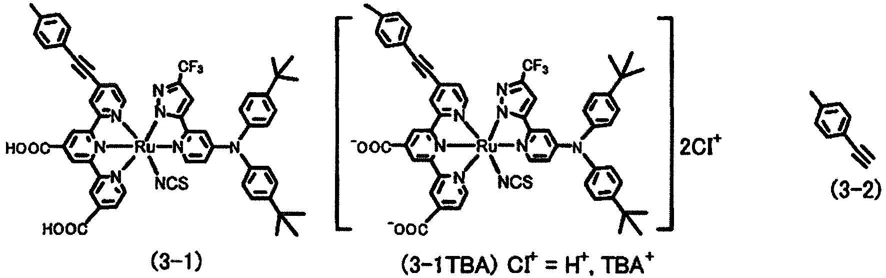

### (i) Synthesis of Metal Complex Dye (3-1)

In the same manner as in Synthesis of Metal Complex Dye (2-1) except that the compound (3-2) was used instead of the compound (2-3) in Synthesis of Metal Complex Dye (2-1), a metal complex dye (3-1) was synthesized.

### Identification of Metal Complex Dye (3-1)

MS (ESI⁺) m/z: 1087.3 ([M+H]⁺)

### (ii) Synthesis of Metal Complex Dye (3-1TBA)

In the same manner as in Synthesis of Metal Complex Dye (1-1TBA) except that the metal complex dye (3-1) was used instead of the metal complex dye (1-1) in Synthesis of Metal Complex Dye (1-1TBA), a metal complex dye (3-1TBA) was synthesized.

### (Synthesis of Metal Complex Dye (4-1) and Metal Complex Dye (4-1TBA))

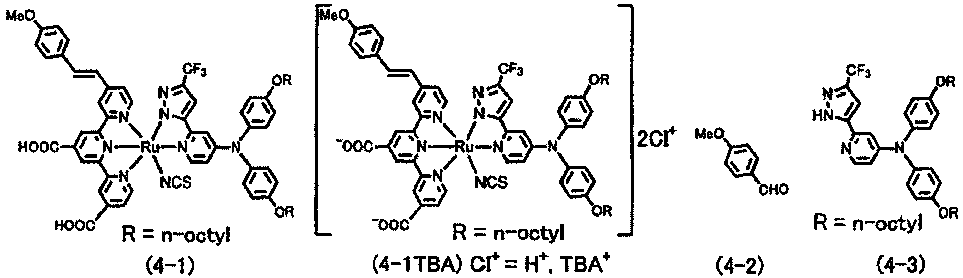

### (i) Synthesis of Metal Complex Dye (4-1)

In the same manner as in Synthesis of Metal Complex Dye (1-1) except that the compound (4-2) was used instead of the compound (1-3) and the compound (4-3) was used instead of the compound (1-10) in Synthesis of Metal Complex Dye (1-1), a metal complex dye (4-1) was synthesized.

### Identification of Metal Complex Dye (4-1)

MS (ESI⁺) m/z: 1249.5 ([M+H]⁺)

### (ii) Synthesis of Metal Complex Dye (4-1 TBA)

In the same manner as in Synthesis of Metal Complex Dye (1-1TBA) except that the metal complex dye (4-1) was used instead of the metal complex dye (1-1) in Synthesis of Metal Complex Dye (1-1TBA), a metal complex dye (4-1TBA) was synthesized.

### (Synthesis of Metal Complex Dye (5-1) and Metal Complex Dye (5-1TBA))

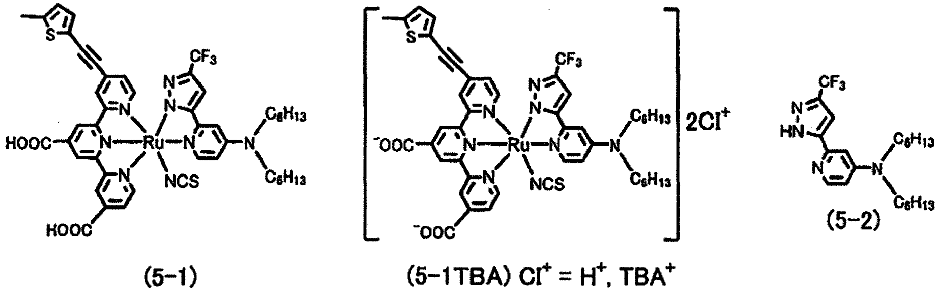

### (i) Synthesis of Metal Complex Dye (5-1)

In the same manner as in Synthesis of Metal Complex Dye (2-1) except that the compound (5-2) was used instead of the compound (1-10) in Synthesis of Metal Complex Dye (2-1), a metal complex dye (5-1) was synthesized.

### Identification of Metal Complex Dye (5-1)

MS (ESI⁺) m/z: 997.2 ([M+H]⁺)

### (ii) Synthesis of Metal Complex Dye (5-1TBA)

In the same manner as in Synthesis of Metal Complex Dye (1-1TBA) except that the metal complex dye (5-1) was used instead of the metal complex dye (1-1) in Synthesis of Metal Complex Dye (1-1TBA), a metal complex dye (5-1TBA) was synthesized.

### (Synthesis of Metal Complex Dye (6-1) and Metal Complex Dye (6-1TBA))

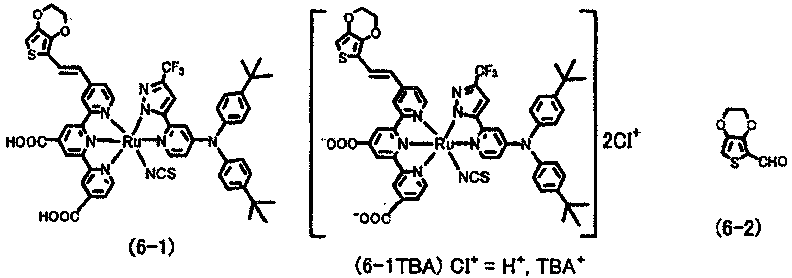

### (i) Synthesis of Metal Complex Dye (6-1)

In the same manner as in Synthesis of Metal Complex Dye (1-1) except that the compound (6-2) was used instead of the compound (1-3) in Synthesis of Metal Complex Dye (1-1), a metal complex dye (6-1) was synthesized.

### Identification of Metal Complex Dye (6-1)

MS (ESI⁺) m/z: 1123.2 ([M+H]⁺)

### (ii) Synthesis of Metal Complex Dye (6-1TBA)

In the same manner as in Synthesis of Metal Complex Dye (1-1TBA) except that the metal complex dye (6-1) was used instead of the metal complex dye (1-1) in Synthesis of Metal Complex Dye (1-1TBA), a metal complex dye (6-1TBA) was synthesized.

### (Synthesis of Metal Complex Dye (7-1) and Metal Complex Dye (7-1TBA))

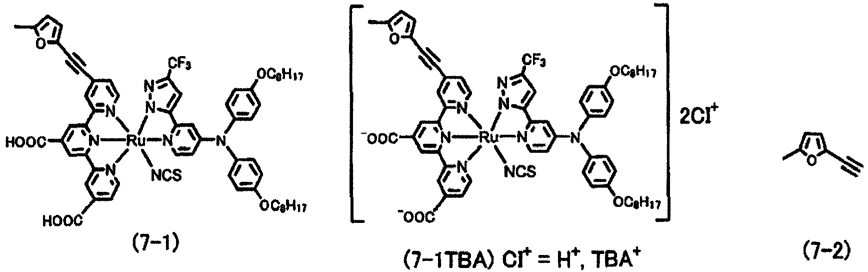

### (i) Synthesis of Metal Complex Dye (7-1)

In the same manner as in Synthesis of Metal Complex Dye (2-1) except that the compound (7-2) was used instead of the compound (2-3) and the compound (4-3) was used instead of the compound (1-10) in Synthesis of Metal Complex Dye (2-1), a metal complex dye (7-1) was synthesized.

### Identification of Metal Complex Dye (7-1)

MS (ESI⁺) m/z: 1221.5 ([M+H]⁺)

### (ii) Synthesis of Metal Complex Dye (7-1TBA)

In the same manner as in Synthesis of Metal Complex Dye (1-1TBA) except that the metal complex dye (7-1) was used instead of the metal complex dye (1-1) in Synthesis of Metal Complex Dye (1-1TBA), a metal complex dye (7-1 TBA) was synthesized.

### (Synthesis of Metal Complex Dye (8-1) and Metal Complex Dye (8-1TBA))

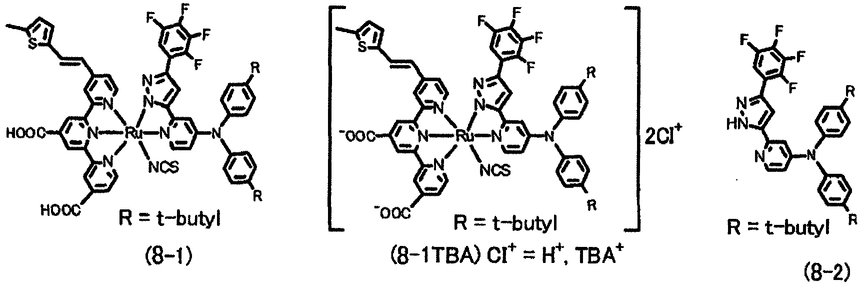

### (i) Synthesis of Metal Complex Dye (8-1)

In the same manner as in Synthesis of Metal Complex Dye (1-1) except that the compound (8-2) was used instead of the compound (1-10) in Synthesis of Metal Complex Dye (1-1), a metal complex dye (8-1) was synthesized.

### Identification of Metal Complex Dye (8-1)

MS (ESI⁺) m/z: 1175.3 ([M+H]⁺)

### (ii) Synthesis of Metal Complex Dye (8-1TBA)

In the same manner as in Synthesis of Metal Complex Dye (1-1 TBA) except that the metal complex dye (8-1) was used instead of the metal complex dye (1-1) in Synthesis of Metal Complex Dye (1-1TBA), a metal complex dye (8-1TBA) was synthesized.

### (Synthesis of Metal Complex Dye (9-1) and Metal Complex Dye (9-1TBA))

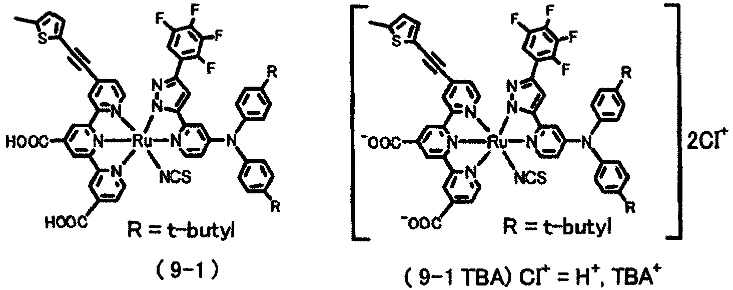

### (i) Synthesis of Metal Complex Dye (9-1)

In the same manner as in Synthesis of Metal Complex Dye (2-1) except that the compound (8-2) was used instead of the compound (1-10) in Synthesis of Metal Complex Dye (2-1), a metal complex dye (9-1) was synthesized.

### Identification of Metal Complex Dye (9-1)

MS (ESI⁺) m/z: 1173.4 ([M+H]⁺)

### (ii) Synthesis of Metal Complex Dye (9-1TBA)

In the same manner as in Synthesis of Metal Complex Dye (1-1TBA) except that the metal complex dye (9-1) was used instead of the metal complex dye (1-1) in Synthesis of Metal Complex Dye (1-ITBA), a metal complex dye (9-1TBA) was synthesized.

### (Synthesis of Metal Complex Dye (10-1) and Metal Complex Dye (10-1TBA))

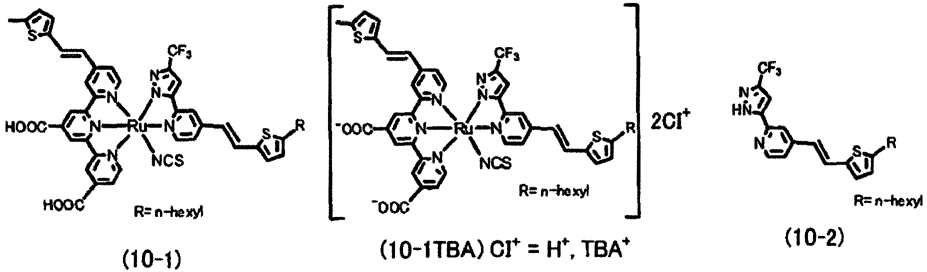

### (i) Synthesis of Metal Complex Dye (10-1)

In the same manner as in Synthesis of Metal Complex Dye (1-1) except that the compound (10-2) was used instead of the compound (1-10) in Synthesis of Metal Complex Dye (1-1), a metal complex dye (10-1) was synthesized.

### Identification of Metal Complex Dye (10-1)

MS (ESI⁺) m/z: 1008.3 ([M+H]⁺)

### (ii) Synthesis of Metal Complex Dye (10-1TBA)

In the same manner as in Synthesis of Metal Complex Dye (1-1TBA) except that the metal complex dye (10-1) was used instead of the metal complex dye (1-1) in Synthesis of Metal Complex Dye (1-1TBA), a metal complex dye (10-1TBA) was synthesized.

### (Synthesis of Metal Complex Dye (11-1) and Metal Complex Dye (11-1TBA))

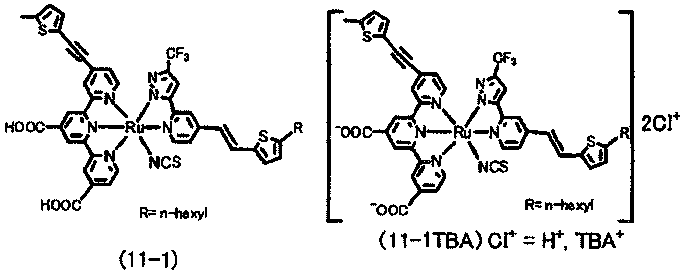

### (i) Synthesis of Metal Complex Dye (11-1)

In the same manner as in Synthesis of Metal Complex Dye (2-1) except that the compound (10-2) was used instead of the compound (1-10) in Synthesis of Metal Complex Dye (2-1), a metal complex dye (11-1) was synthesized.

### Identification of Metal Complex Dye (11-1)

MS (ESI⁺) m/z: 1006.1 ([M+H]⁺)

### (ii) Synthesis of Metal Complex Dye (11-1TBA)

In the same manner as in Synthesis of Metal Complex Dye (1-1TBA) except that the metal complex dye (11-1) was used instead of the metal complex dye (1-1) in Synthesis of Metal Complex Dye (1-1TBA), a metal complex dye (11-1TBA) was synthesized.

### (Synthesis of Metal Complex Dye (12-1) and Metal Complex Dye (12-1TBA))

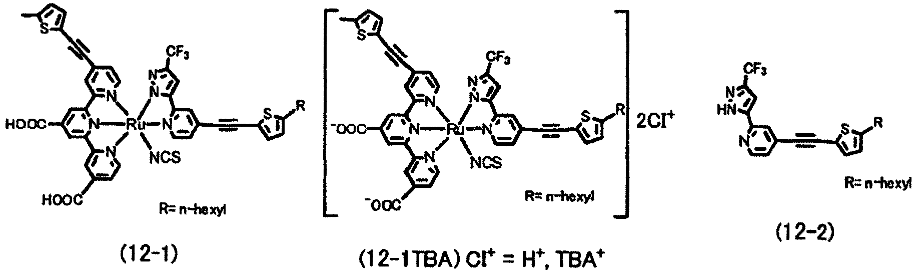

### (i) Synthesis of Metal Complex Dye (12-1)

In the same manner as in Synthesis of Metal Complex Dye (2-1) except that the compound (12-2) was used instead of the compound (1-10) in Synthesis of Metal Complex Dye (2-1), a metal complex dye (12-1) was synthesized.

### Identification of Metal Complex Dye (12-1)

MS (ESI⁺) m/z: 1004.1 ([M+H]⁺)

### (ii) Synthesis of Metal Complex Dye (12-1TBA)

In the same manner as in Synthesis of Metal Complex Dye (1-1TBA) except that the metal complex dye (12-1) was used instead of the metal complex dye (1-1) in Synthesis of Metal Complex Dye (1-1TBA), a metal complex dye (12-1TBA) was synthesized.

### (Synthesis of Metal Complex Dye (13-1) and Metal Complex Dye (13-1TBA))

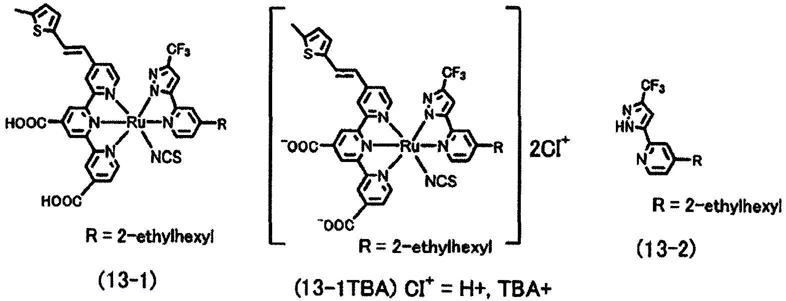

### (i) Synthesis of Metal Complex Dye (13-1)

In the same manner as in Synthesis of Metal Complex Dye (1-1) except that the compound (13-2) was used instead of the compound (1-10) in Synthesis of Metal Complex Dye (1-1), a metal complex dye (13-1) was synthesized.

### Identification of Metal Complex Dye (13-1)

MS (ESI⁺) m/z: 928.2 ([M+H]⁺)

### (ii) Synthesis of Metal Complex Dye (13-1TBA)

In the same manner as in Synthesis of Metal Complex Dye (1-1TBA) except that the metal complex dye (13-1) was used instead of the metal complex dye (1-1) in Synthesis of Metal Complex Dye (1-1TBA), a metal complex dye (13-1TBA) was synthesized.

### (Synthesis of Metal Complex Dye (14-1) and Metal Complex Dye (14-1TBA))

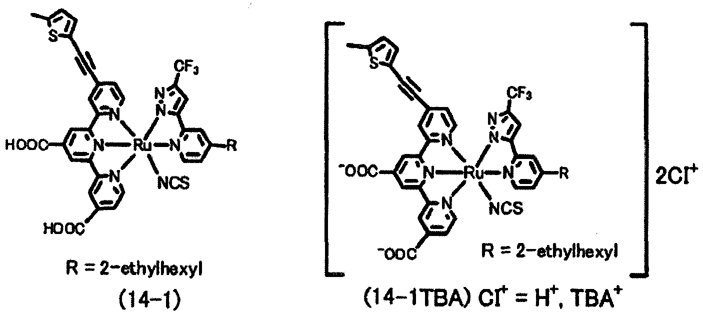

### (i) Synthesis of Metal Complex Dye (14-1)

In the same manner as in Synthesis of Metal Complex Dye (2-1) except that the compound (13-2) was used instead of the compound (1-10) in Synthesis of Metal Complex Dye (2-1), a metal complex dye (14-1) was synthesized.

### Identification of Metal Complex Dye (14-1)

MS (ESI⁺) m/z: 926.3 ([M+H]⁺)

### (ii) Synthesis of Metal Complex Dye (14-1TBA)

In the same manner as in Synthesis of Metal Complex Dye (1-1TBA) except that the metal complex dye (14-1) was used instead of the metal complex dye (1-1) in Synthesis of Metal Complex Dye (1-1TBA), a metal complex dye (14-1TBA) was synthesized.

### (Synthesis of Metal Complex Dye (15-1) and Metal Complex Dye (15-1TBA))

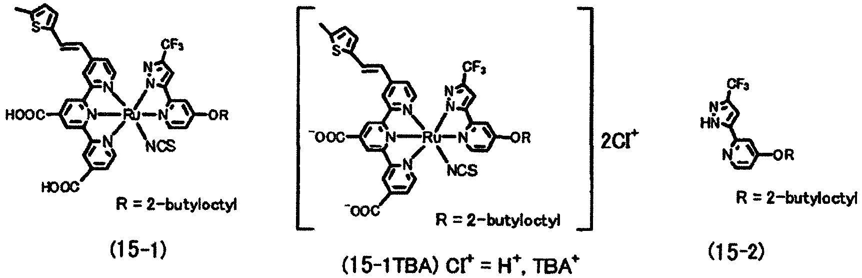

### (i) Synthesis of Metal Complex Dye (15-1)

In the same manner as in Synthesis of Metal Complex Dye (1-1) except that the compound (15-2) was used instead of the compound (1-10) in Synthesis of Metal Complex Dye (1-1), a metal complex dye (15-1) was synthesized.

### Identification of Metal Complex Dye (15-1)

MS (ESI⁺) m/z: 1000.5 ([M+H]⁺)

### (ii) Synthesis of Metal Complex Dye (15-1TBA)

In the same manner as in Synthesis of Metal Complex Dye (1-1TBA) except that the metal complex dye (15-1) was used instead of the metal complex dye (1-1) in Synthesis of Metal Complex Dye (1-1TBA), a metal complex dye (15-1TBA) was synthesized.

### (Synthesis of Metal Complex Dye (16-1) and Metal Complex Dye (16-1TBA))

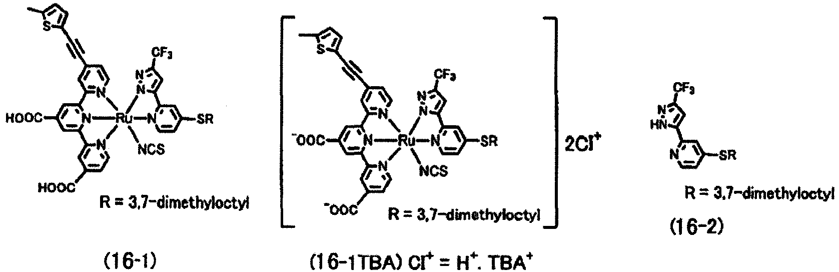

### (i) Synthesis of Metal Complex Dye (16-1)

In the same manner as in Synthesis of Metal Complex Dye (2-1) except that the compound (16-2) was used instead of the compound (1-10) in Synthesis of Metal Complex Dye (2-1), a metal complex dye (16-1) was synthesized.

### Identification of Metal Complex Dye (16-1)

MS (ESI⁺) m/z: 986.1 ([M+H]⁺)

### (ii) Synthesis of Metal Complex Dye (16-1TBA)

In the same manner as in Synthesis of Metal Complex Dye (1-1TBA) except that the metal complex dye (16-1) was used instead of the metal complex dye (1-1) in Synthesis of Metal Complex Dye (1-1TBA), a metal complex dye (16-1TBA) was synthesized.

### (Synthesis of Metal Complex Dye (17-1) and Metal Complex Dye (17-1TBA))

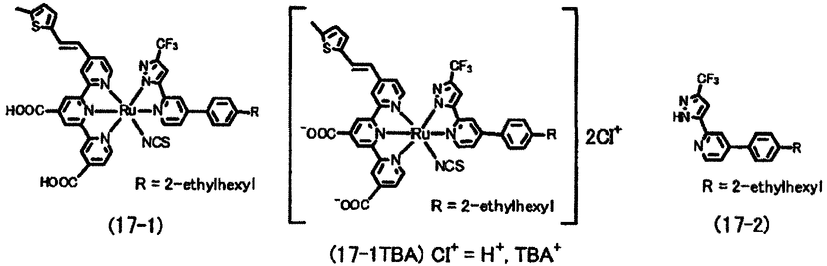

### (i) Synthesis of Metal Complex Dye (17-1)

In the same manner as in Synthesis of Metal Complex Dye (1-1) except that the compound (17-2) was used instead of the compound (1-10) in Synthesis of Metal Complex Dye (1-1), a metal complex dye (17-1) was synthesized.

### Identification of Metal Complex Dye (17-1)

MS (ESI⁺) m/z: 1004.3 ([M+H]⁺)

### (ii) Synthesis of Metal Complex Dye (17-1TBA)

In the same manner as in Synthesis of Metal Complex Dye (1-1TBA) except that the metal complex dye (17-1) was used instead of the metal complex dye (1-1) in Synthesis of Metal Complex Dye (1-1TBA), a metal complex dye (17-1TBA) was synthesized.

### (Synthesis of Metal Complex Dye (18-1) and Metal Complex Dye (18-1TBA))

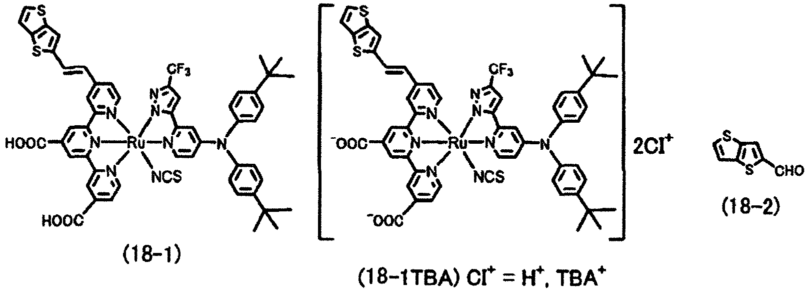

### (i) Synthesis of Metal Complex Dye (18-1)

In the same manner as in Synthesis of Metal Complex Dye (1-1) except that the compound (18-2) was used instead of the compound (1-3) in Synthesis of Metal Complex Dye (1-1), a metal complex dye (18-1) was synthesized.

### Identification of Metal Complex Dye (18-1)

MS (ESI⁺) m/z: 1137.2 ([M+H]⁺)

### (ii) Synthesis of Metal Complex Dye (18-1 TBA)

In the same manner as in Synthesis of Metal Complex Dye (1-1TBA) except that the metal complex dye (18-1) was used instead of the metal complex dye (1-1) in Synthesis of Metal Complex Dye (1-1TBA), a metal complex dye (18-1TBA) was synthesized.

### (Synthesis of Metal Complex Dye (19-1) and Metal Complex Dye (19-1TBA))

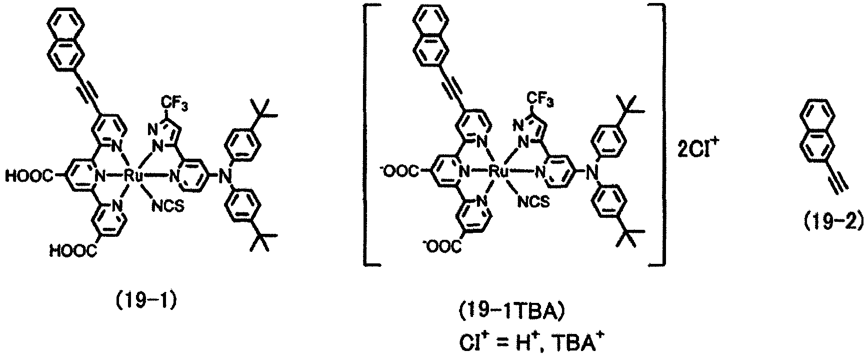

### (i) Synthesis of Metal Complex Dye (19-1)

In the same manner as in Synthesis of Metal Complex Dye (2-1) except that the compound (19-2) was used instead of the compound (2-3) in Synthesis of Metal Complex Dye (2-1), a metal complex dye (19-1) was synthesized.

### Identification of Metal Complex Dye (19-1)

MS (ESI⁺) m/z: 1123.3 ([M+H]⁺)

### (ii) Synthesis of Metal Complex Dye (19-1TBA)

In the same manner as in Synthesis of Metal Complex Dye (1-1TBA) except that the metal complex dye (19-1) was used instead of the metal complex dye (1-1) in Synthesis of Metal Complex Dye (1-1TBA), a metal complex dye (19-1TBA) was synthesized.

### (Synthesis of Metal Complex Dye (20-1) and Metal Complex Dye (20-1TBA))

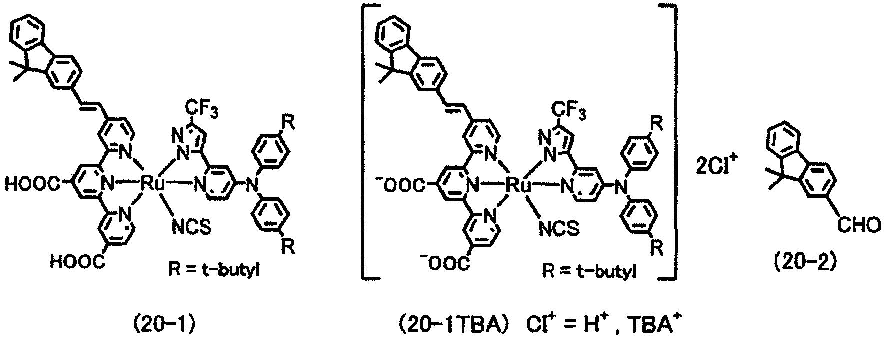

### (i) Synthesis of Metal Complex Dye (20-1)

In the same manner as in Synthesis of Metal Complex Dye (1-1) except that the compound (20-2) was used instead of the compound (1-3) in Synthesis of Metal Complex Dye (1-1), a metal complex dye (20-1) was synthesized.

### Identification of Metal Complex Dye (20-1)

MS (ESI⁺) m/z: 1191.5 ([M+H]⁺)

### (ii) Synthesis of Metal Complex Dye (20-1TBA)

In the same manner as in Synthesis of Metal Complex Dye (1-1TBA) except that the metal complex dye (20-1) was used instead of the metal complex dye (1-1) in Synthesis of Metal Complex Dye (1-1TBA), a metal complex dye (20-1TBA) was synthesized.

### (Synthesis of Metal Complex Dye (21-1) and Metal Complex Dye (21-1TBA))

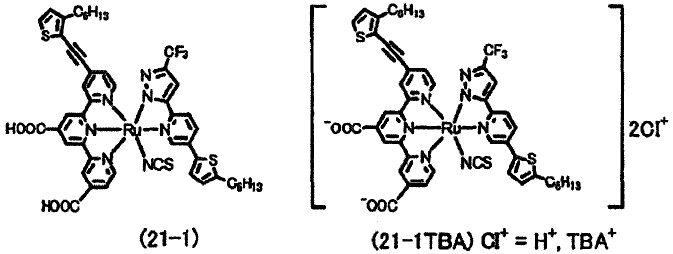

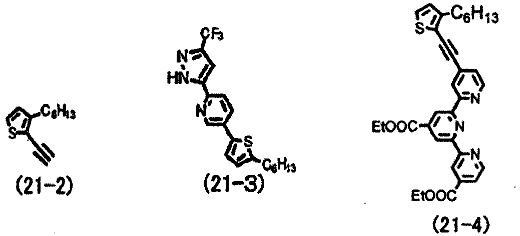

### (i) Synthesis of Metal Complex Dye (21-1)

In the same manner as in Synthesis of Metal Complex Dye (2-1) except that the compound (21-2) was used instead of the compound (2-3) and the compound (21-3) was used instead of the compound (1-10) in Synthesis of Metal Complex Dye (2-1), a metal complex dye (21-1) was synthesized.

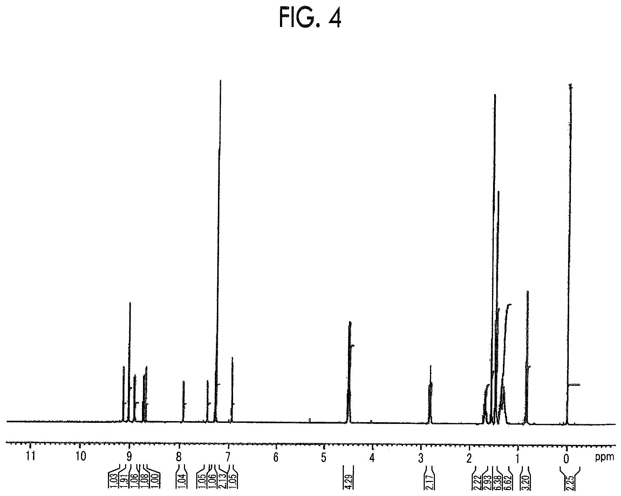

The compound (21-4) is a diethyl esterified product of a terpyridine compound, and was synthesized in the same manner as in Synthesis of Compound (2-5). The compound (21-4) was confirmed from a ¹H-NMR spectrum shown in Fig. 4. In ¹H-NMR, measurement was carried out at a proton resonance frequency of 400 MHz, using a CDCl₃ solvent and tetramethylsilane (TMS) as an internal standard substance.

### Identification of Metal Complex Dye (21-1)

MS (ESI⁺) m/z: 1050.5 ([M+H]⁺)

### (ii) Synthesis of Metal Complex Dye (21-1TBA)

In the same manner as in Synthesis of Metal Complex Dye (2-1TBA) except that the metal complex dye (21-1) was used instead of the metal complex dye (2-1) in Synthesis of Metal Complex Dye (2-1 TBA), a metal complex dye (21-1 TBA) was synthesized.

### (Synthesis of Metal Complex Dye (22-1) and Metal Complex Dye (22-1TBA))

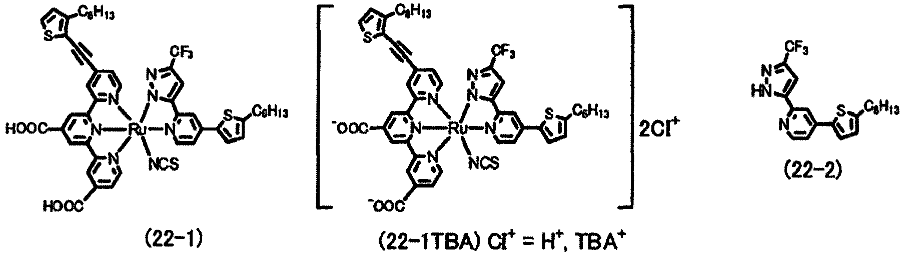

### (i) Synthesis of Metal Complex Dye (22-1)

In the same manner as in Synthesis of Metal Complex Dye (21-1) except that the compound (22-2) was used instead of the compound (21-3) in Synthesis of Metal Complex Dye (21-1), a metal complex dye (22-1) was synthesized.

### Identification of Metal Complex Dye (22-1)

MS (ESI⁺) m/z: 1050.2 ([M+H]⁺)

### (ii) Synthesis of Metal Complex Dye (22-1TBA)

In the same manner as in Synthesis of Metal Complex Dye (21-1 TBA) except that the metal complex dye (22-1) was used instead of the metal complex dye (21-1) in Synthesis of Metal Complex Dye (21-1TBA), a metal complex dye (22-1TBA) was synthesized.

### (Synthesis of Metal Complex Dye (23-1) and Metal Complex Dye (23-1TBA))

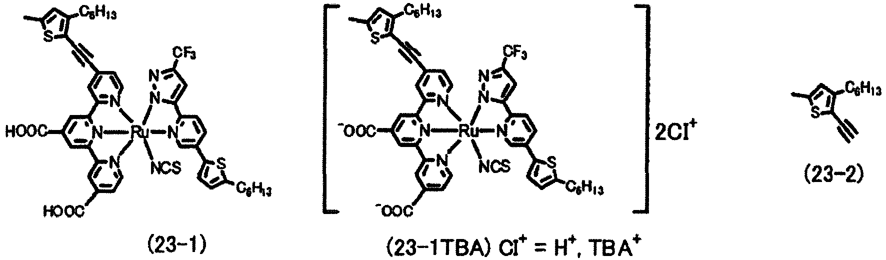

### (i) Synthesis of Metal Complex Dye (23-1)

In the same manner as in Synthesis of Metal Complex Dye (21-1) except that the compound (23-2) was used instead of the compound (21-2) in Synthesis of Metal Complex Dye (21-1), a metal complex dye (23-1) was synthesized.

### Identification of Metal Complex Dye (23-1)

MS (ESI⁺) m/z: 1064.4 ([M+H]⁺)

### (ii) Synthesis of Metal Complex Dye (23-1TBA)

In the same manner as in Synthesis of Metal Complex Dye (21-1 TBA) except that the metal complex dye (23-1) was used instead of the metal complex dye (21-1) in Synthesis of Metal Complex Dye (21-1TBA), a metal complex dye (23-1 TBA) was synthesized.

### (Synthesis of Metal Complex Dye (24-1) and Metal Complex Dye (24-1TBA))

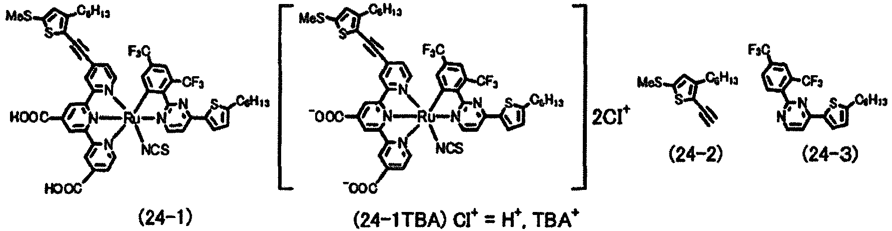

### (i) Synthesis of Metal Complex Dye (24-1)

In the same manner as in Synthesis of Metal Complex Dye (2-1) except that the compound (24-2) was used instead of the compound (2-3) and the compound (24-3) was used instead of the compound (1-10) in Synthesis of Metal Complex Dye (2-1), a metal complex dye (24-1) was synthesized.

### Identification of Metal Complex Dye (24-1)

MS (ESI⁺) m/z: 1175.8 ([M+H]⁺)

### (ii) Synthesis of Metal Complex Dye (24-1TBA)

In the same manner as in Synthesis of Metal Complex Dye (2-1TBA) except that the metal complex dye (24-1) was used instead of the metal complex dye (2-1) in Synthesis of Metal Complex Dye (2-1TBA), a metal complex dye (24-1TBA) was synthesized.

### (Synthesis of Metal Complex Dye (25-1) and Metal Complex Dye (25-1TBA))

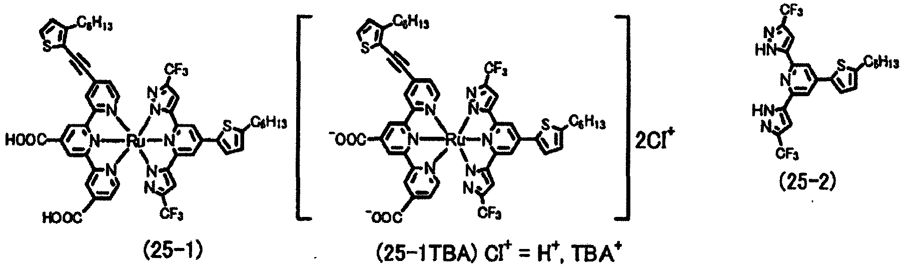

### (i) Synthesis of Metal Complex Dye (25-1)

By the same method as in Synthesis of Metal Complex Dye (21-1) except that the compound (25-2) was used instead of the compound (21-3) in Synthesis of Metal Complex Dye (21-1), or a method equivalent thereto, a metal complex dye (25-1) was synthesized.

### Identification of Metal Complex Dye (25-1)

MS (ESI⁺) m/z: 1125.6 ([M+H]⁺)

### (ii) Synthesis of Metal Complex Dye (25-1TBA)

In the same manner as in Synthesis of Metal Complex Dye (21-1 TBA) except that the metal complex dye (25-1) was used instead of the metal complex dye (21-1) in Synthesis of Metal Complex Dye (21-1TBA), a metal complex dye (25-1 TBA) was synthesized.

### (Synthesis of Metal Complex Dye (26-1) and Metal Complex Dye (26-1TBA))

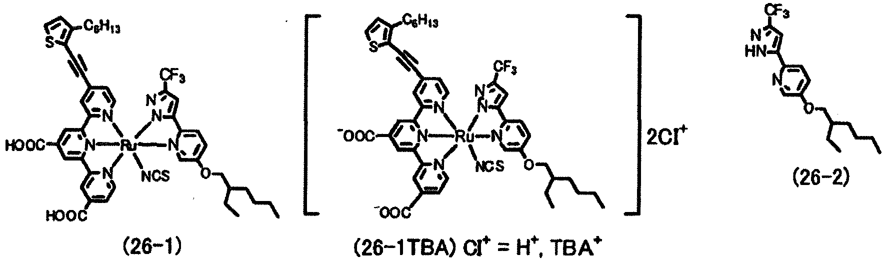

### (i) Synthesis of Metal Complex Dye (26-1)

In the same manner as in Synthesis of Metal Complex Dye (21-1) except that the compound (26-2) was used instead of the compound (21-3) in Synthesis of Metal Complex Dye (21-1), a metal complex dye (26-1) was synthesized.

### Identification of Metal Complex Dye (26-1)

MS (ESI⁺) m/z: 1012.1 ([M+H]⁺)

### (ii) Synthesis of Metal Complex Dye (26-1TBA)

In the same manner as in Synthesis of Metal Complex Dye (21-1TBA) except that the metal complex dye (26-1) was used instead of the metal complex dye (21-1) in Synthesis of Metal Complex Dye (21-1TBA), a metal complex dye (26-1 TBA) was synthesized.

### (Synthesis of Metal Complex Dye (27-1) and Metal Complex Dye (27-1TBA))

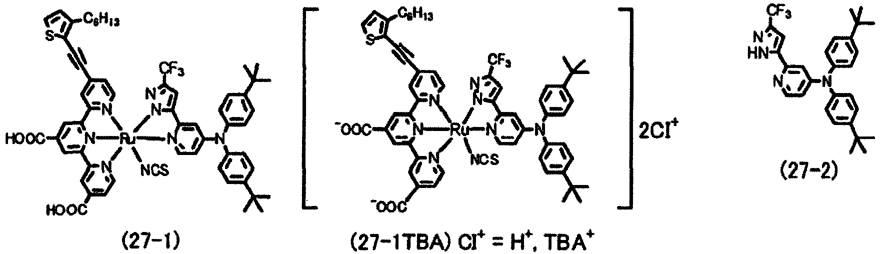

### (i) Synthesis of Metal Complex Dye (27-1)

In the same manner as in Synthesis of Metal Complex Dye (21-1) except that the compound (27-2) was used instead of the compound (21-3) in Synthesis of Metal Complex Dye (21-1), a metal complex dye (27-1) was synthesized.

### Identification of Metal Complex Dye (27-1)

MS (ESI⁺) m/z: 1163.4 ([M+H]⁺)

### (ii) Synthesis of Metal Complex Dye (27-1TBA)

In the same manner as in Synthesis of Metal Complex Dye (21-1TBA) except that the metal complex dye (27-1) was used instead of the metal complex dye (21-1) in Synthesis of Metal Complex Dye (21-1TBA), a metal complex dye (27-1TBA) was synthesized.

### (Synthesis of Metal Complex Dye (28-1) and Metal Complex Dye (28-1TBA))

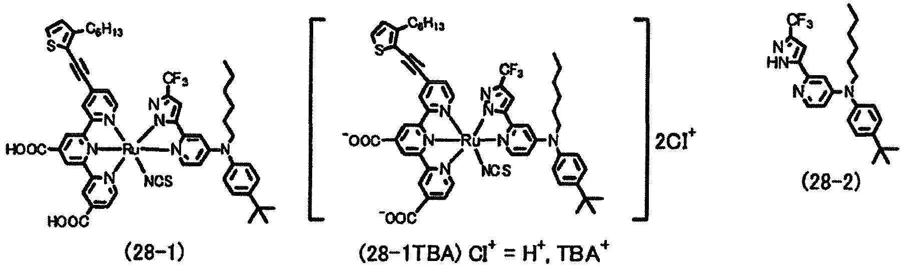

### (i) Synthesis of Metal Complex Dye (28-1)

In the same manner as in Synthesis of Metal Complex Dye (21-1) except that the compound (28-2) was used instead of the compound (21-3) in Synthesis of Metal Complex Dye (21-1), a metal complex dye (28-1) was synthesized.

### Identification of Metal Complex Dye (28-1)

MS (ESI⁺) m/z: 1115.4 ([M+H]⁺)

### (ii) Synthesis of Metal Complex Dye (28-1TBA)

In the same manner as in Synthesis of Metal Complex Dye (21-1 TBA) except that the metal complex dye (28-1) was used instead of the metal complex dye (21-1) in Synthesis of Metal Complex Dye (21-1TBA), a metal complex dye (28-1TBA) was synthesized.

### (Synthesis of Metal Complex Dye (29-1) and Metal Complex Dye (29-1TBA))

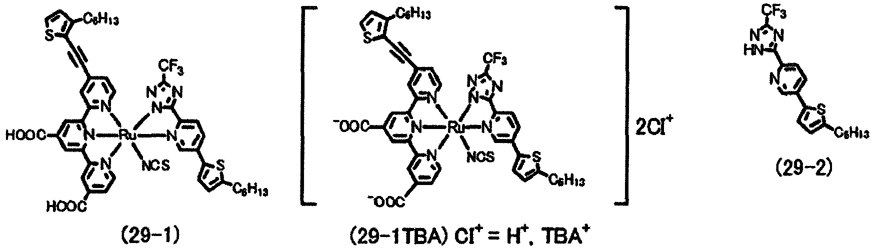

### (i) Synthesis of Metal Complex Dye (29-1)

In the same manner as in Synthesis of Metal Complex Dye (21-1) except that the compound (29-2) was used instead of the compound (21-3) in Synthesis of Metal Complex Dye (21-1), a metal complex dye (29-1) was synthesized.

### Identification of Metal Complex Dye (29-1)

MS (ESI⁺) m/z: 1051.2 ([M+H]⁺)

### (ii) Synthesis of Metal Complex Dye (29-1TBA)

In the same manner as in Synthesis of Metal Complex Dye (21-1TBA) except that the metal complex dye (29-1) was used instead of the metal complex dye (21-1) in Synthesis of Metal Complex Dye (21-1TBA), a metal complex dye (29-1TBA) was synthesized.

### (Synthesis of Metal Complex Dye (30-1) and Metal Complex Dye (30-1TBA))

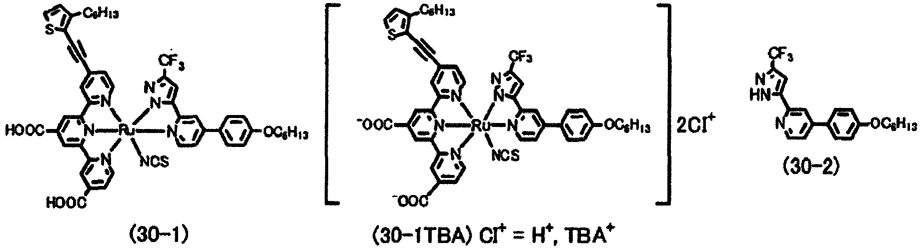

### (i) Synthesis of Metal Complex Dye (30-1)

In the same manner as in Synthesis of Metal Complex Dye (21-1) except that the compound (30-2) was used instead of the compound (21-3) in Synthesis of Metal Complex Dye (21-1), a metal complex dye (30-1) was synthesized.

### Identification of Metal Complex Dye (30-1)

MS (ESI⁺) m/z: 1060.2 ([M+H]⁺)

### (ii) Synthesis of Metal Complex Dye (30-1 TBA)

In the same manner as in Synthesis of Metal Complex Dye (21-1TBA) except that the metal complex dye (30-1) was used instead of the metal complex dye (21-1) in Synthesis of Metal Complex Dye (21-1 TBA), a metal complex dye (30-1 TBA) was synthesized.

### (Synthesis of Metal Complex Dye (31-1) and Metal Complex Dye (31-1TBA))

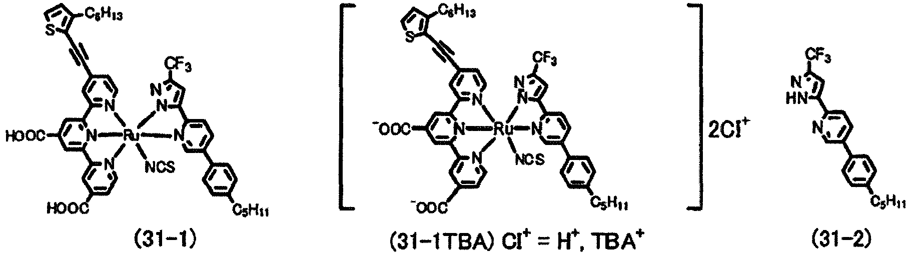

### (i) Synthesis of Metal Complex Dye (31-1)

In the same manner as in Synthesis of Metal Complex Dye (21-1) except that the compound (31-2) was used instead of the compound (21-3) in Synthesis of Metal Complex Dye (21-1), a metal complex dye (31-1) was synthesized.

### Identification of Metal Complex Dye (31-1)

MS (ESI⁺) m/z: 1030.2 ([M+H]⁺)

### (ii) Synthesis of Metal Complex Dye (31-1TBA)

In the same manner as in Synthesis of Metal Complex Dye (21-1TBA) except that the metal complex dye (31-1) was used instead of the metal complex dye (21-1) in Synthesis of Metal Complex Dye (21-1TBA), a metal complex dye (31-1TBA) was synthesized.

### (i) Synthesis of Metal Complex Dye (32-1)

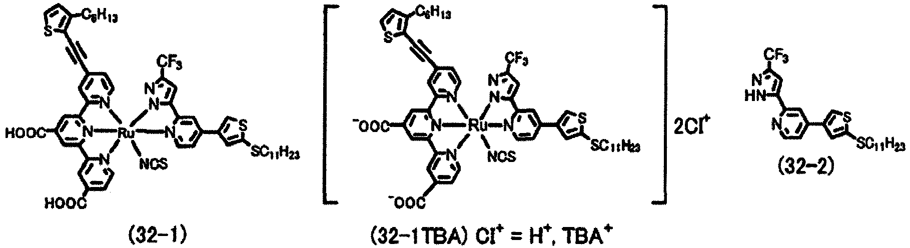

In the same manner as in Synthesis of Metal Complex Dye (21-1) except that the compound (32-2) was used instead of the compound (21-3) in Synthesis of Metal Complex Dye (21-1), a metal complex dye (32-1) was synthesized.

### Identification of Metal Complex Dye (32-1)

MS (ESI⁺) m/z: 1152.6 ([M+H]⁺)

### (ii) Synthesis of Metal Complex Dye (32-1TBA)

In the same manner as in Synthesis of Metal Complex Dye (21-1TBA) except that the metal complex dye (32-1) was used instead of the metal complex dye (21-1) in Synthesis of Metal Complex Dye (21-1TBA), a metal complex dye (32-1TBA) was synthesized.

### (Synthesis of Metal Complex Dye (33-1), Metal Complex Dye (33-1TBA), Metal Complex Dye (33-1K), and Metal Complex Dye (33-1Na))

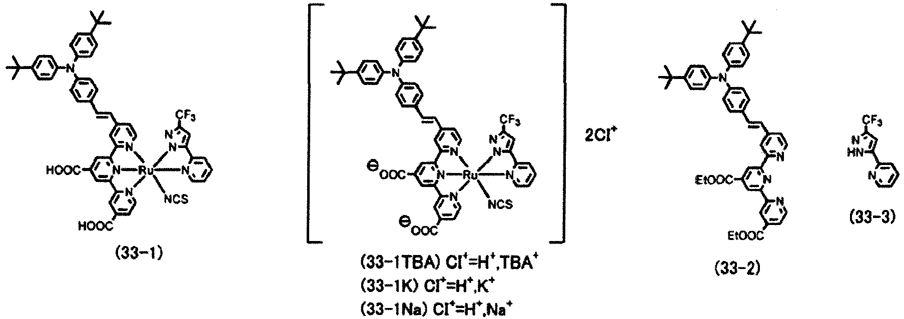

### (i) Synthesis of Metal Complex Dye (33-1)

In the same manner as in Synthesis of Metal Complex Dye (1-1) except that the compound (33-2) was used instead of the compound (1-8) and the compound (33-3) was used instead of the compound (1-10) in Synthesis of Metal Complex Dye (1-1), a metal complex dye (33-1) was synthesized.

### Identification of Metal Complex Dye (33-1)

MS (ESI⁺) m/z: 1075.2 ([M+H]⁺)

### (ii) Synthesis of Metal Complex Dye (33-1TBA)

In the same manner as in Synthesis of Metal Complex Dye (21-1TBA) except that the metal complex dye (33-1) was used instead of the metal complex dye (21-1) in Synthesis of Metal Complex Dye (21-1TBA), a metal complex dye (33-1TBA) was synthesized.

### (iii) Synthesis of Metal Complex Dye (33-1K)

In the same manner as in Synthesis of Metal Complex Dye (33-1 TBA) except that the metal complex dye (33-1) and an equivalent amount of potassium hydroxide (KOH) were used instead of tetrabutylammonium hydroxide (TBAOH) in Synthesis of Metal Complex Dye (33-1TBA), a metal complex dye (33-1K) was synthesized.

### (iv) Synthesis of Metal Complex Dye (33-1Na)

In the same manner as in Synthesis of Metal Complex Dye (33-1TBA) except that the metal complex dye (33-1) and an equivalent amount of sodium hydroxide (NaOH) were used instead of tetrabutylammonium hydroxide (TBAOH) in Synthesis of Metal Complex Dye (33-1TBA), a metal complex dye (33-1Na) was synthesized.

### (Synthesis of Metal Complex Dye (34-1), Metal Complex Dye (34-1TBA), Metal Complex Dye (34-1K), and Metal Complex Dye (34-1Na))

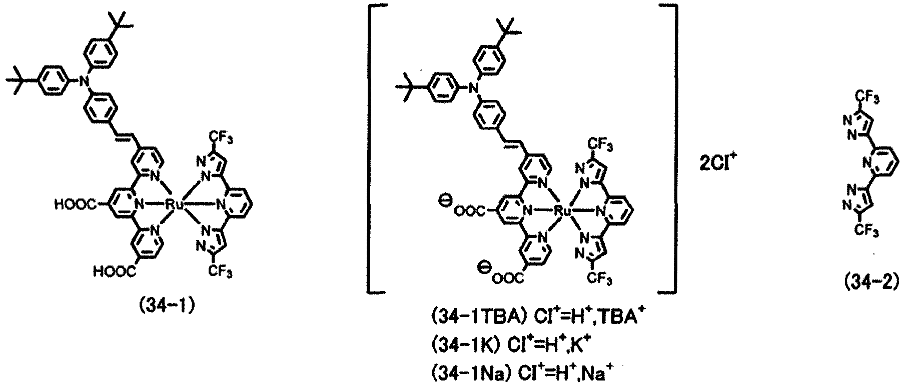

### (i) Synthesis of Metal Complex Dye (34-1)

In the same manner as in Synthesis of Metal Complex Dye (25-1) except that the compound (33-2) was used, and the compound (34-2) was used instead of the compound (25-2) in Synthesis of Metal Complex Dye (25-1), a metal complex dye (34-1) was synthesized.

### Identification of Metal Complex Dye (34-1)

MS (ESI⁺) m/z: 1150.3 ([M+H]⁺)

### (ii) Synthesis of Metal Complex Dye (34-1TBA)

In the same manner as in Synthesis of Metal Complex Dye (21-1TBA) except that the metal complex dye (34-1) was used instead of the metal complex dye (21-1) in Synthesis of Metal Complex Dye (21-1 TBA), a metal complex dye (34-1 TBA) was synthesized.

### (iii) Synthesis of Metal Complex Dye (34-1K)

In the same manner as in Synthesis of Metal Complex Dye (33-1K) except that the metal complex dye (34-1) was used instead of the metal complex dye (33-1) in Synthesis of Metal Complex Dye (33-1K), a metal complex dye (34-1K) was synthesized.

### (iv) Synthesis of Metal Complex Dye (34-1Na)

In the same manner as in Synthesis of Metal Complex Dye (33-1Na) except that the metal complex dye (34-1) was used instead of the metal complex dye (33-1) in Synthesis of Metal Complex Dye (33-1Na), a metal complex dye (34-1Na) was synthesized.

### (Synthesis of Metal Complex Dye (35-1), Metal Complex Dye (35-1TBA), Metal Complex Dye (35-1K), and Metal Complex Dye (35-1Na))

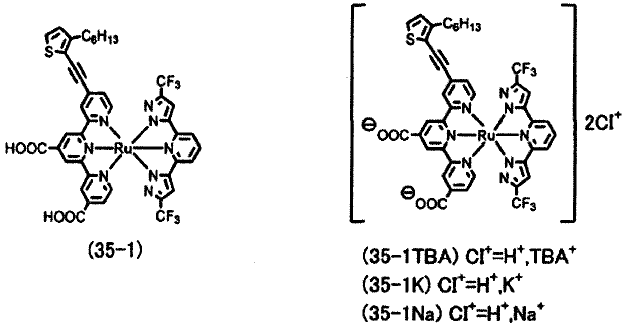

### (i) Synthesis of Metal Complex Dye (35-1)

In the same manner as in Synthesis of Metal Complex Dye (25-1) except that the compound (34-2) was used instead of the compound (25-2) in Synthesis of Metal Complex Dye (25-1), a metal complex dye (35-1) was synthesized.

### Identification of Metal Complex Dye (35-1)

MS (ESI⁺) m/z: 959.1 ([M+H]⁺)

### (ii) Synthesis of Metal Complex Dye (35-1 TBA)

In the same manner as in Synthesis of Metal Complex Dye (21-1 TBA) except that the metal complex dye (35-1) was used instead of the metal complex dye (21-1) in Synthesis of Metal Complex Dye (21-1TBA), a metal complex dye (35-1TBA) was synthesized.

### (iii) Synthesis of Metal Complex Dye (35-1K)

In the same manner as in Synthesis of Metal Complex Dye (33-1K) except that the metal complex dye (35-1) was used instead of the metal complex dye (33-1) in Synthesis of Metal Complex Dye (33-1K), a metal complex dye (35-1K) was synthesized.

### (iv) Synthesis of Metal Complex Dye (35-1Na)

In the same manner as in Synthesis of Metal Complex Dye (33-1Na) except that the metal complex dye (35-1) was used instead of the metal complex dye (33-1) in Synthesis of Metal Complex Dye (33-1Na), a metal complex dye (35-1Na) was synthesized.

### (Synthesis of Metal Complex Dye (36-1), Metal Complex Dye (36-1TBA), Metal Complex Dye (36-1K), and Metal Complex Dye (36-1Na))

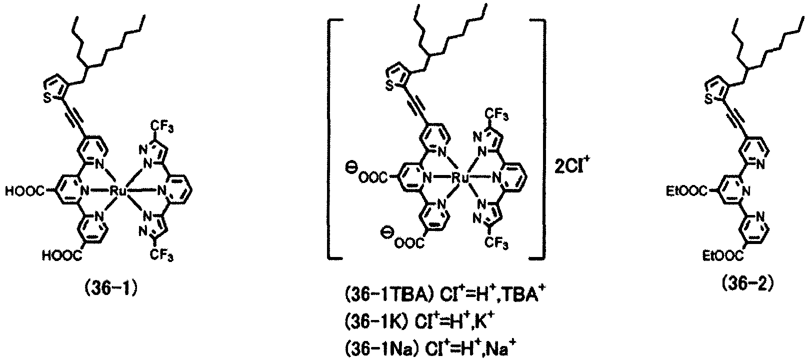

### (i) Synthesis of Metal Complex Dye (36-1)

In the same manner as in Synthesis of Metal Complex Dye (25-1) except that the compound (36-2) was used, and the compound (34-2) was used instead of the compound (25-2) in Synthesis of Metal Complex Dye (25-1), a metal complex dye (36-1) was synthesized.

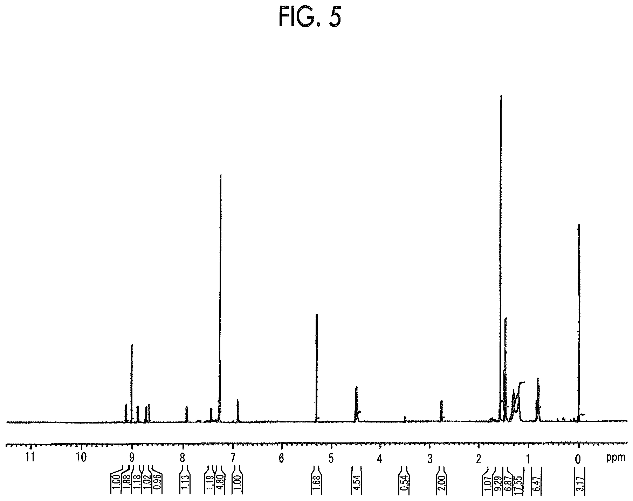

The compound (36-2) is a diethyl esterified product of a terpyridine compound, and was synthesized in the same manner as in Synthesis of Compound (2-5). The compound (36-2) was confirmed from a ¹H-NMR spectrum shown in Fig. 5. In ¹H-NMR, measurement was carried out at a proton resonance frequency of 400 MHz, using a CDCl₃ solvent and tetramethylsilane (TMS) as an internal standard substance.

### Identification of Metal Complex Dye (36-1)

MS (ESI⁺) m/z: 1043.2 ([M+H]⁺)

### (ii) Synthesis of Metal Complex Dye (36-1TBA)

In the same manner as in Synthesis of Metal Complex Dye (21-1TBA) except that the metal complex dye (36-1) was used instead of the metal complex dye (21-1) in Synthesis of Metal Complex Dye (21-1TBA), a metal complex dye (36-1TBA) was synthesized.

### (iii) Synthesis of Metal Complex Dye (36-1K)

In the same manner as in Synthesis of Metal Complex Dye (33-1K) except that the metal complex dye (36-1) was used instead of the metal complex dye (33-1) in Synthesis of Metal Complex Dye (33-1K), a metal complex dye (36-1K) was synthesized.

### (iv) Synthesis of Metal Complex Dye (36-1Na)

In the same manner as in Synthesis of Metal Complex Dye (33-1Na) except that the metal complex dye (36-1) was used instead of the metal complex dye (33-1) in Synthesis of Metal Complex Dye (33-1Na), a metal complex dye (36-1Na) was synthesized.

### (Measurement of Visible Absorption Spectrum)

The visible absorption spectrums of the synthesized metal complex dyes (1-1), (2-1), (21-1), and (26-1) to (28-1) were measured.

The metal complex dye (1-1) was dissolved in a TBAOH methanol solution at a concentration of 340 mmol/L to prepare a TBAOH methanol solution having a concentration of the metal complex dye (1-1) of 17 µmole/L. By using this measurement solution, the light absorption spectrum of the metal complex dye (1-1) was measured. As the measurement device, "UV-3600" (manufactured by Shimadzu Corporation) was used.

Furthermore, in the same manner, the visible absorption spectrums of the metal complex dyes (2-1), (21-1), and (26-1) to (28-1) were measured.

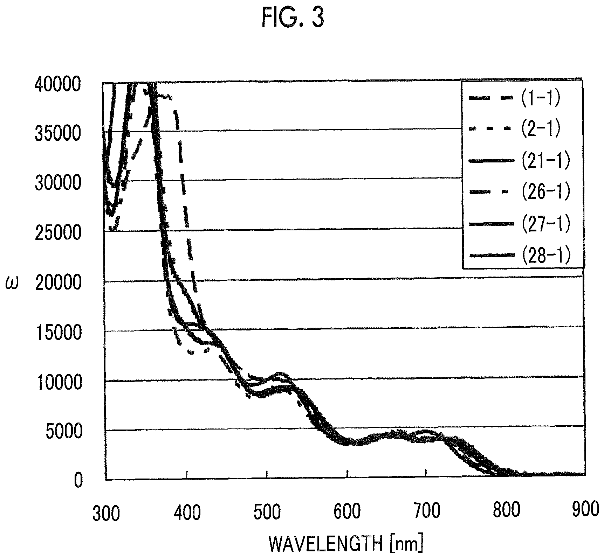

The obtained absorption spectrums are shown in Fig. 3. In the absorption spectrum in Fig. 3, the ε at the vertical axis is a molar light absorption coefficient (L/mol·cm). As shown in Fig. 3, it could be confirmed that the metal complex dyes (1-1), (2-1), (21-1), and (26-1) to (28-1) all have wider bottoms of absorption peaks up to a long-wavelength range having a wavelength of more than 700 nm.

### Example 2 (Production of Dye-Sensitized Solar Cell)

Using the metal complex dye synthesized in Example 1 or each of the following comparative compounds (C1) to (C3), a dye-sensitized solar cell 20 (in a dimension of 5 mm × 5 mm) shown in Fig. 2 was produced and its performance was evaluated according to the procedure shown below. The results are shown in Table 1.

### (Manufacture of Light-Receiving Electrode Precursor A)

An electrically conductive support 41 was prepared in which a fluorine-doped SnO₂ electrically-conductive film (transparent electrically-conductive film 43, film thickness of 500 nm) was formed on a glass substrate (substrate 44, thickness of 4 mm). Further, a titania paste "18NR-T" (manufactured by DyeSol) was screen-printed on the SnO₂ electrically-conductive film, followed by drying at 120°C. Then, the dried titania paste "18NR-T" was screen-printed, followed by drying at 120°C for 1 hour. Thereafter, the dried titania paste was calcined at 500°C to form a semiconductor layer 45 (film thickness; 10 µm). Further, a titania paste "18NR-AO" (manufactured by DyeSol) was screen-printed on this semiconductor layer 45, followed by drying at 120°C for 1 hour. Then, the dried titania paste was calcined at 500°C to form a light-scattering layer 46 (film thickness; 5 µm) on the semiconductor layer 45. Thus, a photoconductor layer 42 (the area of the light-receiving surface; 5 mm × 5 mm, film thickness; 15 µm, a metal complex dye is not carried) was formed on the SnO₂ electrically-conductive film, thereby manufacturing a light-receiving electrode precursor A not carrying a metal complex dye.

### (Manufacture of Light-Receiving Electrode Precursor B)

An electrically conductive support 41 was prepared in which a fluorine-doped SnO₂ electrically-conductive film (transparent electrically-conductive film 43, film thickness; 500 nm) was formed on a glass substrate (substrate 44, thickness of 4 mm). Further, a titania paste "18NR-T" (manufactured by DyeSol) was screen-printed on this SnO₂ electrically-conductive film, followed by drying at 120°C. Then, the dried titania paste was calcined at 500°C to form a semiconductor layer 45 (the area of the light-receiving surface; 5 mm × 5 mm, film thickness; 6 µm, a metal complex dye is not carried). Thus, a photoconductor layer 42 (the area of the light-receiving surface; 5 mm × 5 mm, film thickness; 6 µm, a metal complex dye is not carried) not having the light-scattering layer 46 provided thereon was formed on the SnO₂ electrically-conductive film, thereby manufacturing a light-receiving electrode precursor B not carrying a metal complex dye.

### (Adsorption of Dye)

Next, each of the metal complex dyes ((1-1) to (36-1), (1-1TBA) to (36-1TBA), (33-1K) to (36-1K), and (33-1Na) to (36-1Na)) that had been synthesized in Example 1 was carried onto the photoconductor layer 42 not carrying a metal complex dye, as follows. First, each of the metal complex dyes was dissolved in a mixed solvent of t-butanol and acetonitrile at 1:1 (volume ratio) to 2 × 10⁻⁴ mol/L. Further, 30 mol of deoxycholic acid as a co-adsorbent was added to one mol of the metal complex dye, thereby preparing each of dye solutions. Next, the light-receiving electrode precursor A was immersed in each of the dye solutions at 25°C for 20 hours, and dried after pulling out from the dye solution, thereby manufacturing each of light-receiving electrodes 40 having the respective metal complex dyes carried onto the light-receiving electrode precursor A.

Each of the metal complex dyes was similarly carried on the light-receiving electrode precursor B to manufacture each of light-receiving electrodes 40 having the respective metal complex dyes carried onto the light-receiving electrode precursor B.

### (Assembly of Dye-Sensitized Solar Cell)

Then, a platinum electrode (thickness of a Pt thin film; 100 nm) having the same shape and size as that of the electrically conductive support 41 was manufactured as a counter electrode 48. Further, 0.1 M (mol/L) of iodine, 0.1 M of lithium iodide, 0.5 M of 4-t-butylpyridine, and 0.6 M of 1,2-dimethyl-3-propylimidazolium iodide were dissolved in acetonitrile to manufacture a liquid electrolyte as an electrolytic solution. Further, a Spacer-S (trade name: "SURLYN") manufactured by DuPont, which has a shape matching to the size of the photoconductor layer 42, was prepared.

Each of the light-receiving electrodes 40 manufactured as above and the counter electrode 48 were arranged to face each other through the spacer-S and thermally compressed, and then the liquid electrolyte was filled from the inlet for the electrolytic solution between the photoconductor layer 42 and the counter electrode 48, thereby forming a charge transfer layer 47. The outer periphery and the inlet for the electrolytic solution of thus manufactured cell were sealed and cured using RESIN XNR-5516 manufactured by Nagase ChemteX Corporation, thereby producing each of dye-sensitized solar cells (Sample Nos. 1 to 36).

Each of the dye-sensitized solar cells with the Sample Nos. produced as above includes two kinds of those using electrically neutral metal complex dyes (1-1 to 36-1) and those using metal complex dyes (1-1TBA to 36-1TBA) of TBA salts. The dye-sensitized solar cells with the Sample Nos. 33 to 36 includes those using metal complex dyes (33-1K to 36-1K) of K salts and those using metal complex dyes (33-1Na to 36-1Na) of Na salts.

Furthermore, in each of the dye-sensitized solar cells with the Sample Nos., the dye-sensitized solar cells using electrically neutral metal complex dyes and the dye-sensitized solar cells using metal complex dyes of TBA salts each include two kinds of the dye-sensitized solar cells produced using the light-receiving electrode precursor A ("A" attached to the Sample No.) and the dye-sensitized solar cells produced using the light-receiving electrode precursor B ("B" attached to the Sample No.). For the Sample Nos. 33 to 36, the dye-sensitized solar cells using the metal complex dyes (33-1K to 36-1K) of the K salts, and the metal complex dyes (33-1Na to 36-1Na) of the Na salts each further include dye-sensitized solar cells produced using the light-receiving electrode precursor A and the dye-sensitized solar cells produced using the light-receiving electrode precursor B.

Comparative dye-sensitized solar cells (Sample Nos. c1 to c3) were produced in the same manner as for the production of the dye-sensitized solar cells, except that each of the following comparative metal complex dyes (C1) to (C3) was used instead of the metal complex dye synthesized in Example 1 in the production of the dye-sensitized solar cell.

The metal complex dye (C1) is the compound "HIS-2" described in Advanced Functional Materials 2013, 23, pp. 1817-1823. The metal complex dye (C2) is the compound described in paragraph 00443 of JP2012-36237A. The metal complex dye (C3) was synthesized in accordance with the method for synthesizing the metal complex dye (2-1).

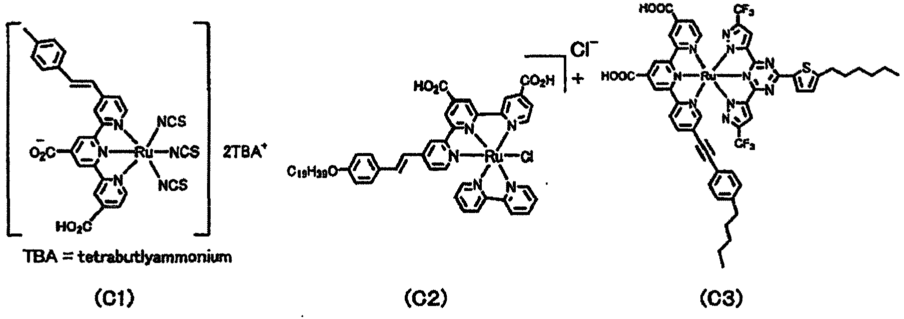

### <Evaluation of Photoelectric Conversion Efficiency>

The cell characteristic test was carried out by using each of the produced dye-sensitized solar cells. The cell characteristic test was carried out by irradiating artificial sunlight of 1,000 W/m² from a xenon lamp through an AM 1.5 filter, using a solar simulator (WXS-85H manufactured by WACOM). The current-voltage characteristics were measured using an I-V tester to determine the photoelectric conversion efficiency.

### (Conversion Efficiency (A))

For each of the dye-sensitized solar cells (Sample Nos. 1A to 36A and c1A to c3A) produced using the light-receiving electrode precursors A in the dye-sensitized solar cells with the respective Sample Nos., the determined photoelectric conversion efficiency (referred to as conversion efficiency (A)) was evaluated according to the following criteria in comparison with that of the conversion efficiency (A_{c2A}) of the comparative dye-sensitized solar cell (Sample No. c2A).

For evaluation of the conversion efficiency (A), A and B are the acceptable levels in the present test, with A being preferable.

The conversion efficiency (A) was evaluated according to the following criteria in comparison with that of the conversion efficiency (A_{c2A}).
A: More than 1.20 times
B: More than 1.10 times and 1.20 times or less
C: More than 1.00 time and 1.10 times or less
D: 1.00 time or less

### (Conversion Efficiency (B))

For each of the dye-sensitized solar cell (Sample Nos. 1B to 36B and c1B to c3B) produced using the light-receiving electrode precursors B in the dye-sensitized solar cells with the respective Sample Nos., each photoelectric conversion efficiency (referred to as conversion efficiency (B)) was determined in the same manner as for the conversion efficiency (A).

The determined conversion efficiency (B) was evaluated according to the following criteria in comparison with that of the conversion efficiency (A_{c2A}) of the comparative dye-sensitized solar cell (Sample No. c2A).

For evaluation of the conversion efficiency (B), S+, S, S-, A+, A-, and B are the acceptable levels of the present test, with S+, S, S-, A+, and A- being preferable.

The conversion efficiency (B) was evaluated as follows in comparison with that of the conversion efficiency (A_{c2A}).
S+: More than 1.20 times
S: More than 1.15 times and 1.20 times or less
S-: More than 1.10 times and 1.15 times or less
A+: More than 1.05 times and 1.10 times or less
A-: More than 1.00 time and 1.05 times or less
B: More than 0.90 times and 1.00 time or less
C: 0.90 times or less

### <Evaluation of Durability>

The heat cycle test was carried out for evaluation on durability (thermal deterioration), using each of the dye-sensitized solar cell (Sample Nos. 1A to 36A and c1A to c3A) produced using the light-receiving electrode precursors A in the dye-sensitized solar cells with the respective Sample Nos.

Each of the dye-sensitized solar cells was alternately introduced into a freezer at -10°C and a constant-temperature tank at 50°C every 12 hours so as to repeat cooling and heating (heat cycle test). The current was measured for the dye-sensitized solar cells before the heat cycle test and the dye-sensitized solar cell at 72 hours after the heat cycle test, respectively. The value determined by dividing the current value (short-circuit current density) determined from the current-voltage characteristic measurement in the dye-sensitized solar cell at 72 hours after the heat cycle test with the current value (short-circuit current density) measured in the dye-sensitized solar cells before the heat cycle test was taken as a current holding ratio. By the current holding ratios thus obtained, the durability was evaluated according to the following criteria.

For evaluation of the durability, A and B are the acceptable levels in the present test, with A being preferable.
A: 0.9 times or more
B: Less than 0.9 times and 0.8 times or more
C: Less than 0.8 times and 0.7 times or more
D: Less than 0.7 times

**[Table 1] Table 1 (Table 1-1)**

| Sample No. | Metal complex dye | Conversion efficiency (A) | Conversion efficiency (B) | Durability | Note | Sample No. | Metal complex dye | conversion efficiency (A) | Conversion efficiency (B) | Durability | Note |
|---|---|---|---|---|---|---|---|---|---|---|---|
| 1 | (1-1) | A | S- | A | The present invention | 18 | (18-1) | A | A- | A | The present invention |
| | (1-1TBA | A | S- | B | The present invention | | (18-1TBA) | A | A- | B | The present invention |
| 2 | (2-1) | A | S | A | The present invention | 19 | (19-1) | A | A+ | A | The present invention |
| | (2-1TBA) | A | S | B | The present invention | | (19-1TBA) | A | A+ | B | The present invention |
| 3 | (3-1) | A | A+ | A | The present invention | 20 | (20-1) | A | A- | A | The present invention |
| | (3-1TBA) | A | A+ | B | The present invention | | (20-1TBA) | A | A- | B | The present invention |
| 4 | (4-1) | A | A- | A | The present invention | 21 | (21-1) | A | S+ | A | The present invention |
| | (4-1TBA) | A | A- | B | The present invention | | (21-1TBA) | A | S+ | B | The present invention |
| 5 | (5-1) | A | A+ | A | The present invention | 22 | (22-1) | A | S+ | A | The present invention |
| | (5-1TBA) | A | A+ | B | The present invention | | (22-1TBA) | A | S+ | B | The present invention |
| 6 | (6-1) | A | S- | B | The present invention | 23 | (23-1) | A | S+ | A | The present invention |
| | (6-1TBA) | A | S- | B | The present invention | | (23-1TBA) | A | S+ | B | The present invention |
| 7 | (7-1) | A | A+ | B | The present invention | 24 | (24-1) | B | A+ | A | The present invention |
| | (7-1TBA) | A | A+ | B | The present invention | | (24-1TBA) | B | A+ | B | The present invention |
| 8 | (8-1) | A | A- | A | The present invention | 25 | (25-1) | A | S+ | A | The present invention |
| | (8-1TBA) | A | A- | B | The present invention | | (25-1TBA) | A | S+ | B | The present invention |
| 9 | (9-1) | A | S | A | The present invention | 26 | (26-1) | A | S | A | The present invention |
| | (9-1TBA) | A | S | B | The present invention | | (26-1TBA) | A | S | B | The present invention |
| 10 | (10-1) | B | A- | A | The present invention | 27 | (27-1) | A | S+ | A | The present invention |
| | (10-1TBA) | B | A- | B | The present invention | | (27-1TBA) | A | S+ | B | The present invention |
| 11 | (11-1) | B | A+ | A | The present invention | 28 | (28-1) | A | S+ | A | The present invention |
| | (11-1TBA) | B | A+ | B | The present invention | | (28-1TBA) | A | S+ | B | The present invention |
| 12 | (12-1) | B | A+ | A | The present invention | 29 | (29-1) | A | S+ | A | The present invention |
| | (12-1TBA) | B | A+ | B | The present invention | | (29-1TBA) | A | S+ | B | The present invention |
| 13 | (13-1) | B | A- | A | The present invention | 30 | (30-1) | A | S+ | A | The present invention |
| | (13-1TBA) | B | A- | B | The present invention | | (30-1TBA) | A | S+ | B | The present invention |
| 14 | (14-1) | B | A+ | A | The present invention | 31 | (31-1) | A | S+ | A | The present invention |
| | (14-1TBA) | B | A+ | B | The present invention | | (31-1TBA) | A | S+ | B | The present invention |
| 15 | (15-1) | B | A- | A | The present invention | 32 | (32-1) | A | S+ | A | The present invention |
| | (15-1TBA) | B | A- | B | The present invention | | (32-1TBA) | A | S+ | B | The present invention |
| 16 | (16-1) | B | A+ | A | The present invention | 33 | (33-1) | A | A- | A | The present invention |
| | (16-1TBA) | B | A+ | B | The present invention | | (33-1TBA) | A | A- | B | The present invention |
| 17 | (17-1) | B | B | A | The present invention | | (33-1K) | A | A- | A | The present invention |
| | (17-1 TBA) | B | B | B | The present invention | | (33-1Na) | A | A- | A | The present invention |

**[Table 2] Table 1 (Table 1-2)**

| Sample No. | Metal complex dye | Conversion efficiency (A) | Conversion efficiency (B) | Durability | Note | Sample No. | Metal complex dye | Conversion efficiency (A) | Conversion efficiency (B) | Durability | Note |
|---|---|---|---|---|---|---|---|---|---|---|---|
| 34 | (34-1) | A | A- | A | The present invention | 36 | (36-1) | A | S- | A | The present invention |
| | (34-1TBA) | A | A- | B | The present invention | | (36-TBA) | A | S- | B | The present invention |
| | (34-1K) | A | A- | A | The present invention | | (36-1 K) | A | S- | A | The present invention |
| | (34-1Na) | A | A- | A | The present invention | | (36-1Na) | A | S- | A | The present invention |
| 35 | (35-1) | A | S- | A | The present invention | c1 | (C1) | C | C | D | Comparative Example |
| | (35-1TBA) | A | S- | B | The present invention | c2 | (C2) | D | C | C | Comparative Example |
| | (35-1K) | A | S- | A | The present invention | c3 | (C3) | C | C | B | Comparative Example |
| | (35-1Na) | A | S- | A | The present invention | | | | | | |

As seen from the results of Table 1, all of the photoelectric conversion elements and the dye-sensitized solar cells of the present invention (Sample Nos. 1 to 36), in which the metal complex dye represented by Formula (1) using a combination of the tridentate ligand L1 represented by Formula (L1-1) and the ligand L2 was carried on semiconductor fine particles, had high conversion efficiency (A) and conversion efficiency (B), as well as a high current holding ratio. Accordingly, it could be seen that by using a combination of the ligand L1 and the ligand L2 as a ligand of the metal complex dye, the photoelectric conversion elements and the dye-sensitized solar cells exhibit excellent photoelectric conversion efficiency and high durability, irrespective of the film thickness of the semiconductor layer, for example, even when they are thin with a thickness of up to 6 µm. The metal complex dye of the present invention provided the same results even when they are electrically neutral, or they are TBA salts, K salts, or Na salts.

It could also be seen that for the ligand L1, if L^{V} is the group represented by Formula (LV-2), in particular, if at least one of specific sp² carbon atoms in a heteroaryl group as R^{V3} in Formula (LV-2) has a substituent, a use of the ligand L1 in combination with the ligand L2 increases the effect of enhancing the conversion efficiency (B).

In addition, it could be seen that for the ligand L2, if R^{VL} in Formula (R^{VL}) is an aromatic ring group or an amino group (a group having an amino group), in particular, if it is a thiophene ring group represented by Formula (S-1) or (S-3), a benzene ring group represented by Formula (S-4), or an amino group (a group having an amino group), a use of the ligand L2 in combination with the ligand L1 increases the effect of enhancing the conversion efficiency (B).

Moreover, from the results of Table 1, it could also be seen that the metal complex dye of the present invention, having the ligand L1 and the ligand L2, can be suitably used as a sensitizing dye of the photoelectric conversion element and the dye-sensitized solar cell of the present invention. It could also be seen that the dye solution of the present invention, containing a metal complex dye having the ligand L1 and the ligand L2, and a solvent, can be suitably used for the preparation of semiconductor fine particles carrying the metal complex dye of the present invention. In addition, it could also be seen that the terpyridine compound of the present invention is suitable as a ligand of the metal complex dye of the present invention, and in particular, an esterified product of the compound is suitable as a ligand precursor of the metal complex dye of the present invention.

To contrary, the comparative photoelectric conversion elements and dye-sensitized solar cells (Sample Nos. c1 to c3), in which the metal complex dyes not using a combination of the ligand L1 and the ligand L2 were carried on semiconductor fine particles were not satisfactory, at least in terms of photoelectric conversion efficiency. Specifically, the metal complex dyes c1 and c2 not having the ligand L2 had low conversion efficiency (A) and conversion efficiency (B), as well as a low current holding ratio. Further, the metal complex dye c3 having a ligand having an ethynyl group substituted at the m-position (3-position) with respect to a ring-constituting nitrogen atom which coordinates to a metal ion M had low conversion efficiency (A) and conversion efficiency (B), even when the metal complex dye c3 has the ligand L2 represented by Formula (L2-4).

Although the present invention has been described with reference to embodiments, it is not intended that the present invention is not limited by any of the details of the description unless otherwise specified, but should rather be construed broadly within the spirit and scope of the present invention as set out in the accompanying claims.

The present application claims the priority based on JP2014-121016 filed on June 11, 2014, and JP2015-046443 filed on March 9, 2015, the contents of which are partly incorporated herein by reference.

### Explanation of References

1, 41: ELECTRICALLY CONDUCTIVE SUPPORT
2, 42: PHOTOCONDUCTOR LAYER
21: DYE
22: SEMICONDUCTOR FINE PARTICLES
3, 47: CHARGE TRANSFER LAYER
4, 48: COUNTER ELECTRODE
5, 40: LIGHT-RECEIVING ELECTRODE
6: EXTERNAL CIRCUIT
10: PHOTOELECTRIC CONVERSION ELEMENT
100: SYSTEM IN WHICH PHOTOELECTRIC CONVERSION ELEMENT IS APPLIED TO CELL USES
M: OPERATING MEANS (FOR EXAMPLE, ELECTRIC MOTOR)
20: DYE-SENSITIZED SOLAR CELL
43: TRANSPARENT ELECTRICALLY-CONDUCTIVE FILM
44: SUBSTRATE
45: SEMICONDUCTOR LAYER
46: LIGHT-SCATTERING LAYER
S: SPACER

## Claims

1. A photoelectric conversion element comprising:
an electrically conductive support;
a photoconductor layer including an electrolyte;
a charge transfer layer including an electrolyte; and
a counter electrode,
wherein the photoconductor layer has semiconductor fine particles carrying a metal complex dye represented by the following Formula (1),
Formula (1) ML1L2(X)ₙ₁)·CI_{mY}
in Formula (1),
M represents a metal ion;
L1 represents a tridentate ligand represented by the following Formula (L2-1), 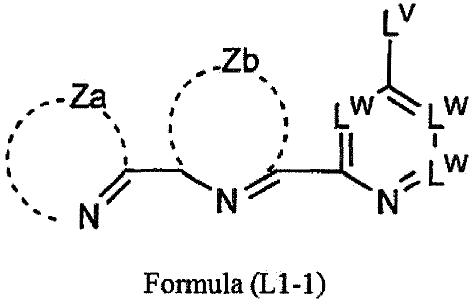 in Formula (L1-1), Za and Zb each independently represent a non-metal atomic group required for completing a 5- or 6-membered ring, in which at least one side of the rings formed by the respective Za and Zb has one or more acidic groups. L^{W}'s each independently represent a nitrogen atom or CR^{W}, R^{W} represents a hydrogen atom or a substituent, and L^{V} represents a group represented by the following Formula (LV-1) or (LV-2),
-R^{V1}=R^{V2}-R^{V3} Formula (LV-1)
-C≡C-R^{V3} Formula (LV-2)
in Formula (LV-1), R^{V1} and R^{V2} each independently represent a nitrogen atom or CR^{V4}, and R^{V4} represents a hydrogen atom or a substituent, and
in Formula (LV-1) and Formula (LV-2), R^{V3} represents an aryl group or a heteroaryl group;
L2 represents a bidentate or tridentate ligand represented by any one of the following Formulae (L2-1) to (L2-8), 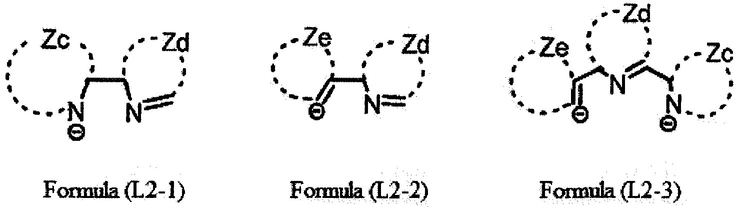 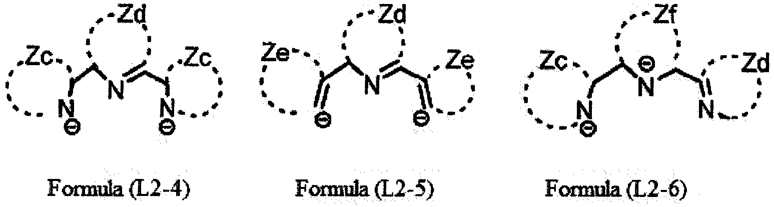 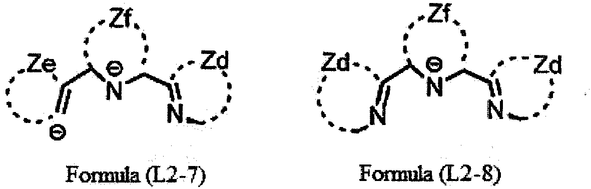 in Formulae (L2-1) to (L2-8), Zc, Zd, Ze, and Zf each independently represent a non-metal atomic group required for completing a 5- or 6-membered aromatic ring, and the ring formed by Zd has an organic group R^{VL} represented by the following Formula (R^{VL}),
Formula (R^{VL}): -(R^{VL})n^{VL}
in Formula (R^{VL}), R^{VL} represents an organic group selected from the group consisting of a monocyclic aromatic ring group bonded to the ring formed by Zd or a polycyclic aromatic ring group including the monocycle as a fused ring, in which the sp² carbon atoms at the α-position with respect to the ring-constituting atom bonded to the ring formed by Zd in a case where the monocycle is a 5-membered ring, and the sp² carbon atoms at the α- and β-positions with respect to the ring-constituting atom bonded to the ring formed by Zd in a case where the monocycle is a 6-membered ring are all bonded to hydrogen atoms or ring-constituting atoms of a fused ring different from the monocycle, an alkyl group, an alkenyl group, an alkynyl group, an alkylthio group, an alkoxy group, an amino group, and a silyl group, and n^{VL} represents an integer of 0 or more, which is an integer that is not more than the number of hydrogen atoms when the ring formed by Zd is unsubstituted;
X represents a monodentate ligand and n1 represents 0 or 1;
CI represents a counterion when the counterion is required to neutralize charges; and
mY represents an integer of 0 to 3.

2. The photoelectric conversion element according to claim 1, wherein the aromatic ring group of R^{VL} is a ring group represented by any one of the following Formulae (S-1) to (S-4), 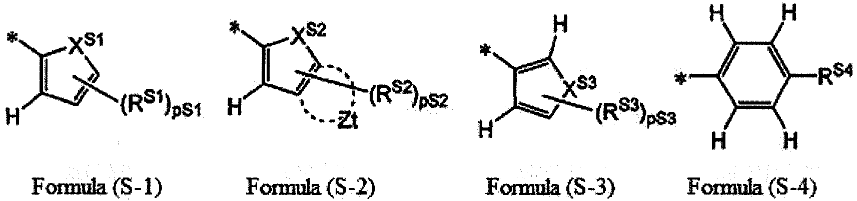
in formulae, X^{S1} to X^{S3} each independently represent -O-, -S-, -NR^{S}-, -C(R^{S})₂-, or -Si(R^{S})₂-, and R^{S}'s each represent a hydrogen atom or a substituent;
Zt represents a non-metal atomic group required for forming a fused ring together with a ring including X^{S2};
R^{S1} to R^{S4} each independently represent a substituent, pS1 represents an integer of 0 to 2, pS2 is an integer of 0 or more, which is not more than the number of hydrogen atoms when the fused ring formed by Zt is unsubstituted, and pS3 represents 0 or 1; and
* represents a bonding moiety to the ring formed by Zd.

3. The photoelectric conversion element according to claim 2, wherein the organic group R^{VL} is an organic group selected from the group consisting of the ring group represented by Formula (S-1), the ring group represented by Formula (S-2), the ring group represented by Formula (S-3), the ring group represented by Formula (S-4), an alkyl group, an alkenyl group, an alkynyl group, an alkylthio group, an alkoxy group, and an amino group.

4. The photoelectric conversion element according to claim 2 or 3, wherein the organic group R^{VL} is a ring group represented by any one of Formula (S-1), Formula (S-2), Formula (S-3), and Formula (S-4), or an amino group.

5. The photoelectric conversion element according to any one of claims 2 to 4, wherein X^{S1} to X^{S3} each independently represent -O- or -S-.

6. The photoelectric conversion element according to any one of claims 1 to 5, wherein the organic group R^{VL} has at least one substituent selected from the group consisting of an aromatic ring group, an alkyl group, an alkenyl group, an alkynyl group, an alkylthio group, an alkoxy group, and an amino group.

7. The photoelectric conversion element according to claim 6, wherein the organic group R^{VL} is an amino group having the substituent.

8. The photoelectric conversion element according to any one of claims 1 to 7, wherein
the ring formed by Za is at least one selected from the group consisting of a pyridine ring, a pyrimidine ring, a pyrazine ring, a pyridazine ring, a triazine ring, a tetrazine ring, a quinoline ring, an isoquinoline ring, a pyrazole ring, an imidazole ring, a triazole ring, a thiazole ring, an oxazole ring, a benzimidazole ring, a benzotriazole ring, a benzoxazole ring, and a benzothiazole ring,
the ring formed by Zb is at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a triazole ring, a thiazole ring, an oxazole ring, a benzimidazole ring, a benzotriazole ring, a benzoxazole ring, and a benzothiazole ring, and
the ring including L^{W} is at least one selected from the group consisting of a pyridine ring, a pyrimidine ring, a pyridazine ring, a triazine ring, a tetrazine ring, and a quinoline ring.

9. The photoelectric conversion element according to any one of claims 1 to 8, wherein
the ring formed by Zc is at least one selected from the group consisting of a pyrazole ring, a pyrrole ring, an imidazole ring, a triazole ring, a benzimidazole ring, and an indole ring,
the ring formed by Zd is at least one selected from the group consisting of a pyridine ring, a pyrimidine ring, a pyrazine ring, a pyridazine ring, a triazine ring, a tetrazine ring, a quinoline ring, a pyrazole ring, an imidazole ring, a triazole ring, a thiazole ring, an oxazole ring, a benzimidazole ring, a benzotriazole ring, a benzoxazole ring, and a benzothiazole ring,
the ring formed by Ze is a benzene ring, and
the ring formed by Zf is at least one selected from the group consisting of a pyrrole ring, an imidazole ring, a benzimidazole ring, and an indole ring.

10. The photoelectric conversion element according to any one of claims 1 to 9, wherein M is Ru²⁺ or Os²⁺.

11. The photoelectric conversion element according to any one of claims 1 to 10, wherein L1 is a tridentate ligand represented by the following Formula (L1-2), 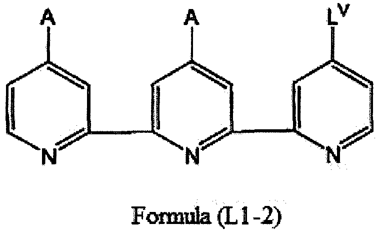 in Formula (L1-2),
A represents an acidic group; and
L^{V} has the same definition as L^{V} of Formula (L1-1).

12. The photoelectric conversion element according to any one of claims 1 to 11, wherein the acidic group is a carboxy group or a salt thereof.

13. The photoelectric conversion element according to any one of claims 1 to 12, wherein the metal complex dye represented by Formula (1) is a metal complex dye represented by any one of the following Formulae (2) to (6), 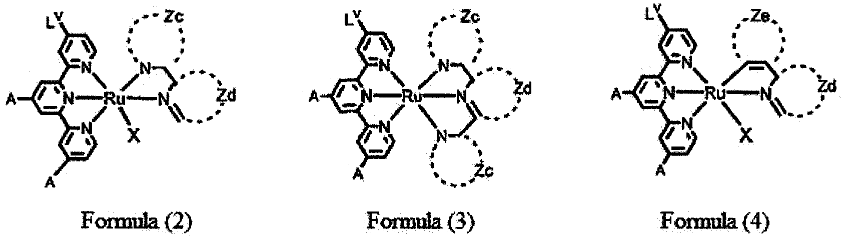 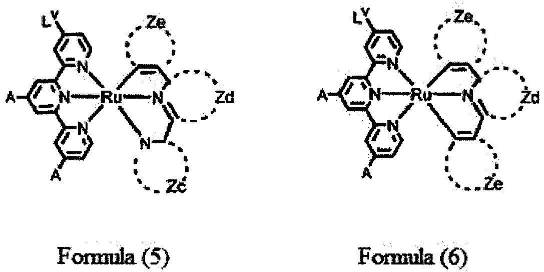 in Formulae (2) to (6),
X has the same definition as X of Formula (1);
Zc, Zd, and Ze have the same definitions as Zc, Zd, and Ze, respectively, in Formulae (L2-1) to (L2-5);
L^{V} has the same definition as L^{V} of Formula (L1-1); and
A represents an acidic group.

14. The photoelectric conversion element according to any one of claims 1 to 13, wherein L^{V} is the group represented by Formula (LV-2).

15. The photoelectric conversion element according to any one of claims 1 to 14, wherein R^{V3} is a heteroaryl group.

16. The photoelectric conversion element according to any one of claims 1 to 15, wherein R^{V3} has an alkyl group, an alkoxy group, or an alkylthio group as a substituent.

17. The photoelectric conversion element according to any one of claims 1 to 16, wherein the heteroaryl group of R^{V3} is a monocyclic group bonded to the ethynylene group in Formula (LV-2) or a polycyclic group including the monocycle as a fused ring, in which at least one of the sp² carbon atoms at the α-position with respect to the ring-constituting atom bonded to the ethynylene group in a case where the monocycle is a 5-membered ring has a substituent, and at least one of the sp² carbon atoms at the α- and β-positions with respect to the ring-constituting atom bonded to the ethynylene group in a case where the monocycle is a 6-membered ring has a substituent.

18. The photoelectric conversion element according to any one of claims 1 to 17, wherein the heteroaryl group of R^{V3} is represented by the following Formula (LV-3), 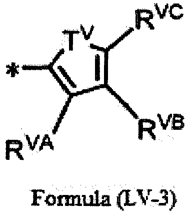 in the formula,
T^{V} represents -O-, -S-, -NR^{TV}-, -C(R^{TV})₂-, or -Si(R^{TV})₂-, and R^{TV}'s each represent a hydrogen atom or a substituent;
R^{VA} represents a substituent,
R^{VB} and R^{VC} each independently represent a hydrogen atom or a substituent; and
* represents a binding position to the ethynylene group in Formula (LV-2).

19. A dye-sensitized solar cell comprising the photoelectric conversion element according to any one of claims 1 to 18.

20. A metal complex dye represented by the following Formula (1),
Formula (1) ML1L2(X)ₙ₁·CI_{mY}
in Formula (1),
M represents a metal ion;
L1 represents a tridentate ligand represented by the following Formula (L1-1), 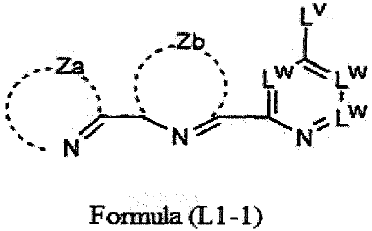 in Formula (L1-1), Za and Zb each independently represent a non-metal atomic group required for completing a 5- or 6-membered ring, in which at least one side of the rings formed by the respective Za and Zb has one or more acidic groups, L^{W}'s each independently represent a nitrogen atom or CR^{W}, and R^{W} represents a hydrogen atom or a substituent; and L^{V} represents a group represented by the following Formula (LV-1) or (LV-2),
**-R^{V1}=R^{V2}-R^{V3}** **Formula (LV-1)**
**-C≡C-R^{V3}** **Formula (L2-2)**
in Formula (LV-1), R^{V1} and R^{V2} each independently represent a nitrogen atom or CR^{V4}, and R^{V4} represents a hydrogen atom or a substituent, and in Formula (LV-1) and Formula (LV-2), R^{V3} represents an aryl group or a heteroaryl group;
L2 represents a bidentate or tridentate ligand represented by any one of the following Formulae (L2-1) to (L2-8), 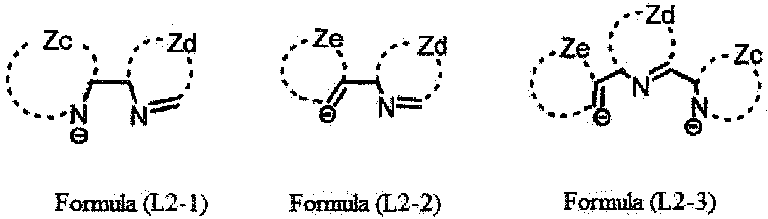 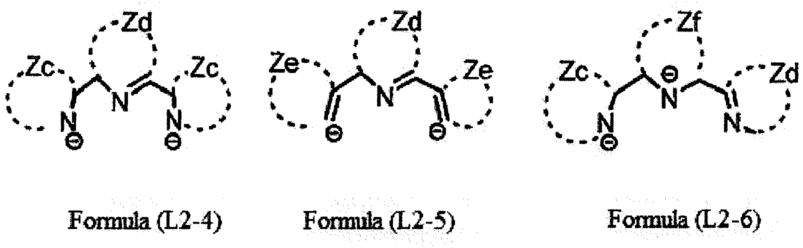 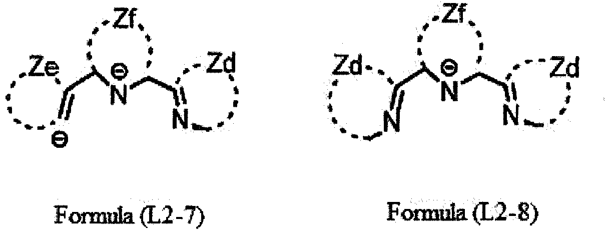 in Formulae (L2-1) to (L2-8), Zc, Zd, Ze, and Zf each independently represent a non-metal atomic group required for completing a 5- or 6-membered aromatic ring, and the ring formed by Zd has an organic group R^{VL} represented by the following Formula (R^{VL}),
Formula (R^{VL}): -(R^{VL})n^{VL}
in Formula (R^{VL}), R^{VL} represents an organic group selected from the group consisting of a monocyclic aromatic ring group bonded to the ring formed by Zd or a polycyclic aromatic ring group including the monocycle as a fused ring, in which the sp² carbon atoms at the α-position with respect to the ring-constituting atom bonded to the ring formed by Zd in a case where the monocycle is a 5-membered ring, and the sp² carbon atoms at the α- and β-position with respect to the ring-constituting atom bonded to the ring formed by Zd in a case where the monocycle is a 6-membered ring are all bonded to hydrogen atoms or ring-constituting atoms of a fused ring different from the monocycle, an alkyl group, an alkenyl group, an alkynyl group, an alkylthio group, an alkoxy group, an amino group, and a silyl group, and n^{VL} represents an integer of 0 or more, which is an integer that is not more than the number of hydrogen atoms when the ring formed by Zd is unsubstituted;
X represents a monodentate ligand and n1 represents 0 or 1;
CI represents a counterion when the counterion is required to neutralize charges; and
mY represents an integer of 0 to 3.

21. A dye solution comprising the metal complex dye according to claim 20 and a solvent.

22. A terpyridine compound represented by the following Formula (L1-2) or an esterified product thereof, 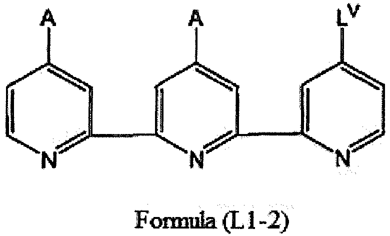 in Formula (L1-2),
A represents an acidic group; and
L^{V} represents a group represented by the following Formula (LV-2),
**-C≡C-R^{V3}** **Formula (LV-2)**
in Formula (LV-2), R^{V3} represents a heteroaryl group.
